# EUROPÄISCHE PATENTSCHRIFT

(11) **EP 2 284 166 B1**
(45) Veröffentlichungstag und Bekanntmachung des Hinweises auf die Patenterteilung: **16.10.2013**
(21) Anmeldenummer: 10178252.2
(22) Anmeldetag: 14.08.2007
(51) Int. Cl.: C07D 401/02, C07D 311/16, C07D 205/04, C07D 207/06, C07D 223/04, C07D 403/04, C07D 403/06

(54) **Arylsulfonamide mit analgetischer Wirkung**
Arylsulfonamides with analgetic activity
Arylsulfonamides ayant activité analgésique

(30) Priorität: 19.08.2006 DE 102006039003
(43) Veröffentlichungstag der Anmeldung: 16.02.2011
(62) Teilanmeldung aus: 07788420.3
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: Kauffmann-Hefner, Iris, 88448 ATTENWEILER (DE); Hauel, Norbert, 88433 SCHEMMERHOFEN (DE); Walter, Rainer, 88400 BIBERACH (DE); Ebel, Heiner, 88400 BIBERACH (DE); Doods, Henri, 88447 WARTHAUSEN (DE); Ceci, Angelo, 88441 MITTELBIBERACH (DE); Schuler-Metz, Annette, 89081 Ulm (DE); Konetzki, Ingo, 52076 Aachen-Oberfrostbach (DE)
(74) Vertreter: Simon, Elke Anna Maria

(56) Entgegenhaltungen:
- WO-A-02/053516
- WO-A-03/106428
- WO-A-2006/036664
- WO-A-2006/048209
- DATABASE CROSSFIRE BEILSTEIN Beilstein Institut zur Förderung der Wissenschaften, Frankfurt am Main, DE; XP002466545, Database accession no. BRN 6007541 & EL-NAGGAR ET AL.: POL. J. CHEM., Bd. 56, Nr. 10-12, 1982, Seiten 1279-1285,
- DATABASE CROSSFIRE BEILSTEIN Beilstein Institut zur Förderung der Wissenschaften, Frankfurt am Main, DE; XP002466834, Database accession no. BRN 6009473 & EL-NAGGAR ET AL.: POL. J. CHEM., Bd. 56, Nr. 10-12, 1982, Seiten 1279-1285,
- DATABASE CROSSFIRE BEILSTEIN Beilstein Institut zur Förderung der Wissenschaften, Frankfurt am Main, DE; XP002466835, Database accession no. BRN 2905397 & BRAICHENKO, V. T.: PHARM. CHEM. J. (ENGL. TRANSL.), Bd. 6, Nr. 8, 1972, Seiten 492-494,
- DATABASE CROSSFIRE BEILSTEIN Beilstein Institut zur Förderung der Wissenschaften, Frankfurt am Main, DE; XP002466836, Database accession no. BRN 2400317 & BRAICHENKO, V. T.: PHARM. CHEM. J. (ENGL. TRANSL.), Bd. 6, Nr. 8, 1972, Seiten 492-494,
- DATABASE CROSSFIRE BEILSTEIN Beilstein Institut zur Förderung der Wissenschaften, Frankfurt am Main, DE; XP002466837, Database accession no. BRN 9271162 & MUKHERJEE, G. N., CHAKRABORTY, P. K.: J. INDIAN CHEM. SOC., Bd. 79, Nr. 2, 2002, Seiten 137-141,
- DATABASE CROSSFIRE BEILSTEIN Beilstein Institut zur Förderung der Wissenschaften, Frankfurt am Main, DE; XP002466838, Database accession no. BRN 8906721 & SELVAMURUGAN, V., AIDHEN, INDRAPAL SINGH: SYNTHESIS, Bd. 15, 2001, Seiten 2239-2246,
- DATABASE CROSSFIRE BEILSTEIN Beilstein Institut zur Förderung der Wissenschaften, Frankfurt am Main, DE; XP002466839, Database accession no. BRN 2709200 & PAUL, H. ET AL: ARCH. PHARM. BER. DTSCH. PHARM. GES., Bd. 301, 1968, Seiten 512-519,
- DATABASE CROSSFIRE BEILSTEIN Beilstein Institut zur Förderung der Wissenschaften, Frankfurt am Main, DE; XP002466840, Database accession no. BRN 8789325 & EL-SHARIEF, A. M. SH., AMMAR, Y. A., ZAHRAN, M. A., ALI, A. H., EL-GABY, M. S. A.: MOLECULES, Bd. 6, Nr. 3, 2001, Seiten 267-278,
- DATABASE CROSSFIRE BEILSTEIN Beilstein Institut zur Förderung der Wissenschaften, Frankfurt am Main, DE; XP002466841, Database accession no. BRN 2664221 & SHOEB ET AL.: INDIAN J. CHEM., Bd. 3, 1965, Seite 507,
- DATABASE CROSSFIRE BEILSTEIN Beilstein Institut zur Förderung der Wissenschaften, Frankfurt am Main, DE; XP002466842, Database accession no. BRN 2674860 & SEN, YAJNIK: J. INDIAN CHEM. SOC., Bd. 42, 1965, Seiten 145-146,
- DATABASE CROSSFIRE BEILSTEIN Beilstein Institut zur Förderung der Wissenschaften, Frankfurt am Main, DE; XP002466843, Database accession no. BRN 2709563 & SEN, YAJNIK: J. INDIAN CHEM. SOC., Bd. 42, 1965, Seiten 145-146,
- DATABASE CROSSFIRE BEILSTEIN Beilstein Institut zur Förderung der Wissenschaften, Frankfurt am Main, DE; XP002466844, Database accession no. BRN 2709564 & SEN, YAJNIK: J. INDIAN CHEM. SOC., Bd. 42, 1965, Seiten 145-146,
- DATABASE CROSSFIRE BEILSTEIN Beilstein Institut zur Förderung der Wissenschaften, Frankfurt am Main, DE; XP002466845, Database accession no. BRN 2710222 & SEN, YAJNIK: J. INDIAN CHEM. SOC., Bd. 42, 1965, Seiten 145-146,
- DATABASE CROSSFIRE BEILSTEIN Beilstein Institut zur Förderung der Wissenschaften, Frankfurt am Main, DE; XP002466846, Database accession no. BRN 2956418 & SEN, YAJNIK: J. INDIAN CHEM. SOC., Bd. 42, 1965, Seiten 145-146,
- DATABASE CROSSFIRE BEILSTEIN Beilstein Institut zur Förderung der Wissenschaften, Frankfurt am Main, DE; XP002466847, Database accession no. BRN 2955989 & SEN, YAJNIK: J. INDIAN CHEM. SOC., Bd. 42, 1965, Seiten 145-146,
- MOLANDER G A ET AL: "Reduction of 2-Acylaziridines by Samarium(II) Iodide. An Efficient and Regioselective Route to beta-Amino Carbonyl Compounds", TETRAHEDRON, ELSEVIER SCIENCE PUBLISHERS, AMSTERDAM, NL, Bd. 53, Nr. 26, 30. Juni 1997 (1997-06-30) , Seiten 8887-8912, XP004106098, ISSN: 0040-4020
- SARTORI E ET AL: "SYNTHESIS AND ACTIVITIES OF NET ARYLSULFONAMIDO THROMBOXANE A2 RECEPTOR ANTAGONISTS", EUROPEAN JOURNAL OF MEDICINAL CHEMISTRY, EDITIONS SCIENTIFIQUE ELSEVIER, PARIS, FR, Bd. 28, Nr. 7/8, 1993, Seiten 625-632, XP000396678, ISSN: 0223-5234

## Beschreibung

Gegenstand der vorliegenden Erfindung sind spezifische Verbindungen der allgemeinen Formel **I** 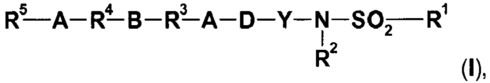 deren Enantiomere, deren Diastereomere, deren Gemische und deren Salze, insbesondere deren physiologisch verträgliche Salze mit organischen oder anorganischen Säuren oder Basen, welche wertvolle Eigenschaften aufweisen, deren Herstellung, die die pharmakologisch wirksamen Verbindungen enthaltenden Arzneimittel, deren Herstellung und deren Verwendung.

In der WO 2006/048209 werden bereits Verbindungen mit B1-antagonistischen Eigenschaften beschrieben. Diese unterscheiden sich jedoch von den Verbindungen der vorliegenden Anmeldung dadurch, dass sie im linken Molekülteil zwingend eine 4-Imidazol-2-yl-phenylgruppe enthalten.

### DETAILLIERTE BESCHREIBUNG DER ERFINDUNG

Verbindungen der allgemeinen Formel **I**, nämlich

| **Beispiel** | **Struktur** |
|---|---|
| (121) | 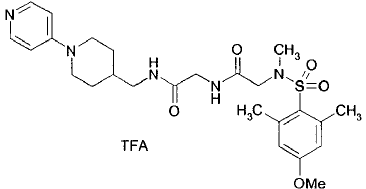 |
| (122) | 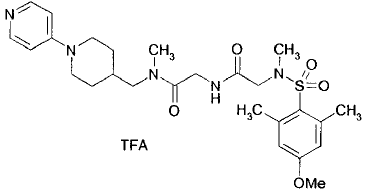 |
| (123) | 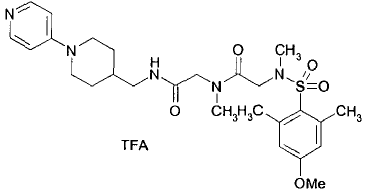 |
| (124) | 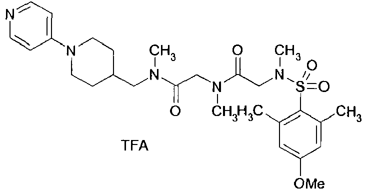 |
| (125) | 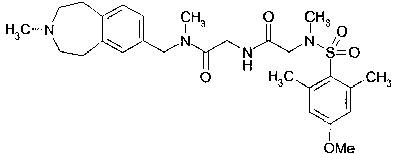 |
| (126) | 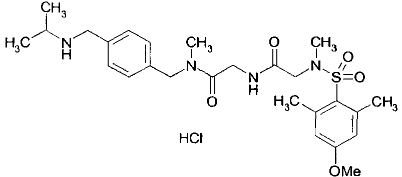 |
| (127) | 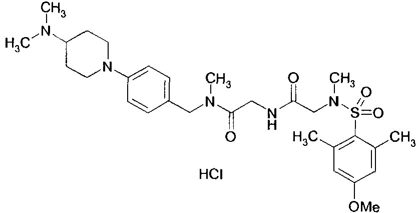 |
| (128) | 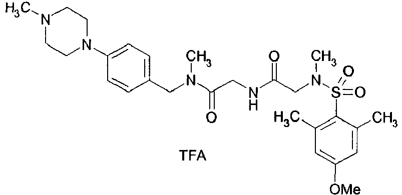 |
| (129) | 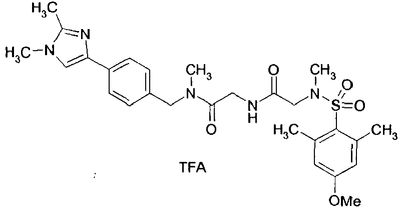 |
| (483) | 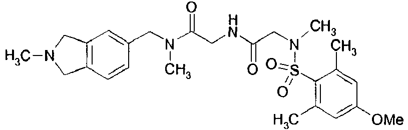 |
| (484) | 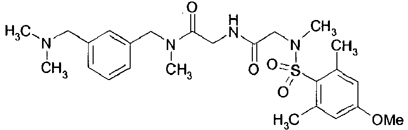 |
| (485) | 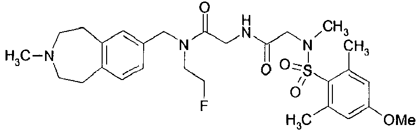 |
| (486) | 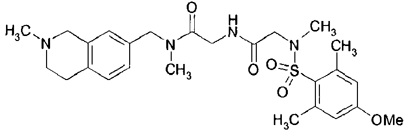 |
| (487) | 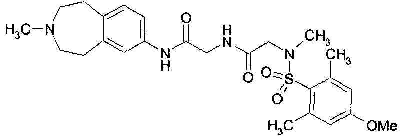 |
| (488) | 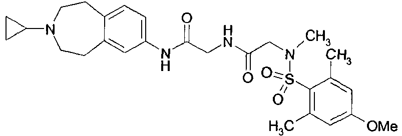 |
| (489) | 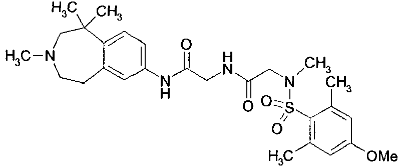 |
| (490) | 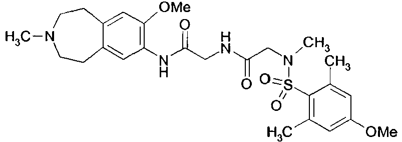 |
| (491) | 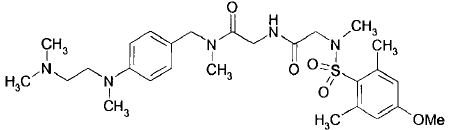 |
| (492) | 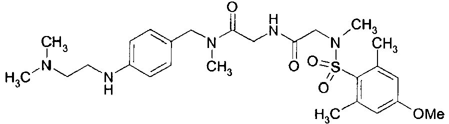 |
| (493) | 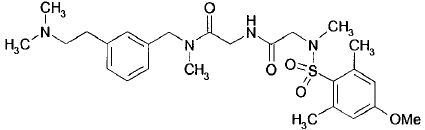 |
| (494) | 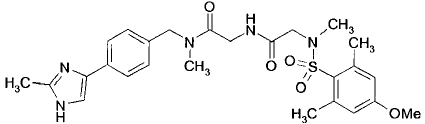 |
| (495) | 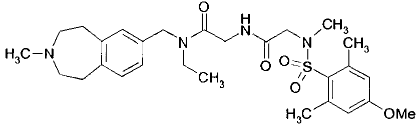 |
| (496) | 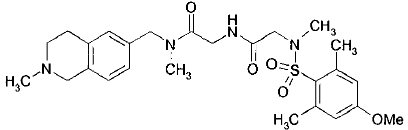 |
| (497) | 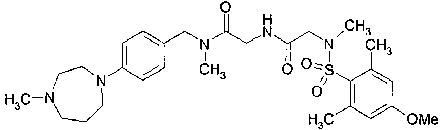 |
| (498) | 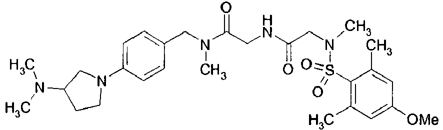 |
| (499) | 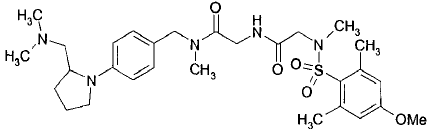 |
| (500) | 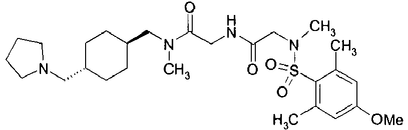 |
| (501) | 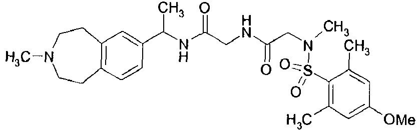 |
| (502) | 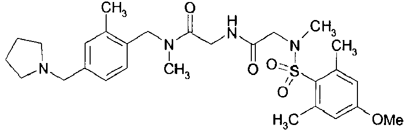 |
| (503) | 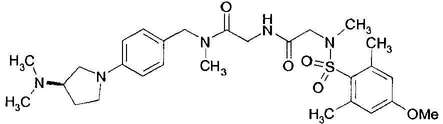 |
| (504) | 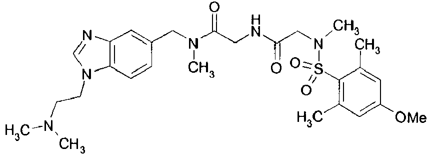 |
| (505) | 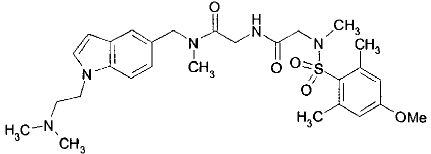 |
| (506) | 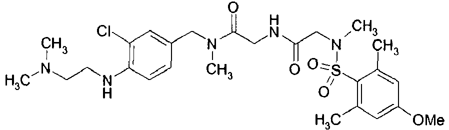 |
| (507) | 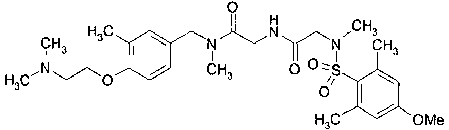 |
| (508) | 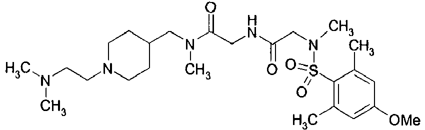 |
| (509) | 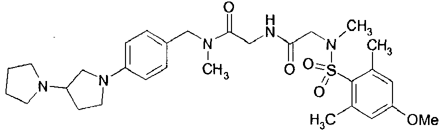 |
| (510) | 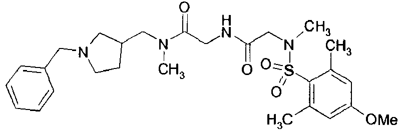 |
| (511) | 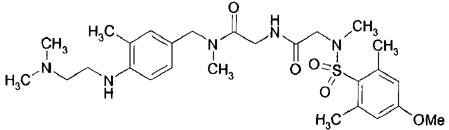 |
| (512) | 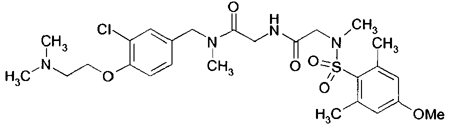 |
| (513) | 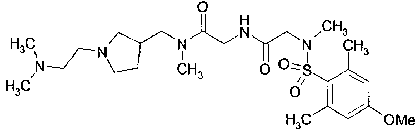 |
| (514) | 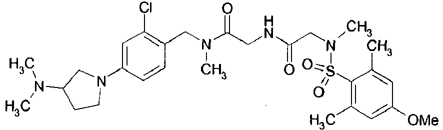 |
| (515) | 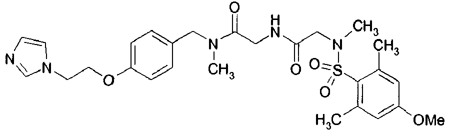 |
| (516) | 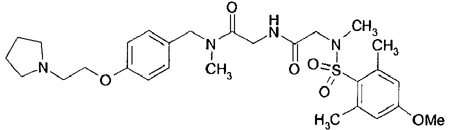 |
| (517) | 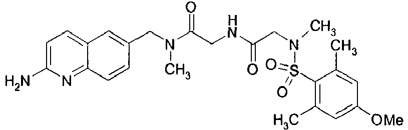 |
| (518) | 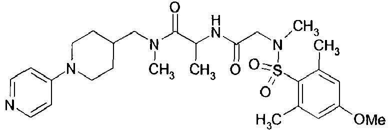 |
| (519) | 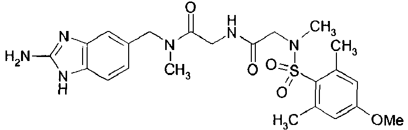 |
| (520) | 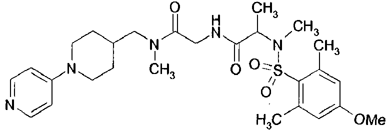 |
| (521) | 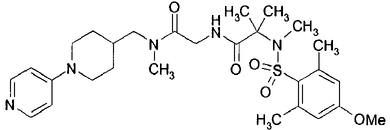 |
| (522) | 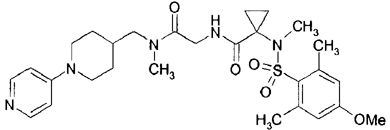 |
| (523) | 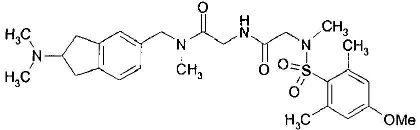 |
| (524) | 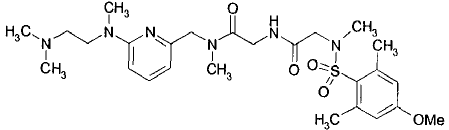 |
| (525) | 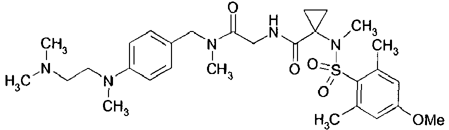 |
| (526) | 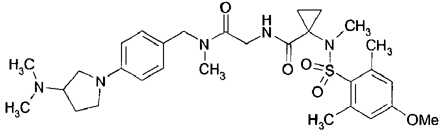 |
| (527) | 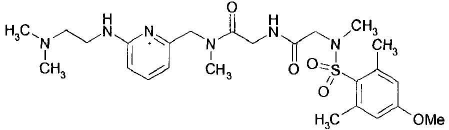 |
| (528) | 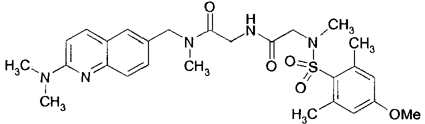 |
| (529) | 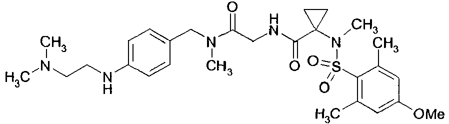 |
| (530) | 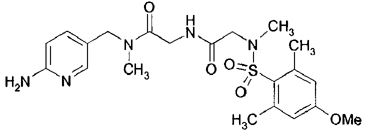 |
| (531) | 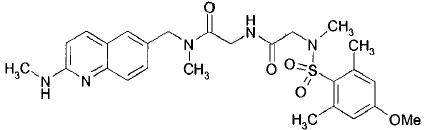 |
| (532) | 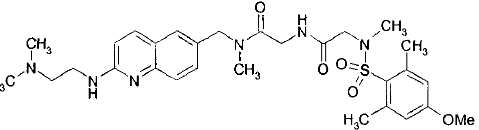 |
| (533) | 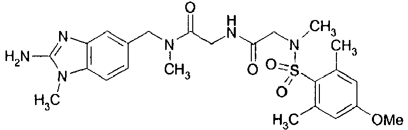 |
| (534) | 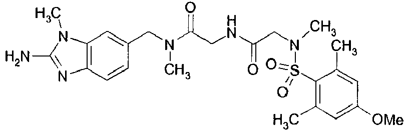 |
| (535) | 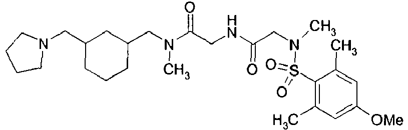 |
| (536) | 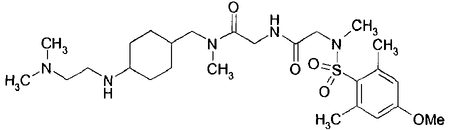 |
| (537) | 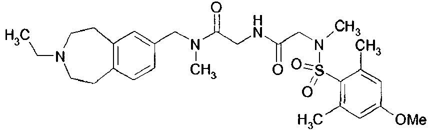 |
| (538) | 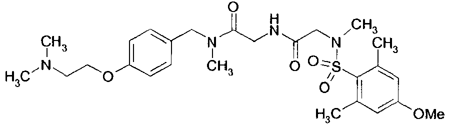 |
| (539) | 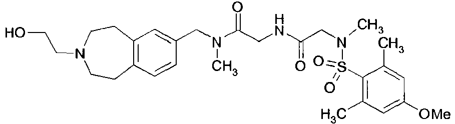 |
| (540) | 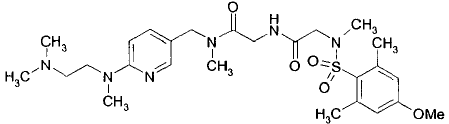 |
| (541) | 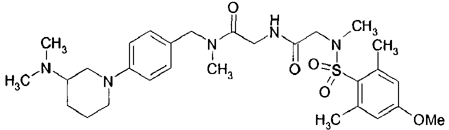 |
| (542) | 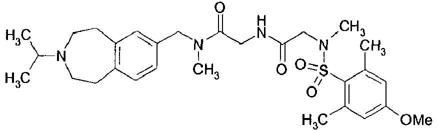 |
| (543) | 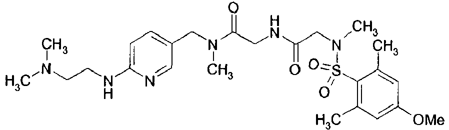 |
| (544) | 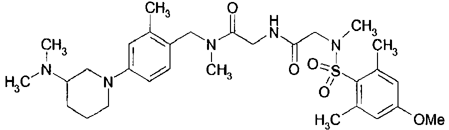 |
| (545) | 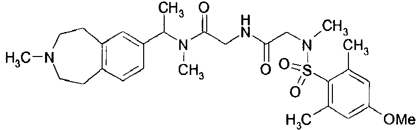 |
| (546) | 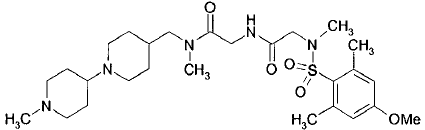 |
| (547) | 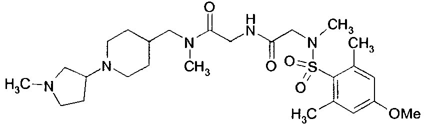 |
| (548) | 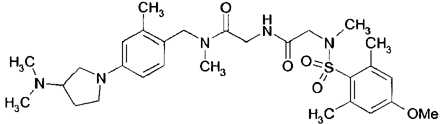 |
| (549) | 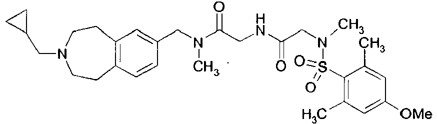 |
| (550) | 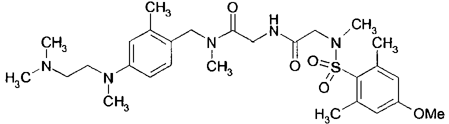 |
| (551) | 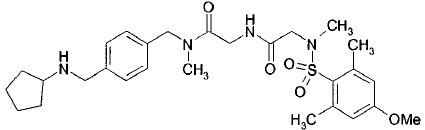 |
| (552) | 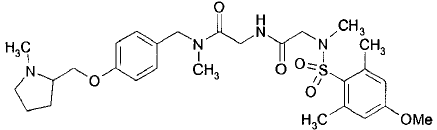 |
| (553) | 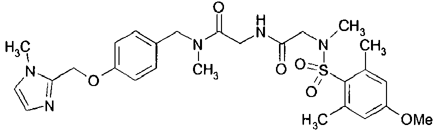 |
| (554) | 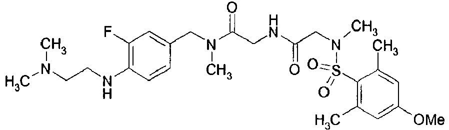 |
| (555) | 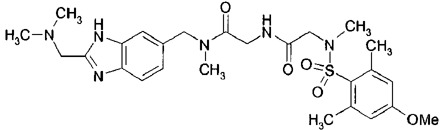 |
| (556) | 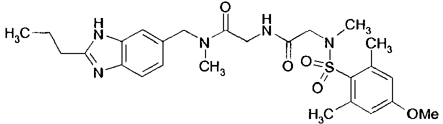 |
| (557) | 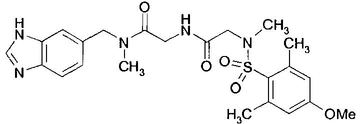 |
| (558) | 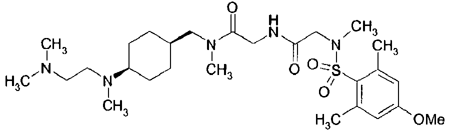 |
| (559) | 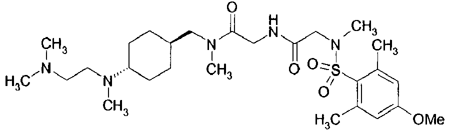 |
| (560) | 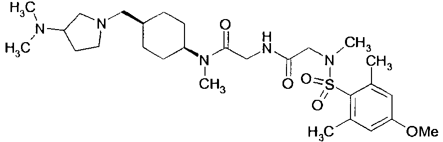 |
| (561) | 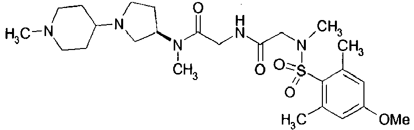 |
| (562) | 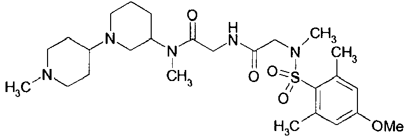 |
| (563) | 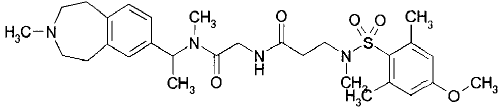 |
| (564) | 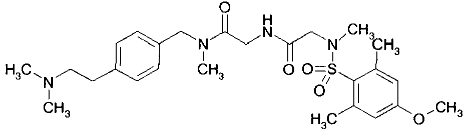 |
| (565) | 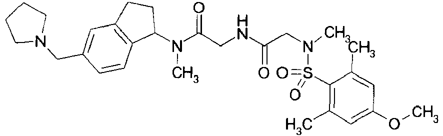 |
| (566) | 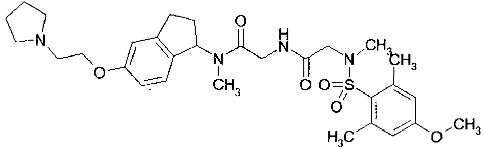 |
| (603) | 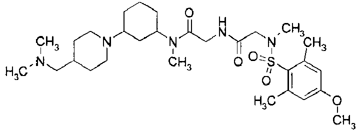 |
| (604) | 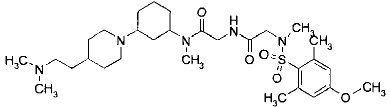 |
| (605) | 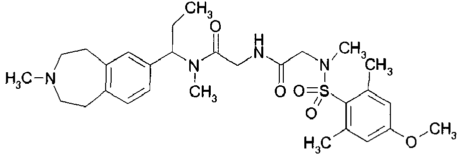 |
| (631) | 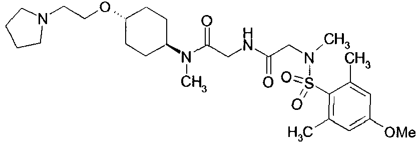 |
| (632) | 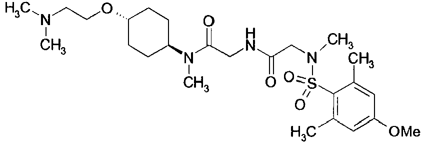 |
| (633) | 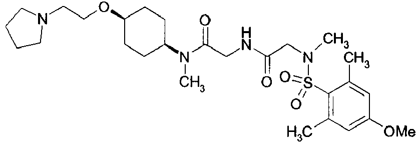 |
| (634) | 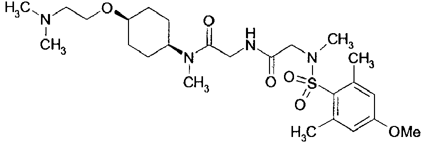 |

deren Enantiomere, deren Diastereomere, deren Gemische und deren Salze, insbesondere deren physiologisch verträgliche Salze mit organischen oder anorganischen Säuren oder Basen.

### VERWENDETE BEGRIFFE UND DEFINITIONEN

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Verbindungen der allgemeinen Formel **I** können, sofern sie geeignete basische Funktionen enthalten, beispielsweise Aminogruppen, insbesondere für pharmazeutische Anwendungen in ihre physiologisch verträglichen Salze mit anorganischen oder organischen Säuren übergeführt werden. Als anorganische Säuren kommen hierfür beispielsweise Bromwasserstoffsäure, Phosphorsäure, Salpetersäure, Salzsäure, Schwefelsäure, Methansulfonsäure, Ethansulfonsäure, Benzolsulfonsäure oder *p*-Toluolsulfonsäure in Frage, als organische Säuren kommen beispielsweise Äpfelsäure, Bernsteinsäure, Essigsäure, Fumarsäure, Maleinsäure, Mandelsäure, Milchsäure, Weinsäure oder Zitronensäure in Betracht. Weiterhin können gegebenenfalls im Molekül vorhandene tertiäre Aminogruppen quarternisiert werden. Zur Umsetzung werden dabei Alkylhalogenide eingesetzt. Erfindungsgemäß bevorzugt wird für die Quartenisierung Methyliodid verwendet.

Weiterhin lassen sich die Verbindungen der allgemeinen Formel **I,** sofern sie geeignete Carbonsäurefunktionen enthalten, gewünschtenfalls in ihre Additionssalze mit anorganischen oder organischen Basen überführen. Als anorganische Basen kommen hierfür beispielsweise Alkali- oder Erdalkalimetallhydroxide, beispielsweise Natriumhydroxid oder Kaliumhydroxid, oder Carbonate, Ammoniak, Zink- oder Ammoniumhydroxide in Frage; als organische Amine kommen beispielsweise Diethylamin, Triethylamin, Ethanolamin, Diethanolamin, Triethanolamin, Cyclohexylamin oder Dicyclohexylamin in Betracht.

Die erfindungsgemäßen Verbindungen können als Racemate vorliegen, sofern sie nur ein Chiralitätselement besitzen, sie können aber auch als reine Enantiomere, d.h. in (*R*)- oder (S)-Form gewonnen werden.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel **I** vorhanden ist, sowie die einzelnen optisch aktiven Enatiomeren, aus denen sich die erwähnten Racemate zusammensetzen.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säure-additionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

### Methodenbeschreibung zur hBK1-Rezeptorbindung

CHO-Zellen, die den hBK1-Rezeptor exprimieren, werden in "Dulbecco's modified Medium" kultiviert. Von konfluenten Kulturen wird das Medium entfernt, die Zellen werden mit PBS-Puffer gewaschen, abgeschabt und durch Zentrifugieren isoliert. Anschließend werden die Zellen in Suspension homogenisiert, das Homogenat zentrifugiert und resuspendiert. Nach Bestimmung des Proteingehalts wird die so erhaltene Membranpräparation bei -80°C eingefroren.

Nach dem Auftauen werden 200 µl des Homogenats (50 bis100 µg Protein/Assay) für 60 Minuten bei Raumtemperatur mit 0.5 bis 1.0 nM Kallidin (DesArg10,Leu9), [3,4-Prolyl-3,43H(N)] und ansteigenden Konzentrationen der Testsubstanz in einem Gesamtvolumen von 250 µl inkubiert. Die Inkubation wird durch rasche Filtration durch GF/B-Glasfaserfilter, die mit Polyethyleneimine (0.3%) vorbehandelt wurden, beendet. Die an das Protein gebundene Radioaktivität wird mit einem TopCount NXT gemessen. Als nichtspezifische Bindung wird die gebundene Radioaktivität in Gegenwart von 1.0 µM Kallidin (DesArg10,Leu9), [3,4-Prolyl-3,43H(N)] definiert. Die Analyse der Konzentrations-Bindungskurve erfolgt mit Hilfe einer computergestützten nichtlinearen Kurvenanpassung. Aus den so erhaltenen Daten wird für die Testsubstanz der entsprechende Kᵢ - Wert ermittelt.

Um zu zeigen, dass die Verbindungen der allgemeinen Formel **I** mit unterschiedlichen Strukturelementen gute bis sehr gute Bradikinin-B1-Rezeptor antagonistische Aktivitäten zeigen, werden in der folgenden Tabelle die Kᵢ-Werte angegeben, die gemäß dem voranstehenden Versuchsprotokoll erhalten wurden. Dazu wird angemerkt, dass die Verbindungen im Hinblick auf ihre unterschiedlichen strukturellen Elemente ausgewählt wurden und nicht um spezifische Verbindungen hervorzuheben:

| **Beispiel** | **Kᵢ [nM]** |
|---|---|
| (492) | 3.2 |

### Indikationen

Aufgrund ihrer pharmakologischen Eigenschaften eignen sich die neuen Verbindungen und deren physiologisch verträglichen Salze zur Behandlung von Krankheiten und Krankheitssymptomen, die wenigstens teilweise durch die Stimulierung von Bradykinin-B1-Rezeptoren hervorgerufen werden.

Aufgrund ihrer pharmakologischen Wirkung eignen sich die Substanzen zur Behandlung
(a) von **akuten Schmerzen** wie z.B. Zahnschmerzen, peri- und postoperativen Schmerzen, traumatischen Schmerzen, Muskelschmerzen, Verbrennungsschmerzen, Schmerzen bei Sonnenbrand, Trigeminusneuralgie, Schmerzen bei Koliken, sowie bei Spasmen des Magen-Darm-Trakts oder des Uterus;
(b) von **Eingeweideschmerzen** wie z.B. chronischen Beckenschmerzen, gynäkologischen Schmerzen, Schmerzen vor und während der Menstruation, Schmerzen bei Pankreatitis, bei peptischen Ulzera, bei interstitieller Zystitis, bei Nierenkolik, bei Angina pectoris, Schmerzen bei Kolon irritabile, bei nicht-ulzeröser Dyspepsie und bei Gastritis, nicht-kardialen Thoraxschmerzen und Schmerzen bei myokardialer Ischämie und Herzinfarkt;
(c) von **neuropathischen Schmerzen** wie z.B. schmerzhaften Polyneuropathien, Schmerzen bei diabetischer Neuropathie, AIDS-assoziierten neuropathischen Schmerzen, Schmerzen bei Lumbago, bei nicht-herpesassoziierter Neuralgie, bei Post-zoster Neuralgie, bei Nervenverletzungen, bei Schädel-Hirn-Trauma, Schmerzen bei Nervenchädigungen infolge von Toxinen oder Chemotherapie, Phantomschmerzen, Schmerzen bei multipler Sklerose, bei Nervenwurzelabriß und schmerzhaften traumatisch bedingten Einzelnervenschädigungen;
(d) von **entzündlichen / Schmerzrezeptor-vermittelten Schmerzen** im Zusammenhang mit Erkrankungen wie Osteoarthritis, rheumatoider Arthritis, rheumatischem Fieber, Tendo-Synovitis, Sehnenentzündungen, Gicht, Vulvodynie, Schädigungen und Erkrankungen der Muskeln und Faszien (muskuläre Verletzungen, Fibromyalgie), Osteoarthritis, juveniler Arthritis, Spondylitis, Gicht-Arthritis, Psoriasis-Arthritis, Fibromyalgie, Myositis, Migräne, Zahnerkrankungen, Influenza und anderen Virusinfektionen wie Erkältungen, systemischer Lupus erythematodes,
(e) von **Tumorschmerzen** im Zusammenhang mit Krebserkrankungen wie lymphatischer oder myeloischer Leukämie, Hodgkin Erkrankung, Non-Hodgkin Lymphomen, Lymphogranulomatose, Lymphosarkomen, soliden malignen Tumoren und ausgedehnten Metastasen;
(f) von **Kopfschmerz-Erkrankungen** wie z.B. Kopfschmerzen unterschiedlicher Ursache, Cluster-Kopfschmerz, Migräne (mit oder ohne Aura) und Spannungskopfschmerz. Weiterhin eigenen sich die Verbindungen zur Behandlung
(g) von entzündlichen Veränderungen im Zusammenhang mit Erkrankungen der Atemwege wie Asthma bronchiale, einschließlich allergischem Asthma (atopisch und nicht-atopisch) sowie Bronchospasmen bei Anstrengung, beruflich-bedingtem Asthma, viraler oder bakterieller Exazerbation einer bestehenden Asthma-Erkrankung und anderen nichtallergisch bedingten asthmatischen Erkrankungen; chronisch obstruktiver LungenErkrankung (COPD) einschließlich Lungenemphysem, akutem Atemnotsyndrom des Erwachsenen (ARDS), Bronchitis, Lungenentzündung, allergischer Rhinitis (saisonal und ganzjährig), vasomotorischer Rhinitis und Staublungen-Erkrankungen wie Aluminose, Anthrakose, Asbestose, Chalikose, Siderose, Silikose, Tabakose und Byssinose;
(h) von Entzündungserscheinungen bei Sonnenbrand und Verbrennungen, Ödemen nach Traumata durch Verbrennungen, Hirnödemen und Angioödemen, Darmerkrankungen einschließlich Morbus Crohn und Kolitis ulzerosa, Reizdarmsyndrom, Pankreatitis, Nephritis, Zystitis (interstitielle Zystitis), Uveitis; entzündlichen Erkrankungen der Haut (wie z.B. Psoriasis und Ekzeme), vaskulären Bindegewebserkrankungen, Lupus, Zerrungen und Frakturen;
(i) von Diabetes Mellitus und dessen Folgen (wie z.B. diabetische Vaskulopathie, diabetische Neuropathie, diabetische Retinopathie) und von diabetischen Symptomen bei Insulitis (z.B. Hyperglycämie, Diurese, Proteinurie und erhöhte renale Nitrit- und Kallikrein-Exkretion);
(j) von neurodegenerativen Erkrankungen wie der Parkinson'schen Erkrankung und der Alzheimer Erkrankung;
(k) von Sepsis und septischem Schock nach bakteriellen Infektionen oder nach Trauma;
(l) von Juckreiz verursachenden Syndromen und allergischen Hautreaktionen;
(m) von Osteoporose;
(n) von Epilepsie;
(o) von Verletzungen des Zentralnervensystems;
(p) von Wunden und Gewebeschädigungen;
(q) von Zahnfleischentzündungen;
(r) von benigner Prostata-Hyperplasie und hyperaktiver Blase;
(s) von Pruritus;
(t) von Vitiligo;
(u) von Störungen der Motilität von respiratorischen, genito-urinalen, gastro-intestinalen oder vaskulären Regionen und
(v) von post-operativem Fieber.

Neben der Eignung als Humantherapeutika, sind diese Substanzen auch nützlich in der veterinärmedizinischen Therapie von Haustieren, exotischen Tieren und Nutztieren.

Zur Behandlung von Schmerzen kann es vorteilhaft sein, die erfindungsgemässen Verbindungen mit belebenden Stoffen wie Koffein oder anderen schmerzlindernden Wirkstoffen zu kombinieren. Stehen zur Behandlung der Ursache der Schmerzen geeignete Wirkstoffe zur Verfügung, so können diese mit den erfindungsgemässen Verbindungen kombiniert werden. Sind unabhängig von der Schmerzbehandlung auch noch weitere medizinische Behandlungen angezeigt, zum Beispiel gegen Bluthochdruck oder Diabetes, so können auch die dafür nötigen Wirkstoffe mit den erfindungsgemässen Verbindungen kombiniert werden.

Für eine Kombinationstherapie kommen beispielsweise die folgenden Verbindungen in Frage:
Nicht-steroidale Antirheumatika (NSAR): COX-2 Hemmer wie Propionsäure-Derivate (Alminoprofen, Benoxaprofen, Bucloxinsäure, Carprofen, Fenhufen, Fenoprofen, Fiuprofen, Fiulbiprofen, Ibuprofen, Indoprofen, Ketoprofen, Miroprofen, Naproxen, Oxaprozin, Pirprofen, Pranoprofen, Suprofen, Tiaprofensäure, Tioxaprofen), Essigsäure-Derivate (Indomethacin, Acemetacin, Alcofenac, Isoxepac, Oxpinax, Sulindac, Tiopinac, Tolmetin, Zidometacin, Zomepirac) Fenaminsäure-Derivate (Meclofenaminsäure, Mefenaminsäure, Tolfenaminsäure), Biphenyl-Carboxylsäure-Derivate, Oxicame (Isoxicam, Meloxicam, Piroxicam, Sudoxicam und Tenoxicam), Salicylsäure-Derivate (Acetylsalicylsäure, Sulfasalazin, warum nicht auch Mesalazin, Olsalazin, und Pyrazolone (Apazon, Bezpiperylon, Feprazon, Mofebutazon, Oxyphenbutazon, Phenylbutazon, warum nicht auch Propyphenazon und Metamizol, und Coxibe (Celecoxib, Valecoxib, Rofecoxib, Etoricoxib).

Opiat Rezeptor Agonisten wie z. B. Morphin, Propoxyphen (Darvon), Tramadol, Buprenorphin.

Cannabinoid Agonisten wie z.B. GW-1000, KDS-2000, SAB-378, SP-104, NVP001-GW-843166, GW-842166X, PRS-211375.

Natriumkanalblocker wie z.B. Carbamazepin, Mexiletin, Lamotrigin, Pregabalin, Tectin, NW-1029, CGX-1002.

N-Typ Calciumkanalblocker wie z.B. Ziconitid, NMED-160, SP1-860.

Serotonerge und noradrenerge Modulatoren wie z.B. SR-57746, Paroxetin, Duloxetin, Clonidin, Amitriptylin, Citalopram.

Corticosteroide wie z.B. Betamethason, Budesonid, Cortison, Dexamethason, Hydrocortison, Methylprednisolon, Prednisolon, Prednison und Triamcinolon.

Histamin H1-Rezeptorantagonisten wie z.B. Bromophtniramint, Chlorpheniramin, Dexchlorpheniramin, Triprolidin, Clemastin, Diphenhydramin, Diphenylpyralin, Tripelennamin, Hydroxyzin, Methdijazin, Promethazin, Trimeprazin Azatadin, Cyproheptadin, Antazolin, Pheniramin, Pyrilamin, Astemizol, Terfenadin, Loratadin, Cetirizin, Desloratadin, fexofenadin, Levocetirizin.

Histamin H2-Rezeptor Antagonisten wie z.B. Cimetidin, Famotidin, und Ranitidin. Protonenpumpenhemmer wie z.B. Omeprazol, Pantoprazol, Esomeprazol.

Leukotrien Antagonisten und 5-Lipoxygenasehemmer wie z.B. Zafirlukast, Montelukast, Pranlukast und Zileuton.

Lokalanästhetika wie z.B. Ambroxol, Lidocain.

VR1 Agonisten und Antagonisten wie z.B. NGX-4010, WL-1002, ALGRX-4975, WL-10001, AMG-517.

Nikotinrezeptor Agonisten wie z.B. ABT-202, A-366833, ABT-594, BTG-102, A-85380, CGX1204.

P2X3-Rezeptor Antagonisten wie z.B. A-317491, ISIS-13920, AZD-9056.

NGF Agonisten und Antagonisten wie z.B. RI-724, RI-1024, AMG-819, AMG-403, PPH 207. NK1 und NK2 Antagonisten wie z.B. DA-5018, R-116301, CP-728663, ZD-2249.

NMDA Antagonisten wie z.B. NER-MD-11, CNS-5161, EAA-090, AZ-756, CNP-3381. Kaliumkanal Modulatoren wie z.B. CL-888, ICA-69673, Retigabin.

GABA Modulatoren wie z.B. Lacosamid.

Serotonerge und noradrenerge Modulatoren wie z.B. SR-57746, Paroxetin, Duloxetin, Clonidin, Amitriptylin, Citalopram, Flibanserin.

Anti-Migräne Therapeutika wie z.B. Sumatriptan, Zolmitriptan, Naratriptan, Eletriptan.

Die zur Erzielung einer schmerzstillenden Wirkung erforderliche Dosierung beträgt bei intravenöser Gabe zweckmäßigerweise 0.01 bis 3 mg/kg Körpergewicht, vorzugsweise 0.1 bis 1 mg/kg, und bei oraler Gabe 0.1 bis 8 mg/kg Körpergewicht, vorzugsweise 0.5 bis 3 mg/kg, jeweils 1 bis 3 x täglich. Die erfindungsgemäß hergestellten Verbindungen können intravenös, subkutan, intramuskulär, intrarektal, intranasal, durch Inhalation, transdermal oder oral verabreicht werden, wobei zur Inhalation insbesondere Aerosolformulierungen geeignet sind. Sie können gegebenenfalls zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden.

### EXPERIMENTELLER TEIL

Für die hergestellten Verbindungen liegen in der Regel IR-, ¹H-NMR und/oder Massenspektren vor. Die bei den Fliessmitteln angegebenen Verhältnisse beziehen sich auf Volumeneinheiten der jeweiligen Lösungsmittel. Die angegebenen Volumeneinheiten bei Ammoniak beziehen sich auf eine konzentrierte Lösung von Ammoniak in Wasser.

Soweit nicht anders vermerkt sind die bei den Aufarbeitungen der Reaktionslösungen verwendeten Säure-, Basen- und Salzlösungen wässrige Systeme der angegebenen Konzentrationen.

Zu chromatographischen Reinigungen wird Kieselgel der Firma Millipore (MATREX*^{™},* 35-70 µm) oder Alox (E. Merck, Darmstadt, Aluminiumoxid 90 standardisiert, 63-200 µm, Artikel-Nr: 1.01097.9050) verwendet.

In den Versuchsbeschreibungen werden die folgenden Abkürzungen verwendet:
- CDI: 1,1'-Carbonyldiimidazol
- DC: Dünnschichtchromatogramm
- DIPEA: Diisopropylethylamin
- DMAP: 4-Dimethylaminopyridin
- DMF: Dimethylformamid
- DMSO: Dimethylsulfoxid
- HATU: *O*-(7-Azabenzotriazol-1-yl)-*N,N,N',N*'-tetramethyluroniumhexafluorphosphat
- *tert*: tertiär
- TBTU: 2-(1*H*-Benzotriazol-1-yl)-1,1,3,3-tetramethyluronium-tetrafluorborat
- THF: Tetrahydrofuran

Folgende analytische HPLC-Methoden wurden verwendet:

| | | | | |
|---|---|---|---|---|
| Methode 1: | Säule: | XTerra™ MS C18, 2.5 µM, 4.6 x 30 mm | | |
| | Detektion: | 210 - 420 nm | | |
| | Fließmittel A: | Wasser / 0.1 % Ameisensäure | | |
| | Fließmittel B: | Acetonitril / 0.1 % Ameisensäure | | |
| | Gradient: | | | |

| | Zeit in min | %A | %B | Flussrate in mL/min |
|---|---|---|---|---|
| | 0.0 | 95.0 | 5.0 | 1.0 |
| | 0.1 | 95.0 | 5.0 | 1.0 |
| | 3.1 | 2.0 | 98.0 | 1.0 |
| | 4.5 | 2.0 | 98.0 | 1.0 |
| | 5.0 | 95.0 | 5.0 | 1.0 |
| | | | | |
| Methode 2: | Säule: | Microsorb C18, 3 µM, 4.6 x 50 mm | | |
| | Detektion: | 220 - 320 nm | | |
| | Fließmittel A: | Wasser / 0.1 % TFA | | |
| | Fließmittel B: | Acetonitril / 0.1 % TFA | | |
| | Gradient: | | | |

| | Zeit in min | %A | %B | Flussrate in mL/min |
|---|---|---|---|---|
| | 0.0 | 95.0 | 5.0 | 1.5 |
| | 0.5 | 95.0 | 5.0 | 1.5 |
| | 3.8 | 2.0 | 98.0 | 1.5 |
| | 4.3 | 2.0 | 98.0 | 1.5 |
| | 4.35 | 95.0 | 5.0 | 1.5 |
| | 4.6 | 95.0 | 5.0 | 1.5 |
| | | | | |
| Methode 3: | Säule: | XTerra™ MS C18, 3.5 µM, 4.6 x 50 mm | | |
| | Detektion: | 210 - 420 nm | | |
| | Fließmittel A: | Wasser / 0.1 % Ameisensäure | | |
| | Fließmittel B: | Acetonitril / 0.1 % Ameisensäure | | |
| | Gradient: | | | |
| | 0.0 | 95.0 | 5.0 | 1.0 |
| | 0.1 | 95.0 | 5.0 | 1.0 |
| | 7.1 | 2.0 | 98.0 | 1.0 |
| | 7.9 | 2.0 | 98.0 | 1.0 |
| | 8.0 | 95.0 | 5.0 | 1.0 |
| | | | | |
| Methode 4: | Säule: | Zorbax Stable Bond C18, 3.5 µM, 4.6 x 75 mm | | |
| | Detektion: | 230 - 360 nm | | |
| | Fließmittel A: | Wasser / 0.1 % Ameisensäure | | |
| | Fließmittel B: | Acetonitril / 0.1 % Ameisensäure | | |
| | Gradient: | | | |

| | Zeit in min | %A | %B | Flussrate in mL/min |
|---|---|---|---|---|
| | 0.0 | 95.0 | 5.0 | 1.6 |
| | 0.1 | 95.0 | 5.0 | 1.6 |
| | 4.5 | 10.0 | 90.0 | 1.6 |
| | 5.09 | 10.0 | 90.0 | 1.6 |
| | 5.5 | 90.0 | 10.0 | 1.6 |
| | | | | |
| Methode 5: | Säule: | Interchim Strategy C18, 5 µM, 4.6 x 50 mm | | |
| | Detektion: | 220 - 320 nm | | |
| | Fließmittel A: | Wasser / 0.1 % TFA | | |
| | Fließmittel B: | Acetonitril | | |
| | Gradient: | | | |

| | Zeit in min | %A | %B | Flussrate in mL/min |
|---|---|---|---|---|
| | 0.0 | 95.0 | 5.0 | 3.0 |
| | 0.3 | 95.0 | 5.0 | 3.0 |
| | 2.0 | 2.0 | 98.0 | 3.0 |
| | 2.4 | 2.0 | 98.0 | 3.0 |
| | 2.45 | 95.0 | 5.0 | 3.0 |
| | 2.8 | 95.0 | 5.0 | 3.0 |
| Methode 6: | Säule: | Merck Cromolith Speed ROD RP18e, 4.6 x 50 mm | | |
| | Detektion: | 190 - 400 nm | | |
| | Fließmittel A: | Wasser / 0.1 % Ameisensäure | | |
| | Fließmittel B: | Acetonitril / 0.1 % Ameisensäure | | |
| | Gradient: | | | |

| | Zeit in min | %A | %B | Flussrate in mL/min |
|---|---|---|---|---|
| | 0.0 | 90.0 | 10.0 | 1.5 |
| | 4.5 | 10.0 | 90.0 | 1.5 |
| | 5.0 | 10.0 | 90.0 | 1.5 |
| | 5.5 | 90.0 | 10.0 | 1.5 |
| | | | | |
| Methode 7: | Säule: | Waters SunFire C18, 3.5 µM, 4.6 x 50 mm | | |
| | Detektion: | 210 - 500 nm | | |
| | Fließmittel A: | Wasser / 0.1 % TFA | | |
| | Fließmittel B: | Acetonitril / 0.1 % TFA | | |
| | Gradient: | | | |

| | Zeit in min | %A | %B | Flussrate in mL/min |
|---|---|---|---|---|
| | 0.0 | 95.0 | 5.0 | 1.5 |
| | 2.0 | 2.0 | 98.0 | 1.5 |
| | 3.0 | 2.0 | 98.0 | 1.5 |
| | 3.4 | 95.0 | 5.0 | 1.5 |
| | | | | |
| Methode 8: | Säule: | Waters XBridge C18, 3.5 µM, 4.6 x 50 mm | | |
| | Detektion: | 210 - 500 nm | | |
| | Fließmittel A: | Wasser / 0.1 % TFA | | |
| | Fließmittel B: | Acetonitril / 0.1 % TFA | | |
| | Gradient: | | | |

| | Zeit in min | %A | %B | Flussrate in mL/min |
|---|---|---|---|---|
| | 0.0 | 95.0 | 5.0 | 1.0 |
| | 0.1 | 95.0 | 5.0 | 1.0 |
| | 5.1 | 2.0 | 98.0 | 1.0 |
| | 6.5 | 2.0 | 98.0 | 1.0 |
| | 7.0 | 95.0 | 5.0 | 1.0 |
| Methode 9: | Säule: | Merck Chromolith™ Flash RP18e, 4.6 x 25 mm | | |
| | Detektion: | 190 - 400 nm | | |
| | Fließmittel A: | Wasser / 0.1 % Ameisensäure | | |
| | Fließmittel B: | Acetonitril / 0.1 % Ameisensäure | | |
| | Gradient: | | | |

| | Zeit in min | %A | %B | Flussrate in mL/min |
|---|---|---|---|---|
| | 0.0 | 90.0 | 10.0 | 1.6 |
| | 2.7 | 10.0 | 90.0 | 1.6 |
| | 3.0 | 10.0 | 90.0 | 1.6 |
| | 3.3 | 90.0 | 10.0 | 1.6 |
| | | | | |
| Methode 10: | Säule: | Merck Chromolith™ Flash RP18e, 4.6 x 25 mm | | |
| | Detektion: | 210 - 400 nm | | |
| | Fließmittel A: | Wasser / 0.1 % TFA | | |
| | Fließmittel B: | Acetonitril / 0.1 % TFA | | |
| | Gradient: | | | |

| | Zeit in min | %A | %B | Flussrate in mL/min |
|---|---|---|---|---|
| | 0.0 | 95.0 | 5.0 | 2.5 |
| | 0.2 | 95.0 | 5.0 | 2.5 |
| | 1.5 | 2.0 | 98.0 | 2.5 |
| | 1.7 | 2.0 | 98.0 | 2.5 |
| | 1.9 | 95.0 | 5.0 | 2.5 |
| | 2.2 | 95.0 | 5.0 | 2.5 |
| | | | | |
| Methode 11: | Säule: | Waters XBridge C18, 3.5 µM, 4.6 x 50 mm | | |
| | Detektion: | 210 - 500 nm | | |
| | Fließmittel A: | Wasser / 0.1 % TFA | | |
| | Fließmittel B: | Acetonitril / 0.1 % TFA | | |
| | Gradient: | | | |

| | Zeit in min | %A | %B | Flussrate in mL/min |
|---|---|---|---|---|
| | 0.0 | 95.0 | 5.0 | 1.5 |
| | 2.0 | 0.0 | 100.0 | 1.5 |
| | 3.0 | 0.0 | 100.0 | 1.5 |
| | 3.4 | 95.0 | 5.0 | 1.5 |
| Methode 12: | Säule: | YMC-Pack ODS-AQ, 3.0 µM, 4.6 x 75 mm | | |
| | Detektion: | 230 - 360 nm | | |
| | Fließmittel A: | Wasser / 0.1 % Ameisensäure | | |
| | Fließmittel B: | Acetonitril / 0.1 % Ameisensäure | | |
| | Gradient: | | | |

| | Zeit in min | %A | %B | Flussrate in mL/min |
|---|---|---|---|---|
| | 0.0 | 95.0 | 5.0 | 1.6 |
| | 4.5 | 10.0 | 90.0 | 1.6 |
| | 5.0 | 10.0 | 90.0 | 1.6 |
| | 5.5 | 90.0 | 10.0 | 1.6 |

Folgende Mikrowellen-Apparatur wurde verwendet: Biotage EmrysOptimizer^{™}

### Herstellung der Endverbindungen

### Beispiel 1

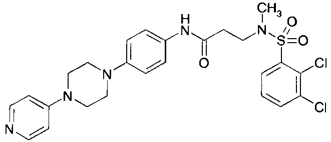

### 1 a)

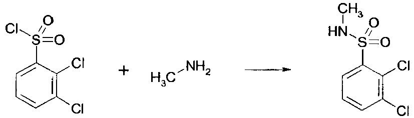

Eine Mischung aus 1.0 g (4.07 mMol) 2,3-Dichlorbenzolsulfonsäurechlorid, 0.33 g (4.89 mMol) Methylamin Hydrochlorid, 2.73 ml (19.55 mMol) Triethylamin und 20 ml Dichlormethan wird über Nacht bei Raumtemperatur gerührt. Die Reaktionsmischung wird anschließend je einmal mit 1 N HCl, gesättigter Natriumhydrogencarbonat-Lösung, Wasser und gesättigter Natriumchloridlösung gewaschen, über Natriumsulfat getrocknet und dann bis zur Trockne eingeengt. C₇H₇Cl₂NO₂S (240.11); [M+H]+ = 240/242/244.

DC: Kieselgel, Petrolether / Ethylacetat 2:1, Rf-Wert = 0.50

### 1 b)

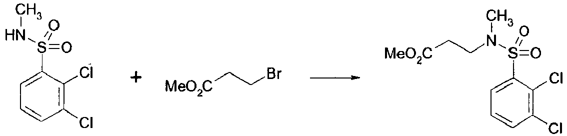

Eine Mischung aus 0.9 g (3.75 mMol) Produkt aus 1a und 20 ml DMF wird vorgelegt und mit 1.55 g (11.24 mMol) Kaliumcarbonat und 0.49 ml (4.50 mMol) 3-Brompropionsäureethylester versetzt. Die Reaktionsmischung wird über Nacht bei Raumtemperatur gerührt und dann mit Wasser versetzt. Man extrahiert zweimal mit Ethylacetat. Die organischen Extrakte werden dreimal mit Wasser und einmal mit gesättigter Natriumchloridlösung gewaschen, über Natriumsulfat getrocknet und zur Trockne eingeengt.

C₁₁H₁₃Cl₂NO₄S (326.20)

[M+H]+ = 326/328/330

DC: Kieselgel, Petrolether / Ethylacetat 2:1, Rf-Wert = 0.45

### 1c)

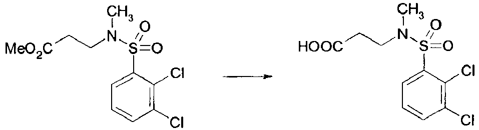

Eine Mischung aus 1.15 g (3.53 mMol) Produkt aus 1 b, 0.74 g (17.63 mMol) Lithiumhydroxid Monohydrat, 15 ml THF und 15 ml Wasser wird eine Stunde bei Raumtemperatur gerührt. Danach wird das THF im Vakuum entfernt und der Rückstand mit konzentrierter HCl sauer gestellt. Die Reaktionsmischung wird anschließend dreimal mit Ethylacetat extrahiert. Die organischen Extrakte werden mit gesättigter Natriumchloridlösung gewaschen, über Natriumsulfat getrocknet und zur Trockne eingeengt. Das Rohprodukt wird mit Diethylether verrieben und abgesaugt.

C₁₀H₁₁Cl₂NO₄S (312.17)

[M+H]+ = 310/312/314

DC: Kieselgel, Petrolether / Ethylacetat 2:1, Rf-Wert = 0.03

### 1d)

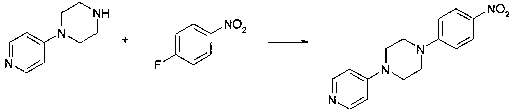

Eine Mischung aus 5.0 g (30.63 mMol) 1-Pyridin-4-yl-piperazin, 4.32 g (30.63 mMol) 1-Fluor-4-nitrobenzol (Aldrich), 10.62 ml (76.59 mMol) Triethylamin und 100 ml DMF wird 50 min unter Rückfluß erhitzt und anschließend bis zur Trockene eingeengt. Das so erhaltene Rohprodukt wird durch Säulenchromatographie über Kieselgel (Elutionsmittel: Dichlormethan / Ethanol / Ammoniak 12:1:0.1 bis 10:1:0.1) gereinigt. C₁₅H₁₆N₄O₂ (284.31); [M+H]+ = 285. DC: Kieselgel, Dichlormethan / Methanol / Ammoniak 9:1:0.1, Rf-Wert = 0.52

### 1 e)

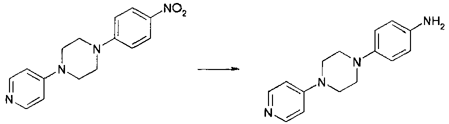

Eine Mischung aus 4.95 g (17.41 mMol) Produkt aus 1d, 0.6 g Palladium auf Kohle (10%), 120 ml Dichlormethan und 20 ml Methanol wird fünf Stunden im Autoklaven bei Raumtemperatur hydriert. Anschließend wird abgesaugt und der Filterkuchen noch sechsmal mit Dichlormethan / Methanol 1:1 aufgekocht und wieder abgesaugt. Die vereinigten Filtrate werden im Vakuum bis zur Trockene eingeengt.

C₁₅H₁₈N₄ (254.33)

[M+H]+ = 255

### 1 f)

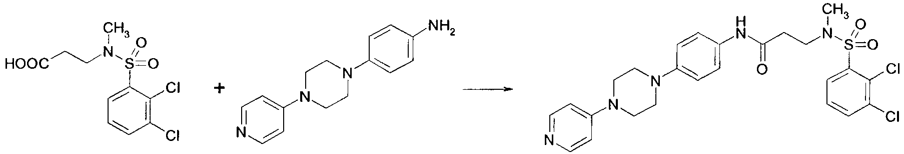

Eine Mischung aus 1.25 g (4.00 mMol) Produkt aus 1c, 2.0 ml (14.34 mMol) Triethylamin, 1.28 g (4.00 mMol) TBTU und 7 ml DMF wird 45 min bei Raumtemperatur gerührt. Danach wird 1.0 g (3.93 mMol) Produkt aus 1 e hinzugefügt und über Nacht weiter bei Raumtemperatur gerührt. Anschließend wird die Reaktionsmischung in Wasser geschüttet und mit Dichlormethan extrahiert. Die organischen Extrakte werden mit Wasser gewaschen, über Na₂SO₄ getrocknet und bis zur Trockene eingeengt. Das so erhaltene Rohprodukt wurde durch Säulenchromatographie über Kieselgel (Elutionsmittel: Dichlormethan mit 5-20 % Methanol) gereinigt.

C₂₅H₂₇Cl₂N₅O₃S (548.49); [M+H]+ = 548/550/552.

DC: Kieselgel, Dichlormethan / Methanol 4:1, Rf-Wert = 0.65

### Beispiel 10

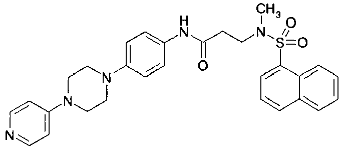

### 10a)

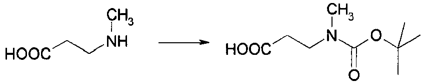

Eine Mischung aus 1.0 g (9.70 mMol) N-Methyl-β-alanin (Convertex), 24 ml Dioxan, 12 ml Wasser und 2.68 g (19.38 mMol) Kaliumcarbonat wasserfrei wird unter Eisbadkühlung mit 2.33 g (10.66 mMol) Boc-Anhydrid versetzt. Die Reaktionsmischung wird drei Tage bei Raumtemperatur gerührt. Danach wird das Dioxan im Vakuum entfernt. Der wässrige Rückstand wird mit Ethylacetat extrahiert (Ethylacetat-Phasen verwerfen), anschließend mit 1 M Salzsäure leicht sauer gestellt und dann mit Dichlormethan extrahiert. Die organischen Dichlormethan-Extrakte werden mit gesättigter Natriumchlorid-Lösung gewaschen, über Natriumsulfat getrocknet und bis zur Trockene eingeengt.

C₉H₁₇NO₄ (203.24)

[M+H]+ = 204

### 10b)

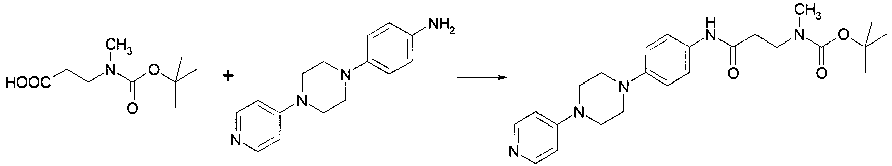

10b wird analog zu 1f aus 1.85 g (9.10 mMol) Produkt aus 10a, 2.32 g (9.10 mMol) Produkt aus 1 e, 3.81 ml (27.31 mMol) Triethylamin und 2.92 g (9.10 mMol) TBTU in 80 ml DMF hergestellt.

C₂₄H₃₃N₅O₃ (439.55)

[M+H]+ = 440

DC: Kieselgel, Dichlormethan / Methanol 9:1, Rf-Wert = 0.49

### 10c)

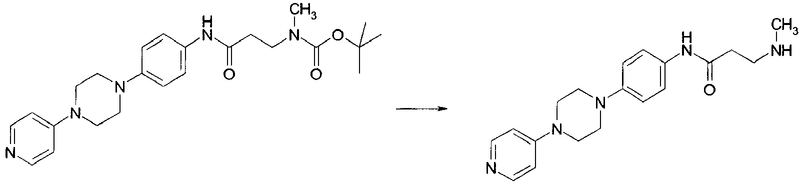

Eine Mischung aus 3.20 g (7.28 mMol) Produkt aus 10b, 20 ml TFA und 60 ml Dichlormethan wird 30 min bei Raumtemperatur gerührt. Danach wird die Reaktionsmischung im Vakuum bis zur Trockene eingeengt. Das so erhaltene Rohprodukt wird durch Säulenchromatographie über Kieselgel (Elutionsmittel: Dichlormethan / Ethanol / Ammoniak 9:1:0.1 bis 4:1:0.1) gereinigt.

C₁₉H₂₅N₅O (339.43)

[M+H]+ = 340

DC: Kieselgel, Dichlormethan / Methanol / Ammoniak 9:1:0.1, Rf-Wert = 0.25

### 10d)

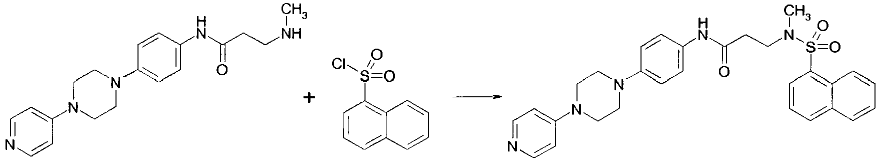

Eine Mischung aus 0.1 g (0.30 mMol) Produkt aus 10c, 0.056 g (0.25 mMol) 1-Naphthylsulfonsäurechlorid, 0.137 ml (0.98 mMol) Triethylamin und 5 ml Dichlormethan wird über Nacht bei Raumtemperatur gerührt. Danach wird die Reaktionsmischung im Vakuum bis zur Trockene eingeengt. Das so erhaltene Rohprodukt wird durch Säulenchromatographie über Kieselgel (Elutionsmittel: Dichlormethan / Ethanol / Ammoniak 12:1:0.1) gereinigt. C₂₉H₃₁N₅O₃S (529.65)

[M+H]+ = 530

DC: Kieselgel, Dichlormethan / Methanol / Ammoniak 9:1:0.1, Rf-Wert = 0.44

### Beispiel 13

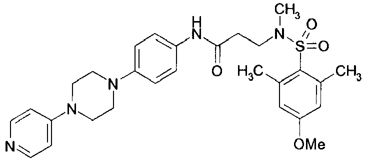

### 13a)

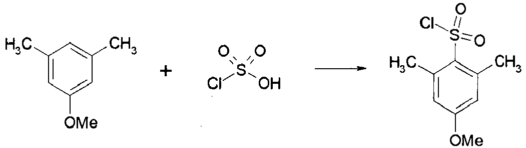

Eine Mischung aus 2.0 g (14.69 mMol) 3,5-Dimethylanisol und 20 ml Dichlormethan wird unter Eisbadkühlung mit 5.85 ml (88.0 mMol) Chlorsulfonsäure versetzt. Die Reaktionsmischung wird danach 20 min bei Raumtemperatur gerührt und anschließend auf 50 ml Eiswasser gegossen. Man extrahiert mit 100 ml Dichlormethan. Die organischen Extrakte werden mit 5%iger Natriumhydrogencarbonat-Lösung gewaschen, über Natriumsulfat getrocknet und bis zur Trockene eingeengt.

C₉H₁₁ClO₃S (234.70)

[M+H]+ = 234/236

DC: Kieselgel, Petrolether / Ethylacetat 9:1, Rf-Wert = 0.46

### 13b)

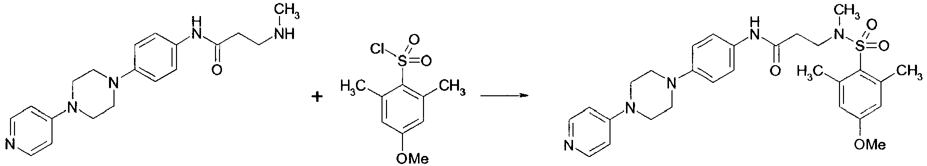

Beispiel 13 wird analog zu 10d aus 0.10 g (0.30 mMol) Produkt aus 10c, 0.058 g (0.25 mMol) Produkt aus 13a, 0.14 ml (98 mMol) Triethylamin in 5 ml Dichlormethan hergestellt. C₂₈H₃₅N₅O₄S (537.67)

[M+H]+ = 538

DC: Kieselgel, Dichlormethan / Methanol / Amoniak 9:1:0.1, Rf-Wert = 0.62

### Beispiel 18

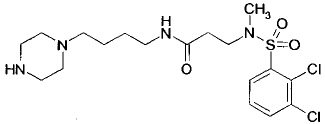

### 18a)

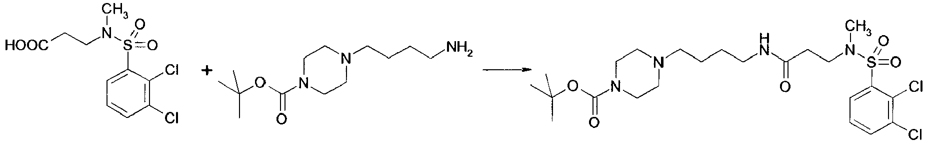

18a wird analog zu 1f aus 0.20 g (0.64 mMol) Produkt aus 1 c, 0.17 g (0.64 mMol) 4-(4-Aminobutyl)-piperazin-1-carbonsäure-tert-butylester (J. Med. Chem. 47, 2004, 4300-4315), 0.27 ml (1.92 mMol) Triethylamin und 0.21 g (0.64 mMol) TBTU in 5 ml THF hergestellt.

C₂₃H₃₆CL₂N₄O₅S (551.53)

M+H]+ = 551/553/555

DC: Kieselgel, Dichlormethan / Methanol 30:1, Rf-Wert = 0.1

### 18b)

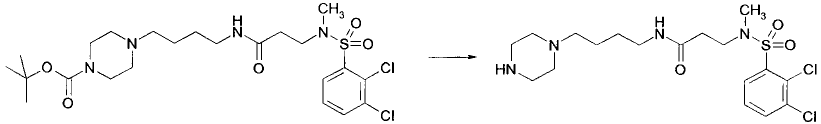

Eine Mischung aus 0.29 g (0.53 mMol) Produkt aus 18a, 0.53 ml TFA und 1 ml Dichlormethan wird zwei Stunden bei Raumtemperatur gerührt. Die Reaktionsmischung wird mit gesättigter Natriumhydrogencarbonat-Lösung gewaschen. Nach der Phasentrennung wird die wässrige Phase noch dreimal mit Dichlormethan extrahiert. Die organischen Extrakte werden über Natriumsulfat getrocknet und bis zur Trockene im Vakuum eingeengt. C₁₈H₂₈Cl₂N₄O₃S (451.41)

[M+H]+ = 451/453/455

HPLC (Methode 2): Retentionszeit = 2.22 min

### Beispiel 22

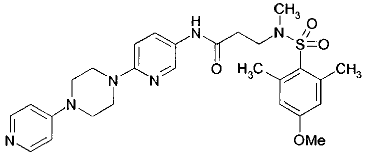

### 22a)

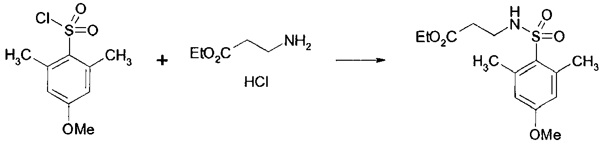

22a wird analog zu 3a aus 3.00 g (12.78 mMol) Produkt aus 13a, 2.16 g (14.06 mMol) β-Alaninethylester Hydrochlorid, 7.13 ml (51.13 mMol) Triethylamin in 70 ml Dichlormethan hergestellt.

C₁₄H₂₁NO₅S (315.39)

[M+H]+ = 316

DC: Kieselgel, Petrolether / Ethylacetat 2:1, Rf-Wert = 0.23

### 22b)

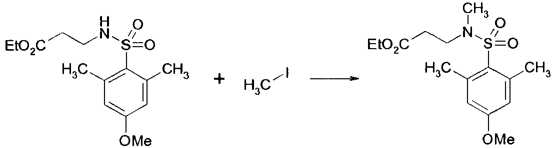

22b wird analog zu 3b aus 4.06 g (12.87 mMol) Produkt aus 22a, 2.40 ml (38.62 mMol) Methyliodid, 3.56 g (25.75 mMol) Kaliumcarbonat wasserfrei in 40 ml DMF hergestellt. C₁₅H₂₃NO₅S (329.41)

[M+H]+ = 330

DC: Kieselgel, Petrolether / Ethylacetat 2:1, Rf-Wert = 0.36

### 22c)

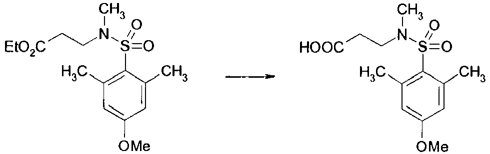

Die Säure wird analog zu 1 c aus 3.83 g (11.63 mMol) Produkt aus 22b, 2.44 g (58.13 mMol) Lithiumhydroxid Monohydrat in 30 ml THF und 30 ml Wasser hergestellt.

C₁₃H₁₉NO₅S (301.36)

[M+H]+ = 302

DC: Kieselgel, Petrolether / Ethylacetat 1:1, Rf-Wert = 0.12

### 22d)

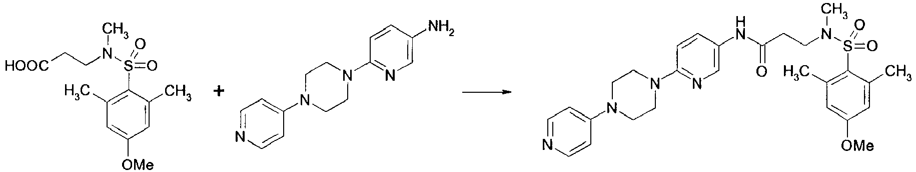

Beispiel 22 wird analog zu 1f aus 0.13 g (0.42 mMol) Produkt aus 22c, 0.089 g (0.35 mMol) Produkt aus 21b, 0.098 ml (0.70 mMol) Triethylamin und 0.13 g (0.42 mMol) TBTU in 15 ml THF hergestellt.

C₂₇H₃₄N₆O₄S (538.66)

[M+H]+ = 539

HPLC (Methode 4): Retentionszeit = 2.6 min

### Beispiel 28

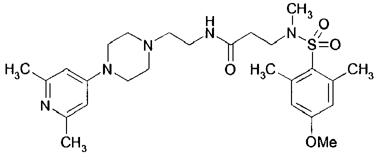

### 28a)

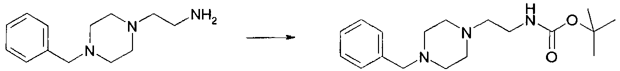

Eine Mischung aus 1.50 g (6.84 mMol) 1-(2-Aminoethyl)-4-benzylpiperazin (Maybridge), 1.64 g (7.52 mMol) Boc-Anhydrid und 30 ml Dichlormethan wird eine Stunde bei Raumtemperatur gerührt. Danach wird die Reaktionslösung mit 100 ml Dichlormethan verdünnt und mit 1 M Natronlauge und Wasser gewaschen. Die organische Phase wird über Natriumsulfat getrocknet und im Vakuum bis zur Trockene eingeengt.

C₁₈H₂₉N₃O₂ (319.44)

HPLC (Methode 4): Retentionszeit = 2.6 min

28b)

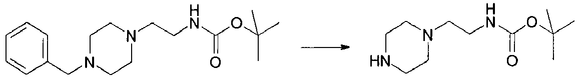

Eine Mischung aus 2.10 g (6.57 mMol) Produkt aus 28a, 0.25 g Palladium auf Kohle (10%) und 30 ml Methanol wird 15 Stunden bei Raumtemperatur im Autoklaven hydriert. Anschließend wird der Katalysator abgesaugt und das Filtrat im Vakuum bis zur Trockene eingeengt. C₁₁H₂₃N₃O₄ (229.32); [M+H]+ = 230.

DC: Kieselgel, Dichlormethan / Ethanol 9:1, Rf-Wert = 0.08

### 28c)

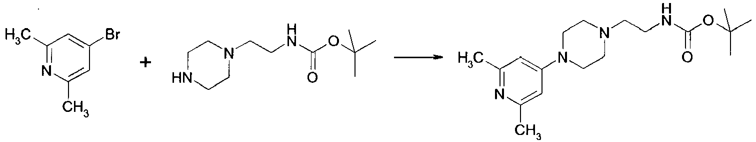

Eine Mischung aus 1.18 g (6.32 mMol) 4-Brom-2,6-dimethylpyridin (Acta Chem. Scand. Ser. B 42, 1988, 373-377), 1.45 g (6.32 mMol) Produkt aus 28b und 2.2 ml DIPEA wird 50 min auf 130°C in der Mikrowelle erhitzt. Man versetzt die Reaktionsmischung mit Ethylacetat und halbgesättigter Kaliumcarbonat-Lösung und trennt danach die Phasen. Die organische Phase wird über Natriumsulfat getrocknet und im Vakuum bis zur Trockene eingeengt. Das so erhaltene Rohprodukt wird durch Säulenchromatographie über Kieselgel (Elutionsmittel: Dichlormethan / Methanol / Ammoniak 95:5:0.5) gereinigt.

C₁₈H₃₀N₄O₂S (334.46)

[M+H]+ = 335

DC: Kieselgel, Dichlormethan / Methanol / Ammoniak 9:1:0.1, Rf-Wert = 0.37

### 28d)

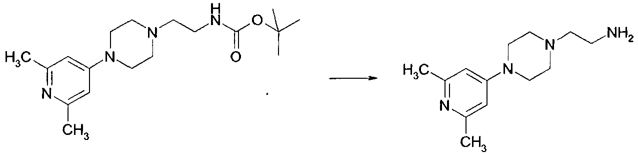

Eine Mischung aus 1.61 g (4.81 mMol) Produkt aus 28c, 3.70 ml TFA und 30 ml Dichlormethan wird sechs Stunden bei Raumtemperatur gerührt. Die Reaktionsmischung wird anschließend mit Dichlormethan verdünnt und mit 5%iger Natriumhydrogencarbonat-Lösung gewaschen. Die wässrige Phase wird noch zweimal mit Ethylacetat extrahiert. Die vereinigten organischen Phasen werden über Natriumsulfat getrocknet und im Vakuum bis zur Trockene eingeengt. Das so erhaltene Rohprodukt wird durch Säulenchromatographie über Kieselgel (Elutionsmittel: Dichlormethan / Methanol / Ammoniak 90:10:1) gereinigt. C₁₃H₂₂N₄ (234.34)

[M+H]+ = 235

DC: Kieselgel, Dichlormethan / Methanol / Ammoniak 9:1:0.1, Rf-Wert = 0.10

### 28e)

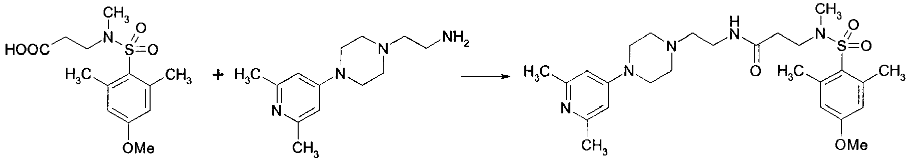

Beispiel 28 wird analog zu 1f aus 0.11 g (0.35 mMol) Produkt aus 22c, 0.082 g (0.35 mMol) Produkt aus 28d, 0.098 ml (0.70 mMol) Triethylamin und 0.13 g (0.42 mMol) TBTU in 7 ml THF und 1 ml DMF hergestellt.

C₂₆H₃₉N₅O₄S (517.69)

[M+H]+ = 518

HPLC (Methode 4): Retentionszeit = 2.4 min

### Beispiel 33

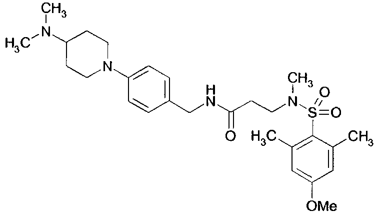

### 33a)

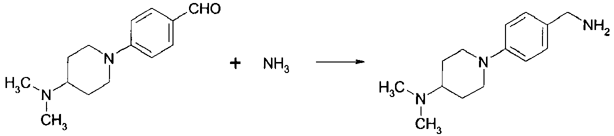

Eine Mischung aus 0.68 g (2.91 mMol) 4-(4-Dimethylamino-piperidin-1-yl)-benzaldehyd (Tetrahedron 57, 2001, 4781-4785), 15 ml 2 M Ammoniak in Ethanol und 0.10 g Raney-Nickel wird bei Raumtemperatur im Autoklaven hydriert. Danach wird der Katalysator abfiltriert und das Filtrat im Vakuum bis zur Trockene eingeengt. C₁₄H₂₃N₃ (233.35)

### 33b)

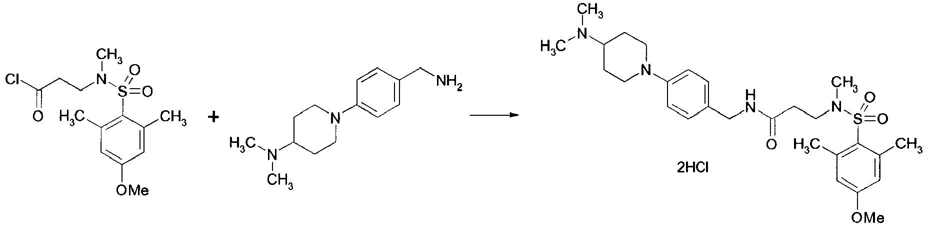

Beispiel 33 wird analog zu 27d aus 0.27 g (0.84 mMol) Produkt aus 27c, 0.63 g (2.68 mMol) Produkt aus 33a, 0.22 ml (1.26 mMol) DIPEA in 3 ml Dichlormethan hergestellt. C₂₇H₄₀N₄O₄S x 2HCl (589.62)

[M+H]+ = 517

HPLC (Methode 5): Retentionszeit = 1.46 min

### Beispiel 38

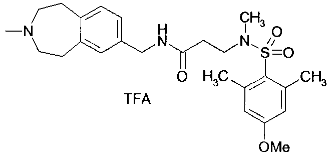

### 38a)

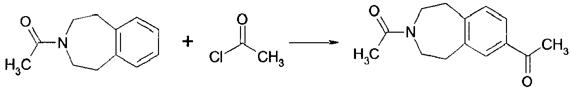

Eine Mischung aus 4.44 g (33.29 mMol) Aluminiumchlorid (Merck) und 16 ml Dichlorethan wird vorgelegt und unter Eisbadkühlung langsam mit 1.24 ml (17.44 mMol) Acetylchlorid (Aldrich) versetzt. Man lässt 30 min bei Raumtemperatur rühren. Anschließend werden 3.00 g (15.85 mMol) 1-(1,2,4,5-Tetrahydrobenzo[d]azepin-3-yl)-ethanon (J. Med.Chem. 46, 2003, 4952-4964) in 7 ml Dichlorethan langsam zur Reaktionsmischung gegeben. Nach zweistündigem Rühren bei Raumtemperatur wird die Reaktionsmischung auf eine Mischung aus 6 M HCl und Eis gegossen. Nach der Phasentrennung wird die wässrige Phase noch dreimal mit Dichlormethan extrahiert. Die vereinigten organischen Phasen werden mit Wasser gewaschen, über Natriumsulfat getrocknet und im Vakuum bis zur Trockene eingeengt. Das so erhaltene Rohprodukt wird mit Diethylether verrieben, abfiltriert und getrocknet. C₁₄H₁₇NO₂ (231.29); [M+H]+ = 232.

### 38b)

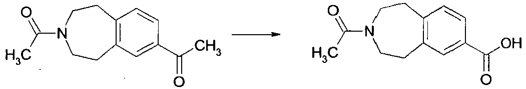

Eine Mischung aus 2.86 g (12.37 mMol) Produkt aus 38a und 79 ml 2.5 M Natronlauge wird bei Raumtemperatur langsam mit 2.48 ml (48.23 mMol) Brom versetzt. Die Reaktionsmischung wird danach eine Stunde bei Raumtemperatur gerührt. Der entstandene Niederschlag wird abfiltriert und das Filtrat mit MTB-Ether extrahiert. Die wässrige Phase wird anschließend unter Eisbadkühlung mit konzentrierter HCl und wenig Natriumdisulfit-Lösung versetzt. Der entstandene Niederschlag wird abfiltriert und im Umlufttrockenschrank bei 30°C getrocknet. C₁₃H₁₅NO₃ (233.26); [M+H]+ = 234.

### 38c)

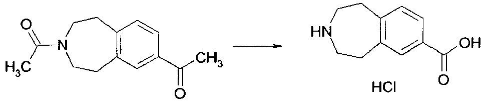

Eine Mischung aus 2.12 g (9.09 mMol) Produkt aus 38b und 20 ml 6 M HCl wird 3.5 Tage auf 100°C erhitzt. Die Reaktionsmischung wird danach mit einer Mischung aus Eis und Ethanol gekühlt. Der entstandene Niederschlag wird abfiltriert, mit wenig gekühltem Aceton und Diethylether gewaschen und im Exsikkator getrocknet.

C₁₁H₁₃NO₂ x HCl (227.69)

[M+H]+ = 192

### 38d)

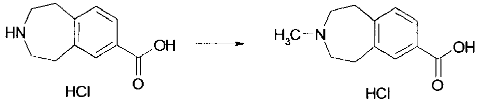

Eine Mischung aus 2.03 g (8.92 mMol) Produkt aus 38c und 3.36 ml (89.16 mMol) Ameisensäure wird erst langsam mit 0.94 ml (17.83 mMol) 50%iger Natronlauge, dann mit 2.66 ml (35.66 mMol) 37%iger Formalin-Lösung versetzt. Die Reaktionsmischung wird zwei Stunden auf 70°C erhitzt und danach im Vakuum bis zur Trockene eingeengt. Der Rückstand wird mit Wasser und konzentrierter HCl versetzt und nochmals bis zur Trockene eingeengt. Das Rohprodukt wird mit wenig eiskaltem Wasser verrieben, abfiltriert und im Umlufttrockenschrank bei 60°C getrocknet.

C₁₂H₁₅NO₂ x HCl (241.71); [M+H]+ = 206.

### 38e)

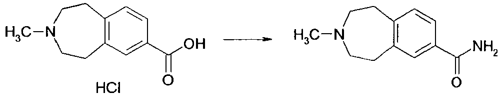

38e wird analog zu 1f aus 2.00 g (8.27 mMol) Produkt aus 38d, 18.20 ml (9.10 mMol) Ammoniak 0.5 M in Dioxan (Aldrich), 3.46 ml (24.82 mMol) Triethylamin und 3.19 g (9.93 mMol) TBTU in 30 ml THF hergestellt.

C₁₂H₁₆N₂O (204.27)

[M+H]+ = 205

HPLC (Methode 2): Retentionszeit = 1.54 min

### 38f)

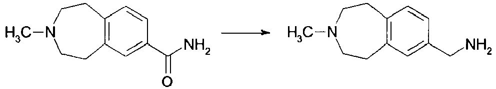

Eine Mischung aus 5.20 ml (5.20 mMol) Lithiumaluminiumhydrid 1 M in THF (Aldrich) und 18 ml THF wird auf 50°C erhitzt und langsam mit 0.84 g (2.64 mMol) Produkt aus 38e versetzt. Die Reaktionsmischung wird danach 30 min bei 50°C gerührt. Man kühlt anschließend auf - 20°C ab und quencht die Reaktionsmischung erst mit einer Mischung aus Wasser und THF, dann mit 2 M Natronlauge. Es wird eine Stunde bei Raumtemperatur gerührt. Der entstandene Niederschlag wird abfiltriert, das Filtrat im Vakuum bis zur Trockene eingeengt. Der Rückstand wird in Dichlormethan aufgenommen und mit gesättigter Natriumhydrogensulfat-Lösung gewaschen. Die organische Phase wird über Natriumsulfat getrocknet und im Vakuum bis zur Trockene eingeengt.

C₁₂H₁₈N₂ (190.28)

[M+H]+ = 191

HPLC (Methode 2): Retentionszeit = 2.21 min

### 38g)

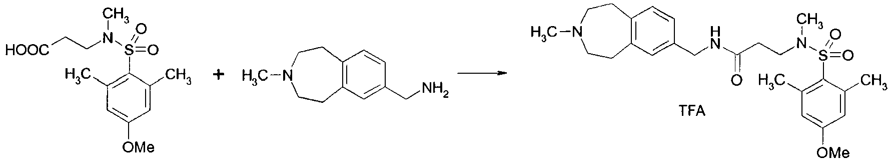

Beispiel 38 wird analog zu 1f aus 0.19 g (0.63 mMol) Produkt aus 22c, 0.12 g (0.63 mMol) Produkt aus 38f, 0.26 ml (1.89 mMol) Triethylamin und 0.20 g (0.63 mMol) TBTU in 5 ml THF hergestellt. C₂₅H₃₅N₃O₄S x C₂HF₃O₂ (587.65). [M+H]+ = 474.

HPLC (Methode 4): Retentionszeit = 2.98 min

### Beispiel 53

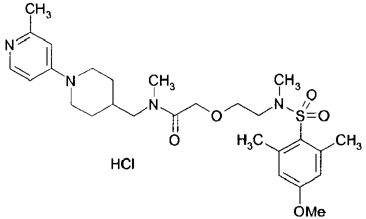

### 53a)

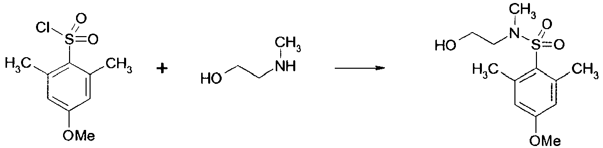

53a wird analog zu 3a aus 4.50 g (19.17 mMol) Produkt aus 13a, 1.69 g (21.10 mMol) N-Methylaminoethanol (BASF), 6.68 ml (47.90 mMol) Triethylamin in 150 ml Dichlormethan hergestellt. C₁₂H₁₉NO₄S (273.35). [M+H]+ = 274.

DC: Kieselgel, Dichlormethan / Ethanol 19:1, Rf-Wert = 0.43

### 53b)

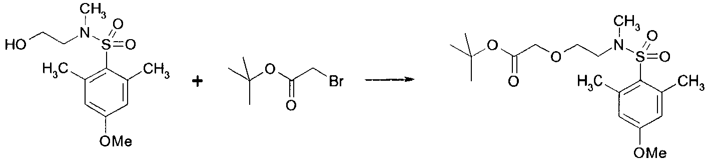

Eine Mischung aus 5.15 g (18.84 mMol) Produkt aus 53a, 1.75 g (6.60 mMol) Tetrabutylammoniumchlorid (Fluka) und 80 ml Toluol wird bei 0°C erst mit 100 ml 35%iger Natronlauge, dann mit 4.18 ml (28.26 mMol) Bromessigsäure-tert-butylester in 20 ml Toluol versetzt. Die Reaktionsmischung wird anschließend 1.5 Stunden bei Raumtemperatur gerührt, dann mit Diethylether verdünnt. Nach der Phasentrennung wird die organische Phase viermal mit Wasser neutral gewaschen, über Natriumsulfat getrocknet und im Vakuum bis zur Trockene eingeengt. Das so erhaltene Rohprodukt wird durch Säulenchromatographie über Kieselgel (Elutionsmittel: Petrolether / Ethylacetat 4: 1) gereinigt. C₁₈H₂₉NO₆S (387.49)

[M+H]+ = 388

DC: Kieselgel, Petrolether / Ethylacetat 7:3, Rf-Wert = 0.59

### 53c)

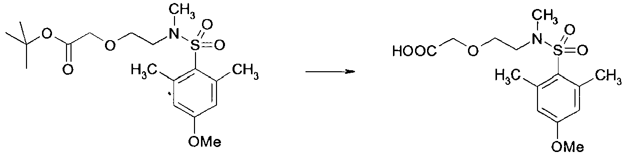

Eine Mischung aus 6.80 g (17.55 mMol) Produkt aus 53b, 8 ml TFA und 100 ml Dichlormethan wird 2.5 Stunden bei Raumtemperatur gerührt. Die Reaktionsmischung wird danach im Vakuum bis zur Trockene eingeengt. Man versetzt den Rückstand mit 1 M Natronlauge und extrahiert zweimal mit Ethylacetat (organische Extrakte verwerfen). Die wässrige Phase wird mit 2 M HCl angesäuert, dann nochmals mit Ethylacetat extrahiert. Die organischen Extrakte werden mit Wasser gewaschen, über Natriumsulfat getrocknet und im Vakuum bis zur Trockene eingeengt. C₁₄H₂₁NO₆S (331.29). [M+H]+ = 332.

HPLC (Methode 4): Retentionszeit = 3.4 min

### 53d)

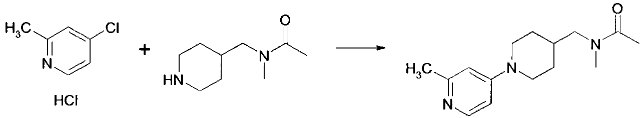

53d wird analog zu 28c aus 1.00 g (6.10 mMol) 4-Chlor-2-methylpyridin Hydrochlorid (Alfa Aesar) und 2.08 g (12.20 mMol) N-Methyl-N-piperidin-4-ylmethyl-acetamid (DE 1100635, Rhöne-Poulenc, 1961) hergestellt.

C₁₅H₂₃N₃O (261.36). [M+H]+ = 262.

HPLC (Methode 4): Retentionszeit = 1.9 min

### 53e)

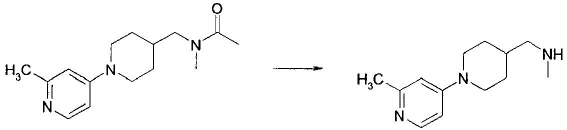

Eine Mischung aus 1.37 g (5.24 mMol) Produkt aus 53d und 15 ml halbkonzentrierte HCl wird vier Tage unter Rückfluss erhitzt. Die Reaktionsmischung wird mit 20%iger Natronlauge alkalisch gestellt und mit Dichlormethan extrahiert. Die organischen Extrakte werden über Natriumsulfat getrocknet und im Vakuum bis zur Trockene eingeengt.

C₁₃H₂₁N₃ (219.33). [M+H]+ = 220.

DC: Kieselgel, Dichlormethan / Methanol / Ammoniak 8:2:0.2, Rf-Wert = 0.48

### 53f)

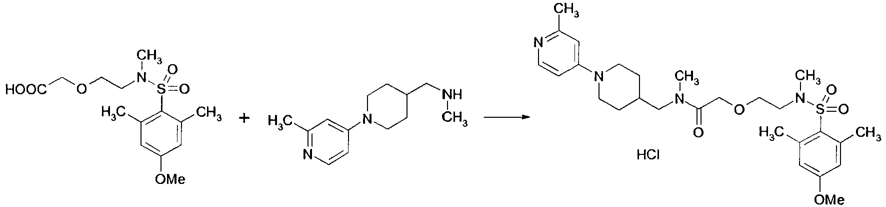

Beispiel 53 wird analog zu 1f aus 0.099 g (0.30 mMol) Produkt aus 53c, 0.066 g (0.30 mMol) Produkt aus 53e, 0.10 ml (0.75 mMol) Triethylamin und 0.12 g (0.36 mMol) TBTU in 8 ml THF und 1 ml DMF hergestellt.

C₂₇H₄₀N₄O₅S x HCl (569.16). [M+H]+ = 533.

HPLC (Methode 4): Retentionszeit = 3.1 min

### Beispiel 54

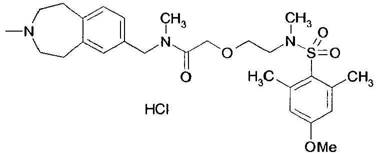

### 54a)

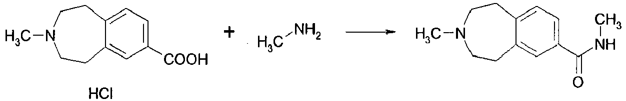

54a wird analog zu 1f aus 2.00 g (8.27 mMol) Produkt aus 38d, 8.28 ml (16.55 mMol) Methylamin 2 M in THF (Aldrich), 3.46 ml (24.82 mMol) Triethylamin und 3.19 g (9.93 mMol) TBTU in 30 ml THF hergestellt.

C₁₃H₁₈N₂O (218.29). [M+H]+ = 219.

DC: Kieselgel, Dichlormethan / Methanol 8:2, Rf-Wert = 0.14

### 54b)

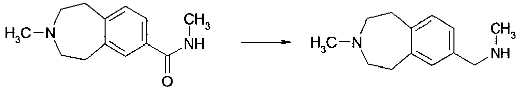

54b wird analog zu 38f aus 1.00 g (4.58 mMol) Produkt aus 54a und 9.00 ml (9.00 mMol) Lithiumaluminiumhydrid 1 M in THF (Aldrich) in 30 ml THF hergestellt.

C₁₃H₂₀N₂ (204.31). [M+H]+ = 205.

DC: Kieselgel, Dichlormethan / Methanol 8:2, Rf-Wert = 0.07

### 54c)

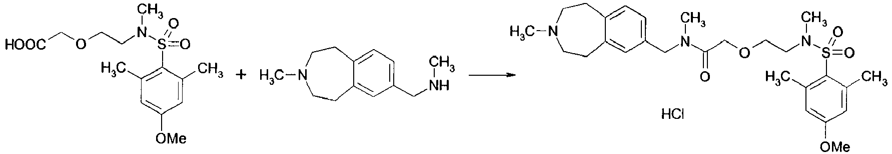

Beispiel 54 wird analog zu 1f aus 0.099 g (0.30 mMol) Produkt aus 53c, 0.088 g (0.30 mMol) Produkt aus 54b, 0.10 ml (0.75 mMol) Triethylamin und 0.12 g (0.36 mMol) TBTU in 7 ml THF und 1 ml DMF hergestellt.

C₂₇H₃₉N₃O₅S x HCl (554.14). [M+H]+ = 518.

HPLC (Methode 4): Retentionszeit = 3.1 min

### Beispiel 59

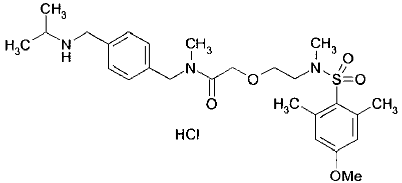

### 59a)

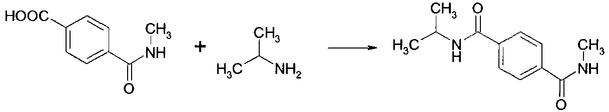

59a wird analog zu 1f aus 2.00 g (11.16 mMol) Terephthalsäuremonomethylamid (EMKA), 1.90 ml (22.32 mMol) Isopropylamin (Aldrich), 3.11 ml (22.32 mMol) Triethylamin und 4.30 g (13.40 mMol) TBTU in 60 ml THF hergestellt.

C₁₂H₁₆N₂O₂ (220.27). [M+H]+ = 221.

HPLC (Methode 4): Retentionszeit = 2.3 min

### 59b)

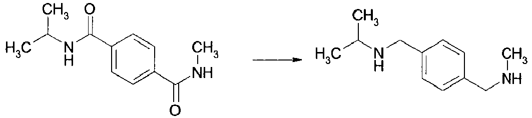

59b wird analog zu 38f aus 1.34 g (6.08 mMol) Produkt aus 59a und 25.00 ml (25.00 mMol) Lithiumaluminiumhydrid 1 M in THF (Aldrich) in 150 ml THF hergestellt.

C₁₂H₂₀N₂ (192.30). [M+H]+ = 193.

DC: Kieselgel, Dichlormethan / Methanol / Ammoniak 9:1:0.1, Rf-Wert = 0.17

### 59c)

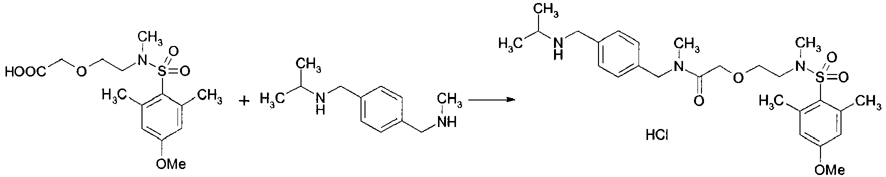

Beispiel 59 wird analog zu 1f aus 0.099 g (0.30 mMol) Produkt aus 53c, 0.058 g (0.30 mMol) Produkt aus 59b, 0.10 ml (0.75 mMol) Triethylamin und 0.12 g (0.36 mMol) TBTU in 8 ml THF hergestellt.

C₂₆H₃₉N₃O₅S x HCl (542.13). [M+H]+ = 506.

HPLC (Methode 4): Retentionszeit = 3.1 min

### Beispiel 61

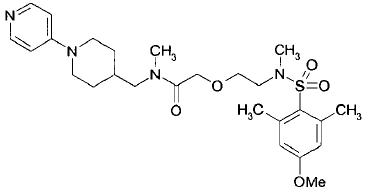

### 61a)

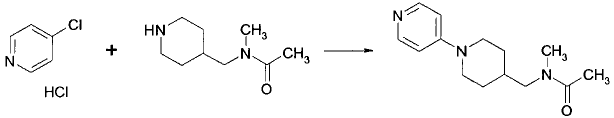

61a wird analog zu 28c aus 1.00 g (6.67 mMol) 4-Chlorpyridin Hydrochlorid (Aldrich) und 2.55 g (15.00 mMol) N-Methyl-N-piperidin-4-ylmethyl-acetamid (DE 110635, Rhöne-Poulenc, 1961) in 1 ml Wasser hergestellt. C₁₄H₂₁N₃O (247.34). [M+H]+ = 248.

### 61b)

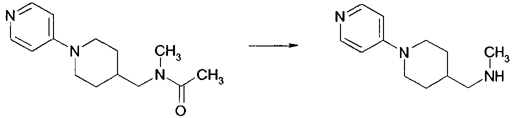

Eine Mischung aus 1.00 g (4.04 mMol) Produkt aus 61a und 10 ml halbkonzentrierte HCl wird drei Tage unter Rückfluss erhitzt. Die Reaktionsmischung wird danach mit Wasser verdünnt, mit 20%iger Natronlauge alkalisch gestellt und mit Ethylacetat extrahiert. Die organischen Extrakte werden über Natriumsulfat getrocknet und im Vakuum bis zur Trockene eingeengt. C₁₂H₁₉N₃ (205.30).

DC: Kieselgel, Dichlormethan / Ethanol 9:1, Rf-Wert = 0.2

### 61c)

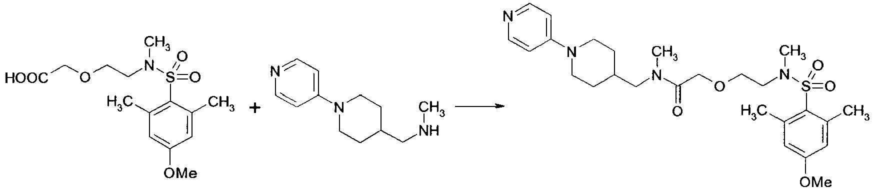

Beispiel 61 wird analog zu 1f aus 0.099 g (0.30 mMol) Produkt aus 53c, 0.062 g (0.30 mMol) Produkt aus 61 b, 0.083 ml (0.60 mMol) Triethylamin und 0.12 g (0.36 mMol) TBTU in 8 ml THF und 1 ml DMF hergestellt.

C₂₆H₃₈N₄O₅S (518.67). [M+H]+ = 519.

HPLC (Methode 4): Retentionszeit = 3.2 min

### Beispiel 62

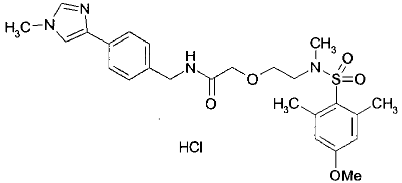

### 62a)

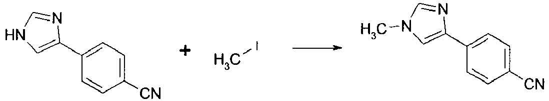

Eine Mischung aus 1.69 g (10.00 mMol) 4-(1 H-Imidazol-4-yl)-benzonitril (J. Am. Chem. Soc. 93, 1971, 4256-4263), 1.12 g (10.00 mMol) Kalium-tert-butylat und 25 ml DMSO wird erst 30 min bei Raumtemperatur gerührt, dann mit 0.62 ml (10.00 mMol) Methyliodid langsam versetzt und anschließend noch weitere 2.5 Stunden bei Raumtemperatur gerührt. Die Reaktionsmischung wird danach in Wasser gegeben, der entstandene Niederschlag wird abfiltriert und im Vakuum getrocknet. Das so erhaltene Rohprodukt wird durch Säulenchromatographie über Kieselgel (Elutionsmittel: Dichlormethan / Ethanol 19:1) gereinigt.

C₁₁H₉N₃ (183.21). [M+H]+ = 184.

HPLC (Methode 4): Retentionszeit = 1.9 min

### 62b)

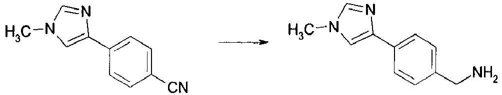

62b wird analog zu 34b aus 1.02 g (5.57 mMol) Produkt aus 62a, 0.20 g Raney-Nickel und 30 ml methanolischer Ammoniak-Lösung hergestellt. C₁₁H₁₃N₃ (187.24).

DC: Kieselgel, Dichlormethan / Methanol / Ammoniak 9:1:0.1, Rf-Wert = 0.27

### 62c)

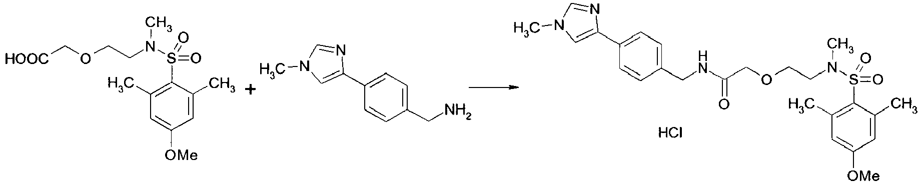

Beispiel 62 wird analog zu 1f aus 0.099 g (0.30 mMol) Produkt aus 53c, 0.056 g (0.30 mMol) Produkt aus 62b, 0.083 ml (0.60 mMol) Triethylamin und 0.12 g (0.36 mMol) TBTU in 7 ml THF und 1 ml DMF hergestellt.

C₂₅H₃₂N₄O₅S x HCl (537.07). [M+H]+ = 501.

HPLC (Methode 4): Retentionszeit = 3.2 min

### Beispiel 63

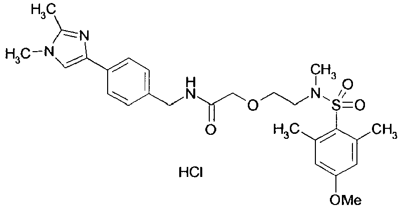

### 63a)

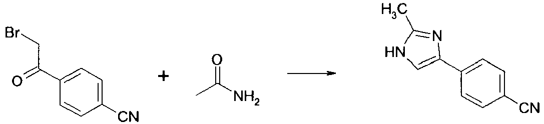

5.00 g (22.32 mMol) 2-Brom-4'-cyano-acetophenon (Aldrich) und 10.00 g (169.29 mMol) Acetamid (Merck) werden zusammen unter Rühren zwei Stunden auf 210°C erhitzt. Die Reaktionsmischung wird nach dem Abkühlen in Wasser eingerührt und mit 2 M HCl angesäuert. Der Niederschlag wird abfiltriert und verworfen. Das Filtrat wird mit konzentrierter Ammoniak-Lösung alkalisch gestellt, der Niederschlag abfiltriert und im Vakuum getrocknet. Das so erhaltene Rohprodukt wird durch Säulenchromatographie über Kieselgel (Elutionsmittel: Dichlormethan / Ethanol 9:1) gereinigt.

C₁₁H₉N₃ (183.21). [M-H]- = 182.

HPLC (Methode 4): Retentionszeit = 1.9 min

### 63b)

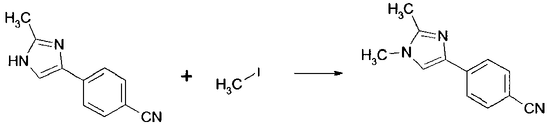

63b wird analog zu 62a aus 2.39 g (13.05 mMol) Produkt aus 63a, 1.46 g (13.05 mMol) Kalium-tert-butylat und 0.81 ml (13.05 mMol) Methyliodid in 50 ml DMSO hergestellt. C₁₂H₁₁N₃ (197.24). [M+H]+ = 198.

HPLC (Methode 4): Retentionszeit = 1.9 min

### 63c)

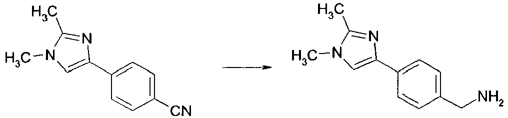

63c wird analog zu 34b aus 2.04 g (10.34 mMol) Produkt aus 63b, 0.40 g Raney-Nickel und 50 ml methanolischer Ammoniak-Lösung hergestellt.

C₁₂H₁₅N₃ (201.27)

DC: Kieselgel, Dichlormethan / Methanol / Ammoniak 9:1:0.1, Rf-Wert = 0.19

### 63d)

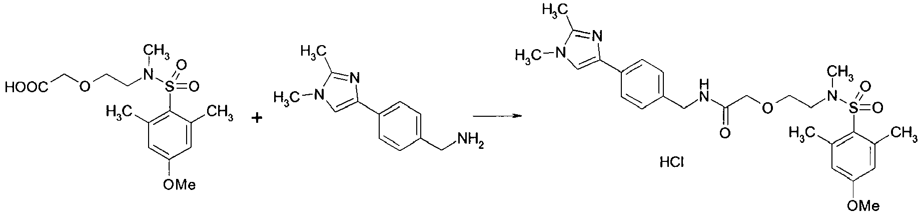

Beispiel 63 wird analog zu 1f aus 0.099 g (0.30 mMol) Produkt aus 53c, 0.060 g (0.30 mMol) Produkt aus 63c, 0.083 ml (0.60 mMol) Triethylamin und 0.12 g (0.36 mMol) TBTU in 7 ml THF und 1 ml DMF hergestellt.

C₂₆H₃₄N₄O₅S x HCl (551.10). [M+H]+ = 515.

HPLC (Methode 4): Retentionszeit = 3.2 min

### Beispiel 67

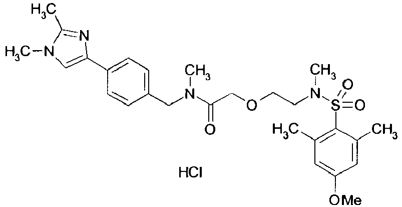

### 67a)

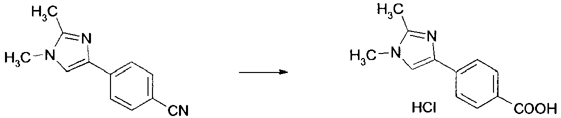

Eine Mischung aus 1.21 g (6.14 mMol) Produkt aus 63b, 20 ml 20%iger Natronlauge und 40 ml Ethanol wird über Nacht unter Rückfluss gerührt. Das Ethanol wird im Vakuum entfernt. Der Rückstand wird mit Wasser verdünnt und mit 4 M HCl sauer gestellt. Das ausgefallene Produkt wird abfiltriert und im Umlufttrockenschrank bei 50°C getrocknet.

C₁₂H₁₂N₂O₂ (252.70). [M+H]+ = 217.

HPLC (Methode 4): Retentionszeit = 1.7 min

### 67b)

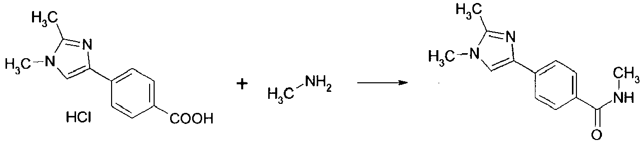

67b wird analog zu 1f aus 1.55 g (6.13 mMol) Produkt aus 67a, 4.60 ml (9.20 mMol) Methylamin 2 M in THF (Aldrich), 1.71 ml (12.30 mMol) Triethylamin und 2.38 g (7.40 mMol) TBTU in 50 ml THF hergestellt.

C₁₃H₁₅N₃O (229.28). [M+H]+ = 230.

DC: Kieselgel, Dichlormethan / Methanol 9:1, Rf-Wert = 0.47

### 67c)

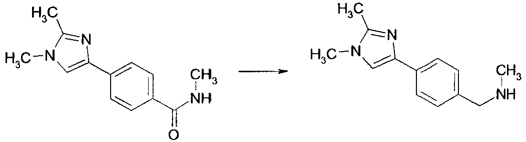

67c wird analog zu 38f aus 1.33 g (5.80 mMol) Produkt aus 67b und 15.00 ml (15.00 mMol) Lithiumaluminiumhydrid 1 M in THF (Aldrich) in 80 ml THF hergestellt.

C₁₃H₁₇N₃ (215.29). [M+H]+ = 216.

DC: Kieselgel, Dichlormethan / Methanol / Ammoniak 9:1:0.1, Rf-Wert = 0.29

### 67d)

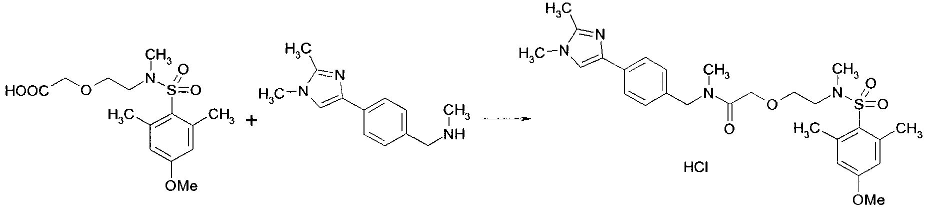

Beispiel 67 wird analog zu 1f aus 0.099 g (0.30 mMol) Produkt aus 53c, 0.065 g (0.30 mMol) Produkt aus 67c, 0.083 ml (0.60 mMol) Triethylamin und 0.12 g (0.36 mMol) TBTU in 7 ml THF und 1 ml DMF hergestellt.

C₂₇H₃₆N₄O₅S x HCl (565.13). [M+H]+ = 529.

HPLC (Methode 4): Retentionszeit = 3.1 min

### Beispiel 80

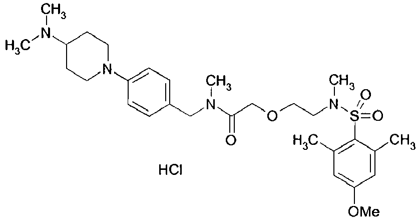

### 80a)

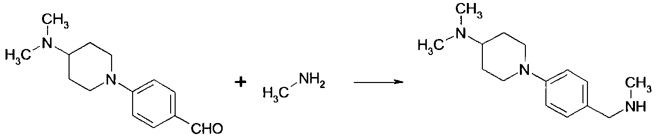

80a wird analog zu 33a aus 0.70 g (3.01 mMol) 4-(4-Dimethylamino-piperidin-1-yl)-benzaldehyd (Tetrahedron 57, 2001, 4781-4785), 3.00 ml (31.88 mMol) Methylamin 33% in Ethanol (Aldrich), 0.20 g Raney-Nickel in 25 ml Ethanol hergestellt.

C₁₅H₂₅N₃ (247.38). [M+H]+ = 248.

DC: Kieselgel, Dichlormethan / Methanol/Ammoniak 9:1:0.1, Rf-Wert = 0.16

### 80b)

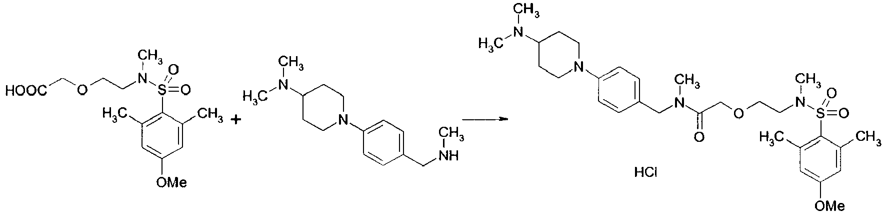

Beispiel 80 wird analog zu 1f aus 0.15 g (0.45 mMol) Produkt aus 53c, 0.11 g (0.45 mMol) Produkt aus 80a, 0.13 ml (0.90 mMol) Triethylamin und 0.17 g (0.54 mMol) TBTU in 5 ml DMF hergestellt.

C₂₉H₄₄N₄O₅S x HCl (597.21). [M+H]+ = 561.

HPLC (Methode 1): Retentionszeit = 2.33 min

### Beispiel 121

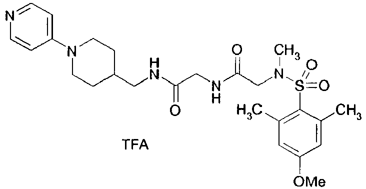

### 121a)

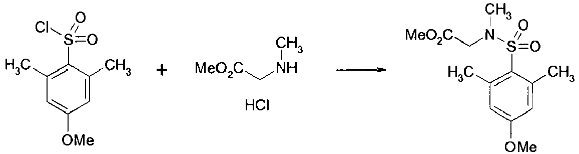

Eine Mischung aus 0.98 g (7.05 mMol) Sarcosin Methylester Hydrochlorid (Fluka), 1.65 g (7.05 mMol) Produkt aus 13a und 50 ml Pyridin wird eine Stunde bei Raumtemperatur gerührt. Die Reaktionsmischung wird danach im Vakuum bis zur Trockene eingeengt. Der Rückstand wird danach in 1 M HCl aufgenommen und mit Ethylacetat extrahiert. Die organischen Extrakte werden über Natriumsulfat getrocknet und im Vakuum bis zur Trockene eingeengt. C₁₃H₁₉NO₅S (301.36). [M+H]+ = 302.

### 121b)

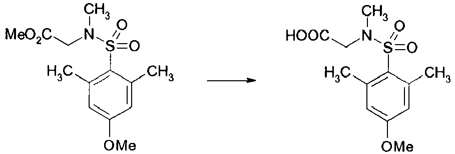

Eine Mischung aus 1.90 g (6.29 mMol) Produkt aus 121 a, 6.45 ml (12.90 mMol) 2 M Natronlauge und 9 ml Methanol wird drei Tage bei Raumtemperatur gerührt. Das Methanol wird im Vakuum entfernt, der wässrige Rückstand wird auf 1 M HCl gegossen. Der entstandene Niederschlag wird abfiltriert und im Vakuumexsikkator über Nacht getrocknet. C₁₂H₁₇NO₅S (287.33). [M+H]+ = 288.

### 121c)

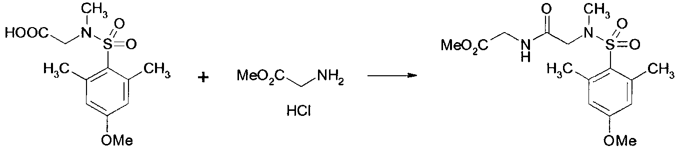

121c wird analog zu 1f aus 0.20 g (0.70 mMol) Produkt aus 121 b, 0.087 g (0.70 mMol) Glycin Methylester Hydrochlorid (Aldrich), 0.29 ml (2.09 mMol) Triethylamin und 0.22 g (0.70 mMol) TBTU in 5 ml THF hergestellt.

C₁₅H₂₂N₂O₆S (358.41). [M+H]+ = 359.

HPLC (Methode 5): Retentionszeit = 1.72 min

### 121d)

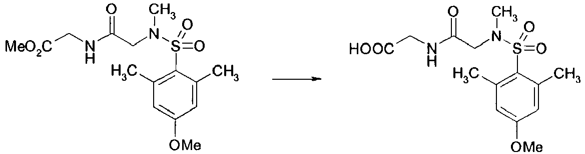

121 d wird analog zu 121 b aus 0.23 g (0.63 mMol) Produkt aus 121 c und 0.64ml (1.29 mMol) 2 M Natronlauge in 1 ml Methanol hergestellt.

C₁₄H₂₀N₂O₆S (344.38). [M+H]+ = 345.

### 121e)

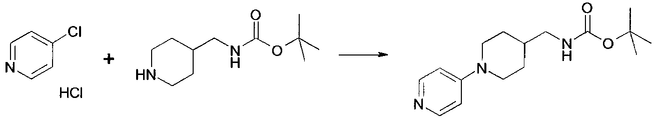

121 e wird analog zu 28c aus 3.00 g (14.00 mMol) Piperidin-4-ylmethyl-carbamatsäure-tert-butylester (EMKA), 2.10 g (14.00 mMol) 4-Chlorpyridin Hydrochlorid (Aldrich) und 7.80 ml (56.32 mMol) Triethlamin in 15 ml Isopropanol hergestellt.

C₁₆H₂₅N₃O₂ (291.39). [M+H]+ = 292.

HPLC (Methode 5): Retentionszeit = 1.40 min

### 121f)

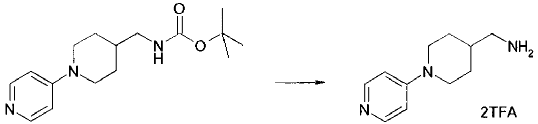

121f wird analog zu 18b aus 1.44 g (4.95 mMol) Produkt aus 121 e und 4.95 ml TFA in 8 ml Dichlormethan hergestellt.

C₁₁H₁₇N₃ x 2C₂HF₃O₂ (419.32). [M+H]+ = 192.

HPLC (Methode 5): Retentionszeit = 0.36 min

### 121g)

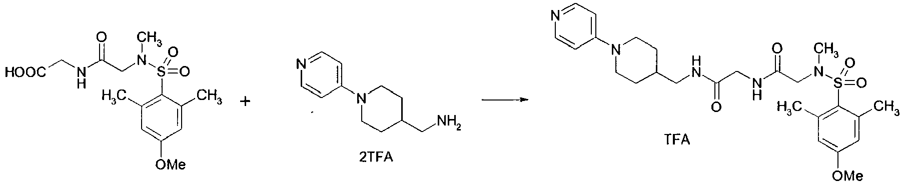

Beispiel 121 wird analog zu 1f aus 0.09 g (0.26 mMol) Produkt aus 121d, 0.11 g (0.26 mMol) Produkt aus 121f, 0.15 ml (1.05 mMol) Triethylamin und 0.084 g (0.26 mMol) TBTU in 1.9 ml THF hergestellt.

C₂₅H₃₅N₅O₅S x C₂HF₃O₂ (631.67). [M+H]+ = 518.

HPLC (Methode 5): Retentionszeit = 1.46 min

### Beispiel 122

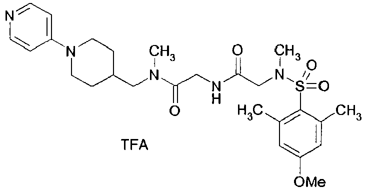

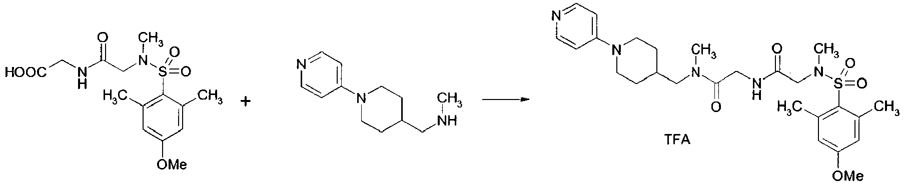

Beispiel 122 wird analog zu 1f aus 0.09 g (0.26 mMol) Produkt aus 121d, 0.054 g (0.26 mMol) Produkt aus 61 b, 0.11 ml (0.78 mMol) Triethylamin und 0.084 g (0.26 mMol) TBTU in 1.9 ml THF hergestellt.

C₂₆H₃₇N₅O₅S x C₂HF₃O₂ (645.69). [M+H]+ = 532.

HPLC (Methode 5): Retentionszeit = 1.50 min

### Beispiel 123

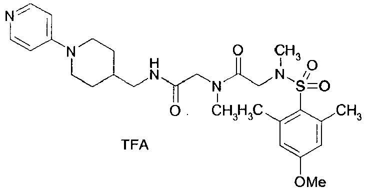

### 123a)

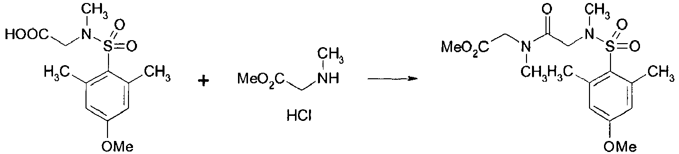

123a wird analog zu 1f aus 0.20 g (0.70 mMol) Produkt aus 121b, 0.097 g (0.70 mMol) Sarcosin Methylester Hydrochlorid (Fluka), 0.29 ml (2.09 mMol) Triethylamin und 0.22 g (0.70 mMol) TBTU in 5 ml THF hergestellt.

C₁₆H₂₄N₂O₆S (372.44). [M+H]+ = 373.

HPLC (Methode 5): Retentionszeit = 1.78 min

### 123b)

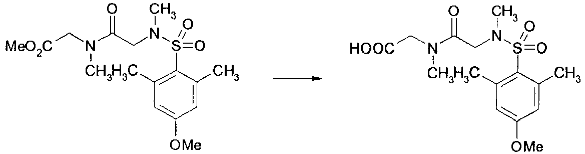

123b wird analog zu 121 b aus 0.23 g (0.60 mMol) Produkt aus 123a und 0.62 ml (1.24 mMol) 2 M Natronlauge in 1 ml Methanol hergestellt.

C₁₅H₂₂N₂O₆S (258.41). [M+H]+ = 359.

### 123c)

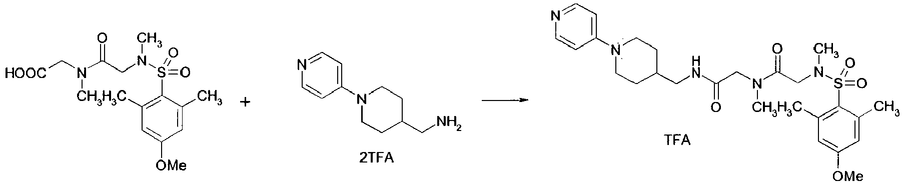

Beispiel 123 wird analog zu 1f aus 0.094 g (0.26 mMol) Produkt aus 123b, 0.11 g (0.26 mMol) Produkt aus 121f, 0.15 ml (1.05 mMol) Triethylamin und 0.084 g (0.26 mMol) TBTU in 1.9 ml THF hergestellt.

C₂₆H₃₇N₅O₅S x C₂HF₃O₂ (645.69). [M+H]+ = 532.

HPLC (Methode 5): Retentionszeit = 1.47 min

### Beispiel 124

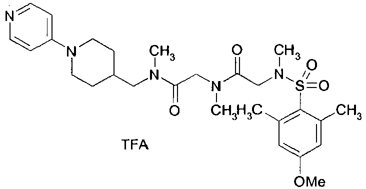

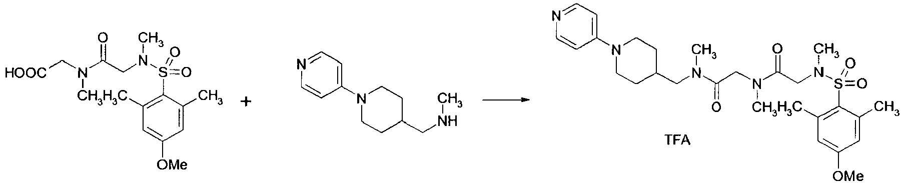

Beispiel 124 wird analog zu 1f aus 0.094 g (0.26 mMol) Produkt aus 123b, 0.054 g (0.26 mMol) Produkt aus 61 b, 0.11 ml (0.78 mMol) Triethylamin und 0.084 g (0.26 mMol) TBTU in 1.9 ml THF hergestellt.

C₂₇H₃₉N₅O₅S x C₂HF₃O₂ (659.72). [M+H]+ = 548.

HPLC (Methode 5): Retentionszeit = 1.49 min

### Beispiel 125

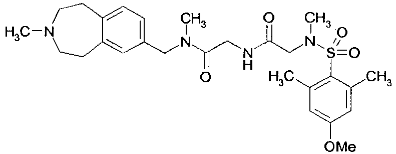

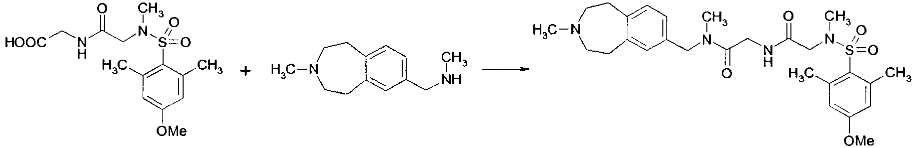

Beispiel 125 wird analog zu 1f aus 0.09 g (0.26 mMol) Produkt aus 121d, 0.076 g (0.26 mMol) Produkt aus 54b, 0.11 ml (0.78 mMol) Triethylamin und 0.084 g (0.26 mMol) TBTU in 1.9 ml THF hergestellt.

C₂₇H₃₈N₄O₅S (530.68). [M+H]+ = 531.

HPLC (Methode 1): Retentionszeit = 2.43 min

### Beispiel 126

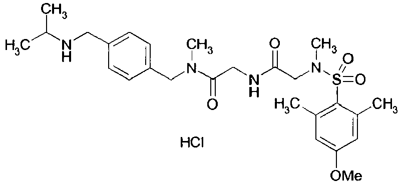

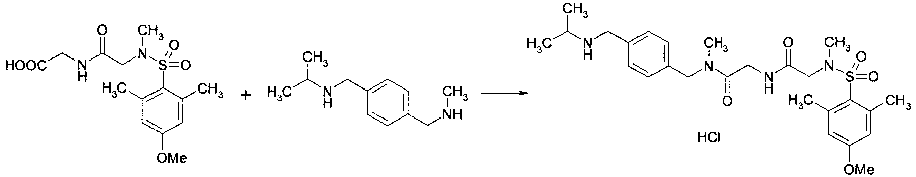

Beispiel 126 wird analog zu 1f aus 0.09 g (0.26 mMol) Produkt aus 121d, 0.05 g (0.26 mMol) Produkt aus 59b, 0.11 ml (0.78 mMol) Triethylamin und 0.084 g (0.26 mMol) TBTU in 1.9 ml THF hergestellt.

C₂₆H₃₈N₄O₅S x HCl (555.13). [M+H]+ = 519.

HPLC (Methode 1): Retentionszeit = 2.42 min

### Beispiel 127

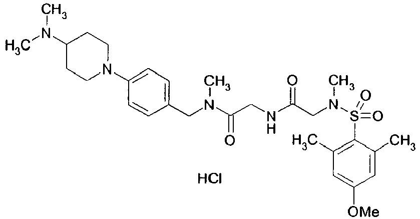

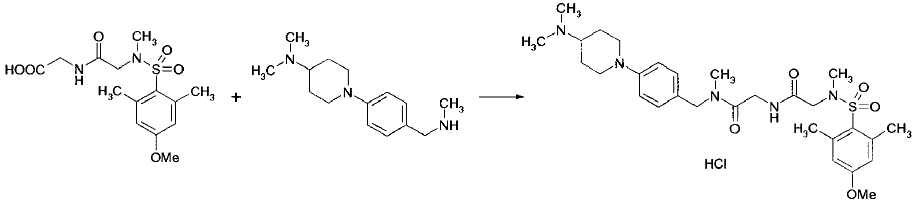

Beispiel 127 wird analog zu 1f aus 0.09 g (0.26 mMol) Produkt aus 121d, 0.065 g (0.26 mMol) Produkt aus 80a, 0.11 ml (0.78 mMol) Triethylamin und 0.084 g (0.26 mMol) TBTU in 1.9 ml THF hergestellt.

C₂₉H₄₃N₅O₅S x HCl (610.21)

[M+H]+ = 574

HPLC (Methode 1): Retentionszeit = 2.38 min

### Beispiel 128

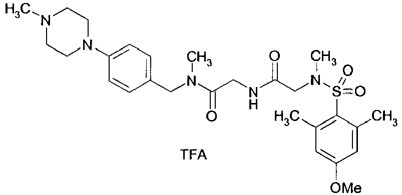

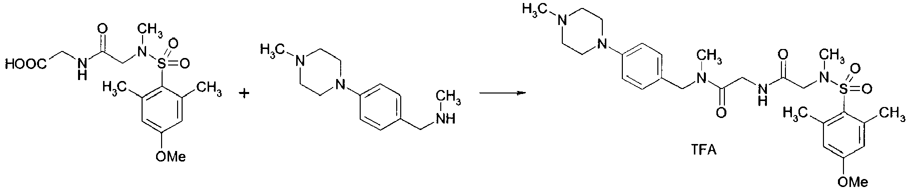

Beispiel 128 wird analog zu 1f aus 0.09 g (0.26 mMol) Produkt aus 121d, 0.057 g (0.26 mMol) Produkt aus 81 a, 0.11 ml (0.78 mMol) Triethylamin und 0.084 g (0.26 mMol) TBTU in 1.9 ml THF hergestellt.

C₂₇H₃₉N₅O₅S x C₂HF₃O₂ (610.21). [M+H]+ = 546.

HPLC (Methode 5): Retentionszeit = 1.51 min

### Beispiel 129

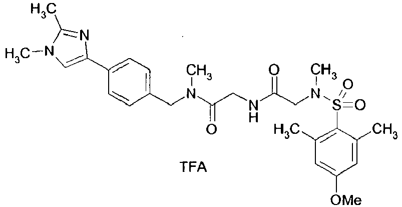

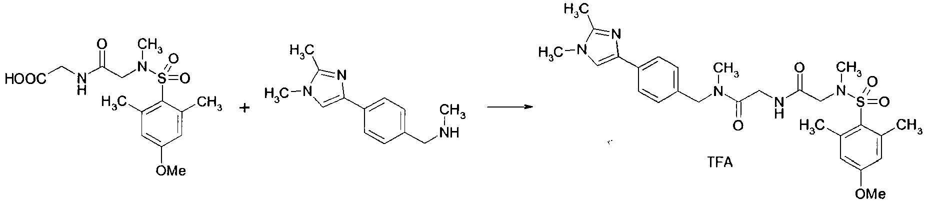

Beispiel 129 wird analog zu 1f aus 0.09 g (0.26 mMol) Produkt aus 121d, 0.056 g (0.26 mMol) Produkt aus 67c, 0.11 ml (0.78 mMol) Triethylamin und 0.084 g (0.26 mMol) TBTU in 1.9 ml THF hergestellt.

C₂₇H₃₅N₅O₅S x C₂HF₃O₂ (655.69). [M+H]+ = 542.

HPLC (Methode 5): Retentionszeit = 1.52 min

Die folgenden Verbindungen wurden analog zu Beispiel 121 hergestellt:

### Beispiel 483

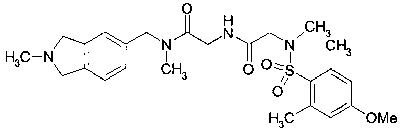

C₂₅H₃₄N₄O₅S x C₂HF₃O₂ (616.65). [M+H]+ = 503.

HPLC (Methode 6): Retentionszeit = 2.30 min

### Beispiel 484

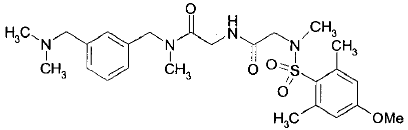

C₂₅H₃₆N₄O₅S x HCl (541.10). [M+H]+ = 505.

HPLC (Methode 5): Retentionszeit = 1.49 min

### Beispiel 485

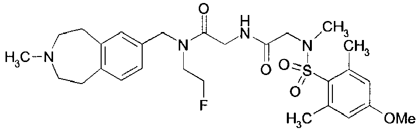

C₂₈H₃₉FN₄O₅S x C₂HF₃O₂ (676.72). [M+H]+ = 563.

HPLC (Methode 5): Retentionszeit = 1.55 min

### Beispiel 486

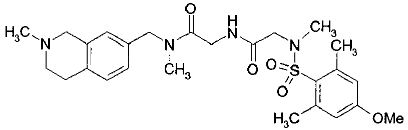

C₂₆H₃₆N₄O₅S x HCl (553.11). [M+H]+ = 517.

HPLC (Methode 11): Retentionszeit = 1.73 min

### Beispiel 487

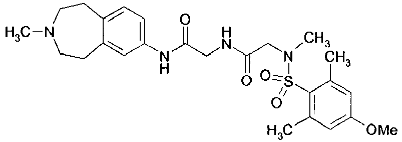

C₂₅H₃₄N₄O₅S x C₂HF₃O₂ (616.65). [M+H]+ = 503.

HPLC (Methode 5): Retentionszeit = 1.49 min

### Beispiel 488

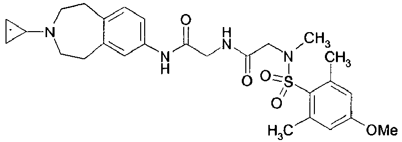

C₂₇H₃₆N₄O₅S x C₂HF₃O₂ (642.69). [M+H]+ = 529.

HPLC (Methode 5): Retentionszeit = 1.50 min

### Beispiel 489

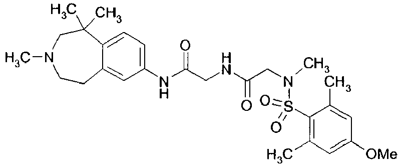

C₂₇H₃₈N₄O₅S x C₂HF₃O₂ (644.70). [M+H]+ = 531.

HPLC (Methode 5): Retentionszeit = 1.54 min

### Beispiel 490

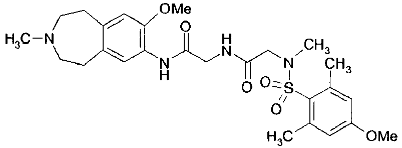

C₂₆H₃₆N₄O₆S x C₂HF₃O₂ (646.68)

[M+H]+ = 533

HPLC (Methode 5): Retentionszeit = 1.51 min

### Beispiel 491

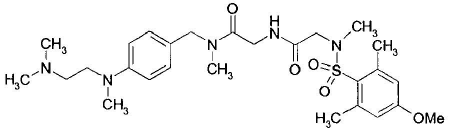

C₂₇H₄₁N₅O₅S x HCl (584.17). [M+H]+ = 548.

HPLC (Methode 5): Retentionszeit = 1.53 min

### Beispiel 492

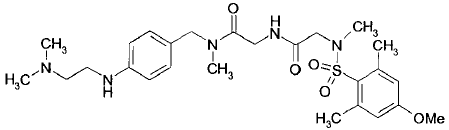

C₂₆H₃₉N₅O₅S x HCl (570.15). [M+H]+ = 534.

HPLC (Methode 5): Retentionszeit = 1.52 min

### Beispiel 493

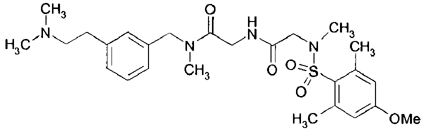

C₂₆H₃₈N₄O₅S x HCl (555.13). [M+H]+ = 519.

HPLC (Methode 5): Retentionszeit = 1.51 min

### Beispiel 494

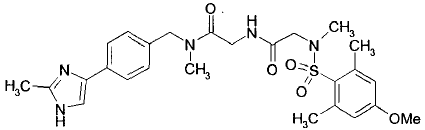

C₂₆H₃₃N₅O₅S (527.64)

[M+H]+ = 528

HPLC (Methode 4): Retentionszeit = 3.0 min

### Beispiel 495

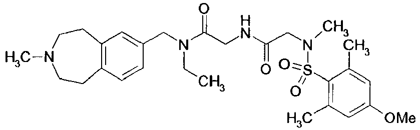

C₂₈H₄₀N₄O₅S x HCl (581.17). [M+H]+ = 545.

HPLC (Methode 4): Retentionszeit = 3.1 min

### Beispiel 496

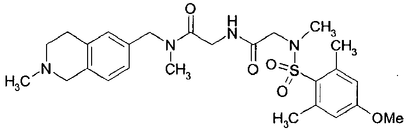

C₂₆H₃₆N₄O₅S x HCl (553.11). [M+H]+ = 517.

HPLC (Methode 5): Retentionszeit = 1.48 min

### Beispiel 497

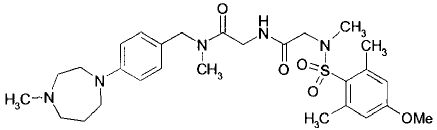

C₂₈H₄₁N₅O₅S x HCl (596.18)

[M+H]+=560

HPLC (Methode 5): Retentionszeit = 1.52 min

### Beispiel 498

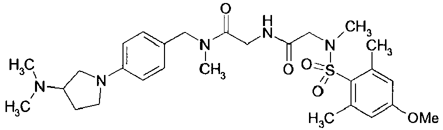

C₂₈H₄₁N₅O₅S x HCl (596.18)

[M+H]+ = 560

HPLC (Methode 5): Retentionszeit = 1.52 min

### Beispiel 499

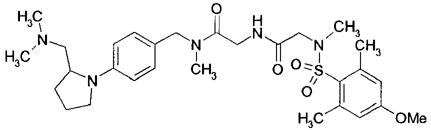

C₂₉H₄₃N₅O₅S x HCl (610.21). [M+H]+ = 574.

HPLC (Methode 5): Retentionszeit = 1.57 min

### Beispiel 500

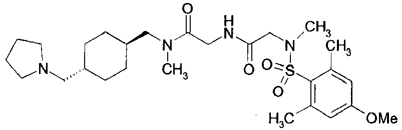

C₂₇H₄₄N₄O₅S x HCl (573.19)

[M+H]+=537

DC: Kieselgel, Dichlormethan / Methanol / Ammoniak 8:2:0.01, Rf-Wert = 0.66

### Beispiel 501

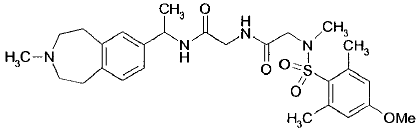

C₂₇H₃₈N₄O₅S x HCl (567.14)

[M+H]+ = 531

HPLC (Methode 4): Retentionszeit = 3.0 min

### Beispiel 502

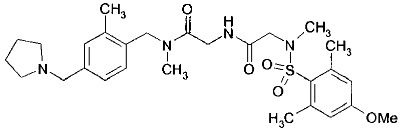

C₂₈H₄₀N₄O₅S x HCl (581.17)

[M+H]+=545

DC: Kieselgel, Dichlormethan / Methanol 9:1, Rf-Wert = 0.18

### Beispiel 503

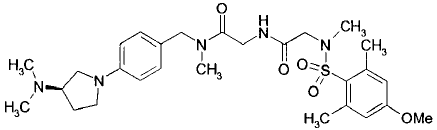

C₂₈H₄₁N₅O₅S x HCl (596.18). [M+H]+ = 560.

DC: Kieselgel, Ethylacetat / Methanol / Ammoniak 9:1:0.1, Rf-Wert = 0.42

### Beispiel 504

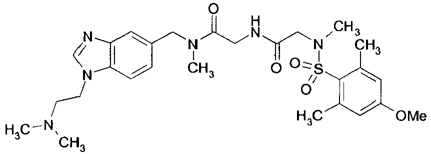

C₂₇H₃₈N₆O₅S (558.69). [M+H]+ = 559.

HPLC (Methode 6): Retentionszeit = 2.23 min

### Beispiel 505

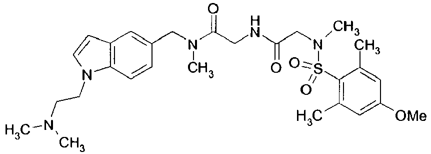

C₂₈H₃₉N₅O₅S (557.71). [M+H]+ = 558.

HPLC (Methode 6): Retentionszeit = 2.35 min

### Beispiel 506

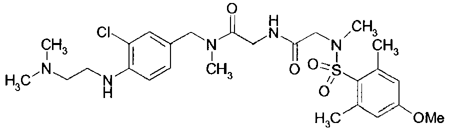

C₂₆H₃₈ClN₅O₅S x CH₂O₂ (614.16)

[M+H]+ = 568/570

HPLC (Methode 6): Retentionszeit = 2.55 min

### Beispiel 507

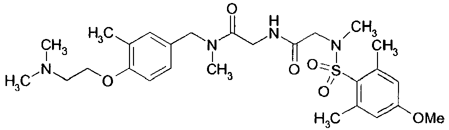

C₂₇H₄₀N₄O₆S (548.70). [M+H]+ = 549.

HPLC (Methode 6): Retentionszeit = 2.49 min

### Beispiel 508

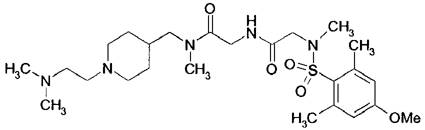

C₂₅H₄₃N₅O₅S (525.71). [M+H]+ = 526.

HPLC (Methode 6): Retentionszeit = 1.85 min

### Beispiel 509

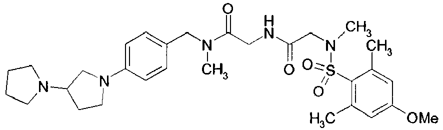

C₃₀H₄₃N₅O₅S (585.76)

[M+H]+ = 586

HPLC (Methode 6): Retentionszeit = 2.66 min

### Beispiel 510

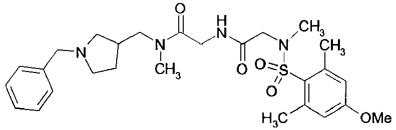

C₂₇H₃₈N₄O₅S (530.68)

[M+H]+ = 531

HPLC (Methode 6): Retentionszeit = 2.52 min

### Beispiel 511

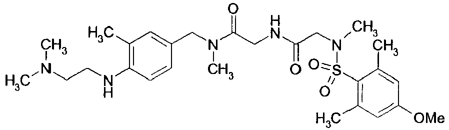

C₂₇H₄₁N₅O₅S (547.71). [M+H]+ = 548.

HPLC (Methode 6): Retentionszeit = 2.44 min

### Beispiel 512

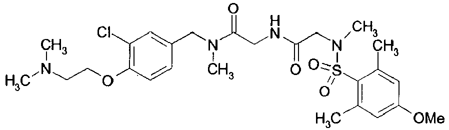

C₂₆H₃₇ClN₄O₆S (569.11). [M+H]+ = 570.

HPLC (Methode 6): Retentionszeit = 2.56 min

### Beispiel 513

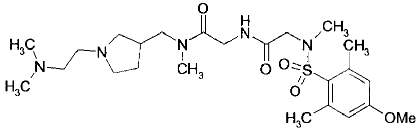

C₂₄H₄₁N₅O₅S (511.68)

[M+H]+ = 512

HPLC (Methode 6): Retentionszeit = 1.80 min

### Beispiel 514

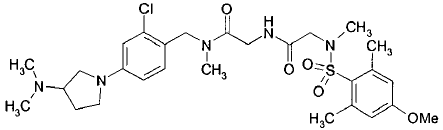

C₂₈H₄₀ClN₅O₅S (594.17)

[M+H]+ = 594/596

HPLC (Methode 6): Retentionszeit = 2.68 min

### Beispiel 515

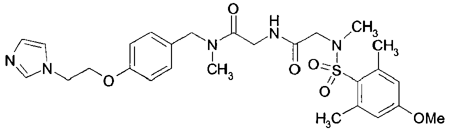

C₂₇H₃₅N₅O₆S (557.66). [M+H]+ = 558.

HPLC (Methode 6): Retentionszeit = 2.51 min

### Beispiel 516

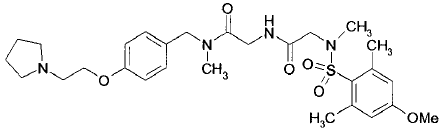

C₂₈H₄₀N₄O₆S (560.71)

[M+H]+ = 561

HPLC (Methode 6): Retentionszeit = 2.51 min

### Beispiel 517

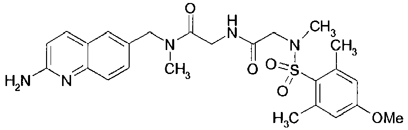

C₂₅H₃₁N₅O₅S (513.61)

[M+H]+ = 514

HPLC (Methode 6): Retentionszeit = 2.35 min

### Beispiel 518

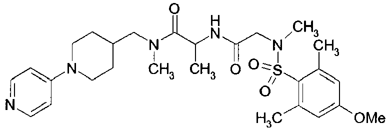

C₂₇H₃₉N₅O₅S x C₂HF₃O₂ (659.72)

[M+H]+ = 546

HPLC (Methode 6): Retentionszeit = 2.44 min

### Beispiel 519

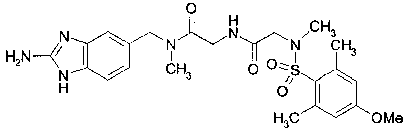

C₂₃H₃₀N₆O₅S (502.59). [M+H]+ = 503.

HPLC (Methode 6): Retentionszeit = 2.37 min

### Beispiel 520

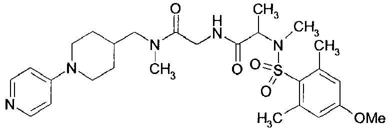

C₂₇H₃₉N₅O₅S x C₂HF₃O₂ (659.72). [M+H]+ = 546.

HPLC (Methode 6): Retentionszeit = 2.52 min

### Beispiel 521

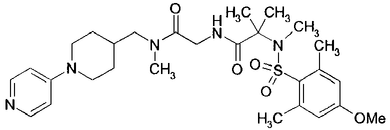

C₂₈H₄₁N₅O₅S x C₂HF₃O₂ (673.75)

[M+H]+ = 560

HPLC (Methode 6): Retentionszeit = 2.58 min

### Beispiel 522

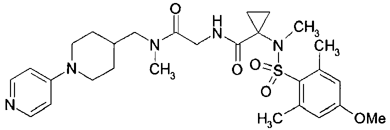

C₂₈H₃₉N₅O₅S x C₂HF₃O₂ (671.73)

[M+H]+ = 558

HPLC (Methode 6): Retentionszeit = 2.57 min

### Beispiel 523

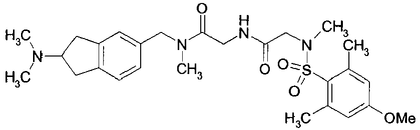

C₂₇H₃₈N₄O₅S x C₂HF₃O₂ (644.70). [M+H]+ = 531.

HPLC (Methode 6): Retentionszeit = 2.45 min

### Beispiel 524

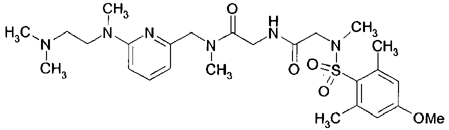

C₂₆H₄₀N₆O₅S (548.70). [M+H]+ = 549.

HPLC (Methode 6): Retentionszeit = 2.52 min

### Beispiel 525

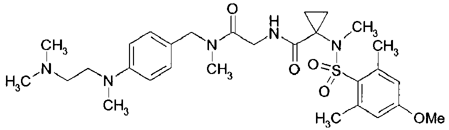

C₂₉H₄₃N₅O₅S x C₂HF₃O₂ (687.77)

[M+H]+ = 574

HPLC (Methode 6): Retentionszeit = 2.72 min

### Beispiel 526

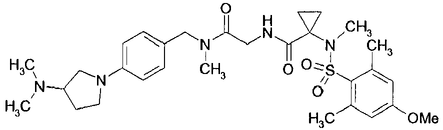

C₃₀H₄₃N₅O₅S (585.76)

[M+H]+ = 586

HPLC (Methode 6): Retentionszeit = 2.70 min

### Beispiel 527

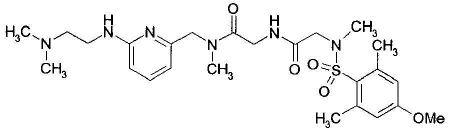

C₂₅H₃₈N₆O₅S x CH₂O₂ (580.70). [M+H]+ = 535.

HPLC (Methode 6): Retentionszeit = 2.16 min

### Beispiel 528

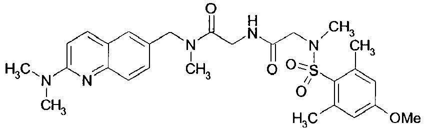

C₂₇H₃₅N₅O₅S x C₂HF₃O₂ (655.69). [M+H]+ = 542.

HPLC (Methode 6): Retentionszeit = 2.48 min

### Beispiel 529

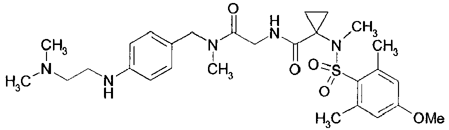

C₂₈H₄₁N₅O₅S x C₂HF₃O₂ (673.75)

[M+H]+ = 560

HPLC (Methode 6): Retentionszeit = 2.61 min

### Beispiel 530

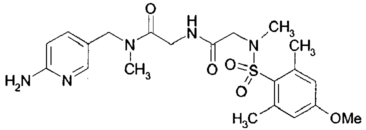

C₂₁H₂₉N₅O₅S (463.55)

[M+H]+ = 464

HPLC (Methode 9): Retentionszeit = 1.47 min

### Beispiel 531

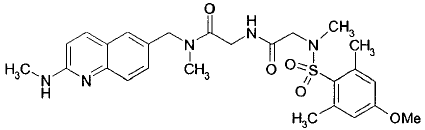

C₂₆H₃₃N₅O₅S (527.64). [M+H]+ = 528.

HPLC (Methode 9): Retentionszeit = 1.60 min

### Beispiel 532

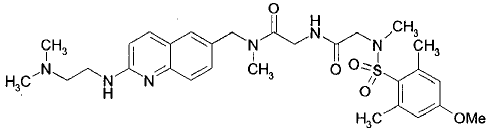

C₂₉H₄₀N₆O₅S (584.73)

[M+H]+ = 585

HPLC (Methode 9): Retentionszeit = 1.37 min

### Beispiel 533

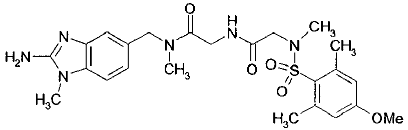

C₂₄H₃₂N₆O₅S x C₂HF₃O₂ (630.64)

[M+H]+ = 517

HPLC (Methode 9): Retentionszeit = 1.58 min

### Beispiel 534

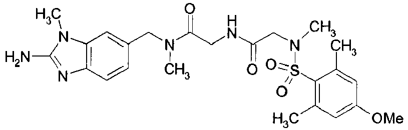

C₂₄H₃₂N₆O₅S x C₂HF₃O₂ (630.64)

[M+H]+ = 517

HPLC (Methode 9): Retentionszeit = 1.57 min

### Beispiel 535

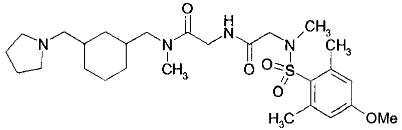

C₂₇H₄₄N₄O₅S x C₂HF₃O₂ (650.75). [M+H]+ = 537.

HPLC (Methode 9): Retentionszeit = 1.68 min

### Beispiel 536

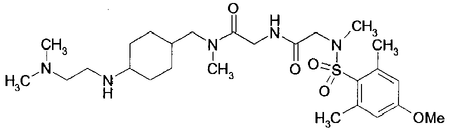

C₂₆H₄₅N₅O₅S x 2HCl (612.65)

[M+H]+ = 540

HPLC (Methode 5): Retentionszeit = 1.39 min

### Beispiel 537

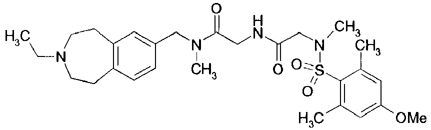

C₂₈H₄₀N₄O₅S x C₂HF₃O₂ (658.73)

[M+H]+ = 545

HPLC (Methode 5): Retentionszeit = 1.53 min

### Beispiel 538

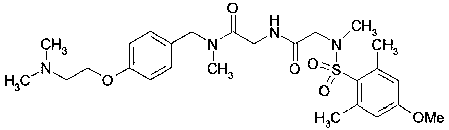

C₂₆H₃₈N₄O₆S x C₂HF₃O₂ (648.69)

[M+H]+ = 535

HPLC (Methode 5): Retentionszeit = 1.43 min

### Beispiel 539

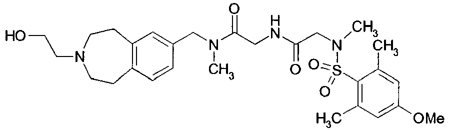

C₂₈H₄oN₄O₆S (560.71). [M+H]+ = 561.

HPLC (Methode 5): Retentionszeit = 1.50 min

### Beispiel 540

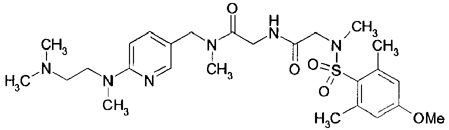

C₂₆H₄₀N₆O₅S (548.70)

[M+H]+ = 549

HPLC (Methode 5): Retentionszeit = 1.39 min

### Beispiel 541

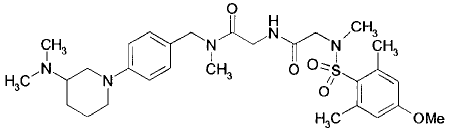

C₂₉H₄₃N₅O₅S x HCl (610.21)

[M+H]+ = 574

HPLC (Methode 7): Retentionszeit = 1.88 min

### Beispiel 542

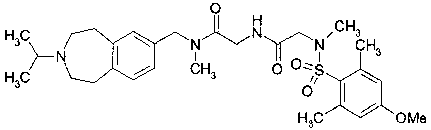

C₂₉H₄₂N₄O₅S x C₂HF₃O₂ (672.76)

[M+H]+ = 559

HPLC (Methode 5): Retentionszeit = 1.55 min

### Beispiel 543

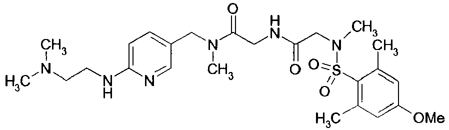

C₂₅H₃₈N₆O₅S x 2HCl (607.59). [M+H]+ = 535.

HPLC (Methode 5): Retentionszeit = 1.39 min

### Beispiel 544

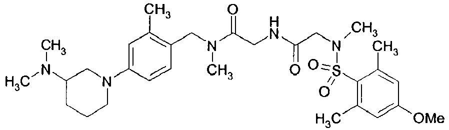

C₃₀H₄₅N₅O₅S x C₂HF₃O₂ (701.80)

[M+H]+ = 588

HPLC (Methode 5): Retentionszeit = 1.55 min

### Beispiel 545

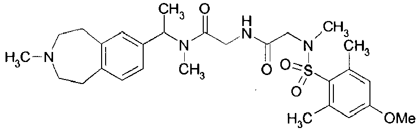

C₂₈H₄₀N₄O₅S x HCl (581.17)

[M+H]+ = 545

HPLC (Methode 4): Retentionszeit = 3.3 min

### Beispiel 546

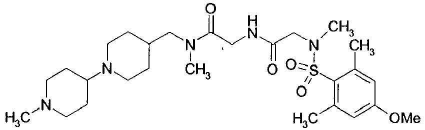

C₂₇H₄₅N₅O₅S x 2HCl (624.66)

[M+H]+ = 552

DC: Kieselgel, Dichlormethan / Methanol / Ammoniak 8:2:0.01, Rf-Wert = 0.25

### Beispiel 547

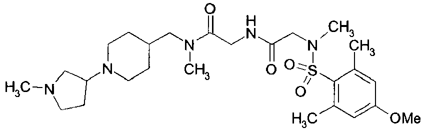

C₂₆H₄₃N₅O₅S x HCl (574.18). [M+H]+ = 538.

DC: Kieselgel, Dichlormethan / Methanol / Ammoniak 8:2:0.01, Rf-Wert = 0.10

### Beispiel 548

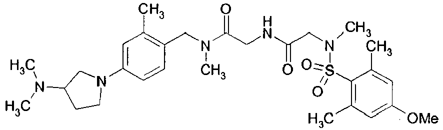

C₂₉H₄₃N₅O₅S x HCl (610.21)

[M+H]+ = 574

DC: Kieselgel, Dichlormethan / Methanol 9:1, Rf-Wert = 0.25

### Beispiel 549

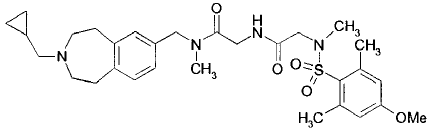

C₃₁H₄₄N₄O₅S x HCl (621.23)

[M+H]+ = 585

HPLC (Methode 12): Retentionszeit = 3.0 min

### Beispiel 550

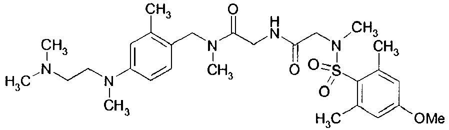

C₂₈H₄₃N₅O₅S x HCl (598.20)

[M+H]+ = 562

DC: Kieselgel, Dichlormethan / Methanol 9:1, Rf-Wert = 0.14

### Beispiel 551

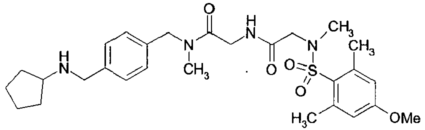

C₂₉H₄₂N₄O₅S x HCl (595.19). [M+H]+ = 559.

DC: Kieselgel, Dichlormethan / Methanol / Ammoniak 9:1:0.1, Rf-Wert = 0.32

### Beispiel 552

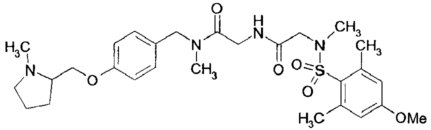

C₂₈H₄₀N₄O₆S (560.71)

[M+H]+ = 561

HPLC (Methode 9): Retentionszeit = 1.66 min

### Beispiel 553

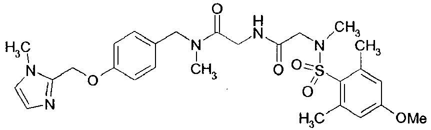

C₂₇H₃₅N₅O₆S (557.66)

[M+H]+ = 558

HPLC (Methode 9): Retentionszeit = 1.64 min

### Beispiel 554

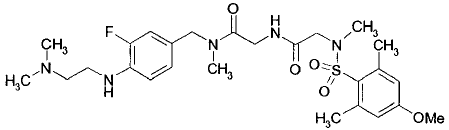

C₂₆H₃₈FN₅O₅S (551.68)

[M+H]+ = 552

HPLC (Methode 6): Retentionszeit = 2.30 min

### Beispiel 555

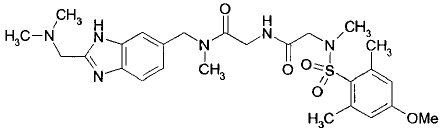

C₂₆H₃₆N₆O₅S (544.67). [M+H]+ = 545.

HPLC (Methode 6): Retentionszeit = 1.48 min

### Beispiel 556

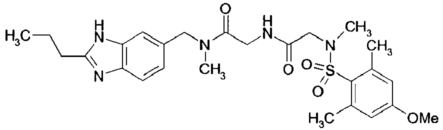

C₂₆H₃₅N₅O₅S (529.65)

[M+H]+ = 530

HPLC (Methode 9): Retentionszeit = 1.61 min

### Beispiel 557

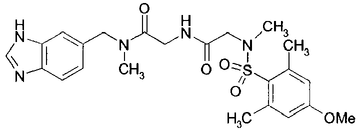

C₂₃H₂₉N₅O₅S (487.57)

[M+H]+ = 488

HPLC (Methode 9): Retentionszeit = 1.53 min

### Beispiel 558

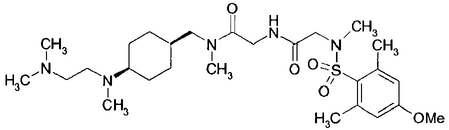

C₂₇H₄₇N₅O₅S x 2HCl (626.68)

[M+H]+ = 554

HPLC (Methode 7): Retentionszeit = 1.61 min

### Beispiel 559

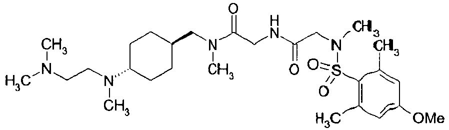

C₂₇H₄₇N₅O₅S x 2HCl (626.68). [M+H]+ = 554.

HPLC (Methode 7): Retentionszeit = 1.61 min

### Beispiel 560

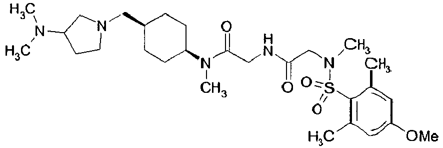

C₂₈H₄₇N₅O₅S x CH₂O₂ (611.80). [M+H]+ = 566.

HPLC (Methode 9): Retentionszeit = 1.33 min

### Beispiel 561

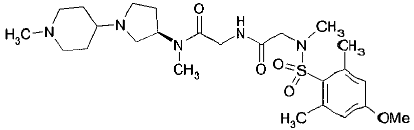

C₂₅H₄₁N₅O₅S x 2HCl (596.61)

[M+H]+ = 524

HPLC (Methode 12): Retentionszeit = 2.3 min

### Beispiel 562

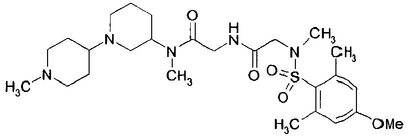

C₂₆H₄₃N₅O₅S x 2HCl (610.64)

[M+H]+ = 538

DC: Kieselgel, Dichlormethan / Methanol / Ammoniak 8:2:0.2, Rf-Wert = 0.53

### Beispiel 563

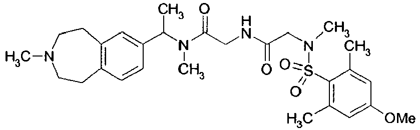

C₂₉H₄₂N₄O₅S x HCl (595.19). [M+H]+ = 559.

HPLC (Methode 12): Retentionszeit = 2.8 min

### Beispiel 564

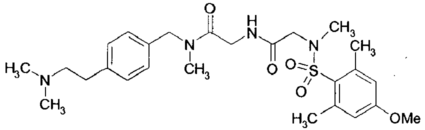

C₂₆H₃₈N₄O₅S x C₂HF₃O₂ (632.69)

[M+H]+ = 519

HPLC (Methode 9): Retentionszeit = 1.61 min

### Beispiel 565

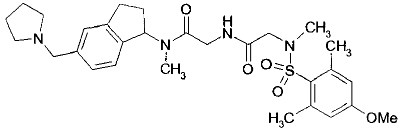

C₂₉H₄₀N₄O₅S (556.72)

[M+H]+ = 557

HPLC (Methode 9): Retentionszeit = 1.69 min

### Beispiel 566

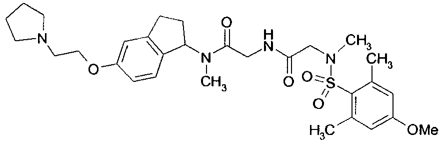

C₃₀H₄₂N₄O₆S (586.74). [M+H]+ = 587.

HPLC (Methode 9): Retentionszeit = 1.74 min

### Beispiel 603

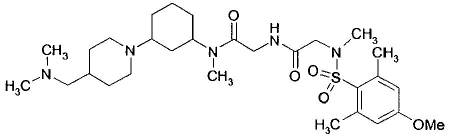

C₂₉H₄₉N₅O₅S x 2HCl (652.72). [M+H]+ = 580

HPLC (Methode 10): Retentionszeit =1.11 min

### Beispiel 604

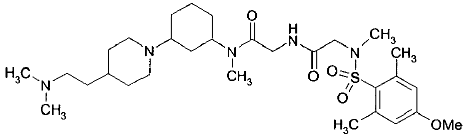

C₃₀H₅₁N₅O₅S x 2HCl (666.74). [M+H]+ = 594.

HPLC (Methode 10): Retentionszeit = 1.11 min

### Beispiel 605

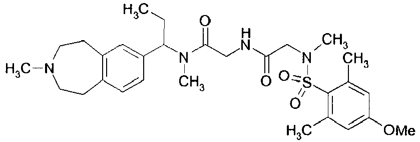

C₂₉H₄₂N₄O₅S x HCl (595.19). [M+H]+ = 559.

HPLC (Methode 12): Retentionszeit = 3.1 min

### Beispiel 631

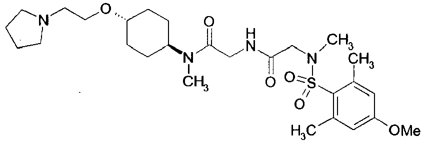

C₂₇H₄₄N₄O₆S x HCl (589.19)

[M+H]+ = 553

HPLC (Methode 5): Retentionszeit = 1.35 min

### Beispiel 632

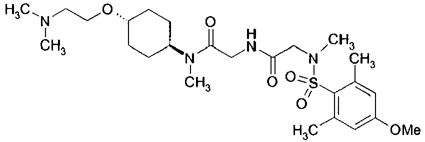

C₂₅H₄₂N₄O₆S x HCl (563.15)

[M+H]+ = 527

HPLC (Methode 5): Retentionszeit = 1.38 min

### Beispiel 633

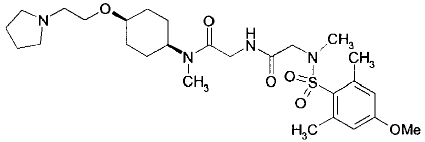

C₂₇H₄₄N₄O₆S x HCl (589.19)

[M+H]+ = 553

HPLC (Methode 5): Retentionszeit = 1.35 min

### Beispiel 634

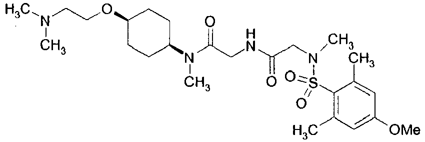

C₂₅H₄₂N₄O₆S x HCl (563.15)

[M+H]+ = 527

HPLC (Methode 5): Retentionszeit = 1.32 min

Die nachfolgenden Beispiele beschreiben pharmazeutischer Darreichungsformen, die als Wirkstoff eine beliebige Verbindung der allgemeinen Formel I enthalten:

### Beispiel I

Trockenampulle mit 75 mg Wirkstoff pro 10 ml

Zusammensetzung:

| | |
|---|---|
| Wirkstoff | 75.0 mg |
| Mannitol | 500 mg |
| Wasser für Injektionszwecke ad | 10.0 ml |

Herstellung:
   Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel II

Tablette mit 50 mg Wirkstoff

Zusammensetzung:

| | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215,0 mg |

Herstellung:
   (1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.

Durchmesser der Tabletten: 9 mm.

### Beispiel III

Tablette mit 350 mg Wirkstoff

Zusammensetzung:

| | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

Herstellung:
   (1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.

Durchmesser der Tabletten: 12 mm.

### Beispiel IV

Kapseln mit 50 mg Wirkstoff

Zusammensetzung:

| | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

Herstellung:
   (1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

### Beispiel V

Kapseln mit 350 mg Wirkstoff

Zusammensetzung:

| | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

Herstellung:
   (1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Gr6Be 0 abgefüllt.

### Beispiel VI

Suppositorien mit 100 mg Wirkstoff

**1 Zäpfchen enthält:**

| | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2000.0 mg |

## Patentansprüche

1. Verbindungen der allgemeinen Formel I 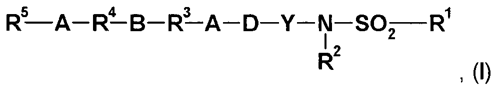 in der A, B, D, Y, R¹, R², R³, R⁴ und R⁵ wie in den folgenden Verbindungen definiert sind:
| **Beispiel** | **Struktur** |
|---|---|
| (121) | 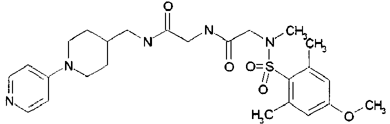 |
| (122) | 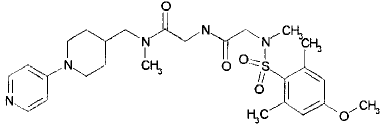 |
| (123) | 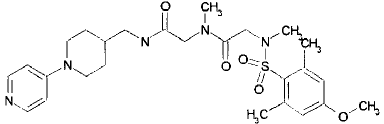 |
| (124) | 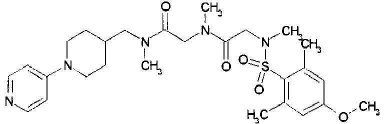 |
| (125) | 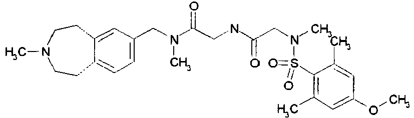 |
| (126) | 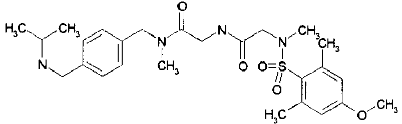 |
| (127) | 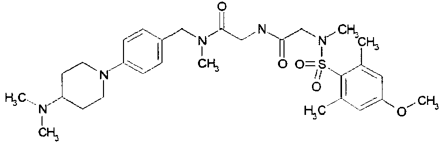 |
| (128) | 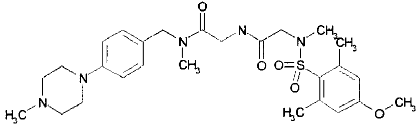 |
| (129) | 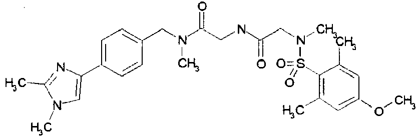 |
| (483) | 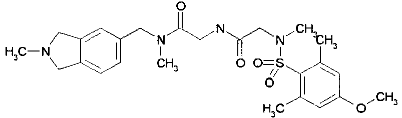 |
| (484) | 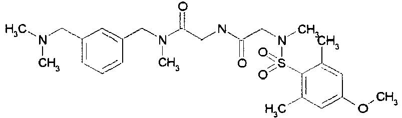 |
| (485) | 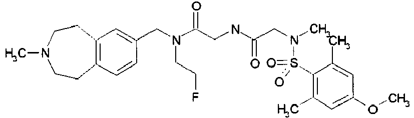 |
| (486) | 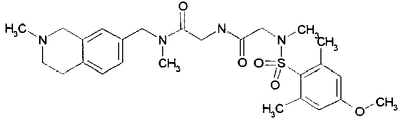 |
| (487) | 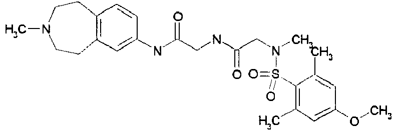 |
| (488) | 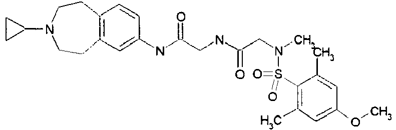 |
| (489) | 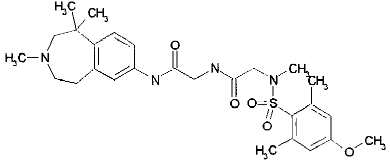 |
| (490) | 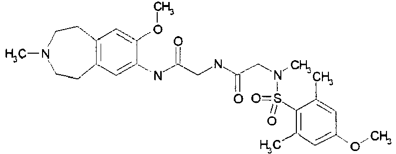 |
| (491) | 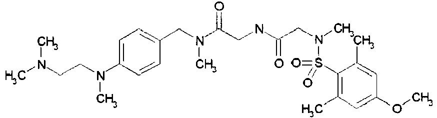 |
| (492) | 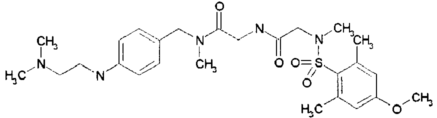 |
| (493) | 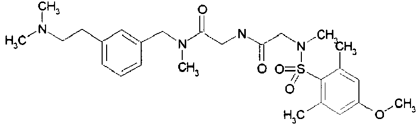 |
| (494) | 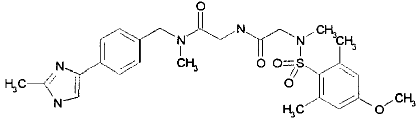 |
| (495) | 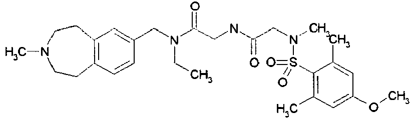 |
| (496) | 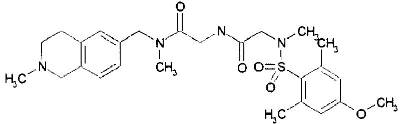 |
| (497) | 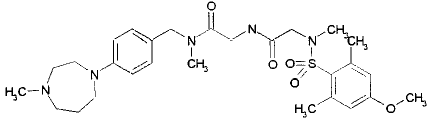 |
| (498) | 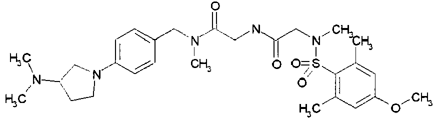 |
| (499) | 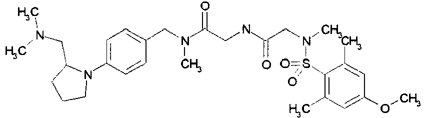 |
| (500) | 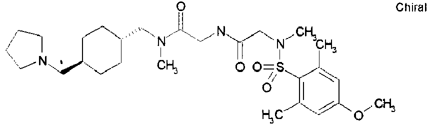 |
| (501) | 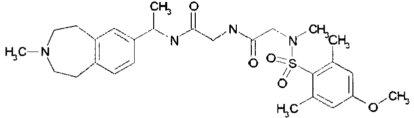 |
| (502) | 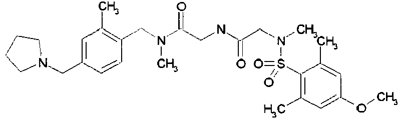 |
| (503) | 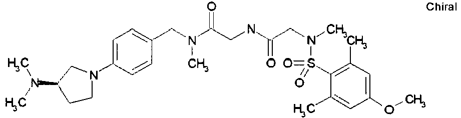 |
| (504) | 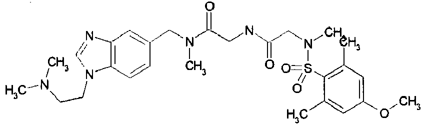 |
| (505) | 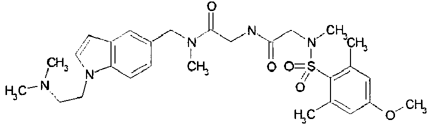 |
| (506) | 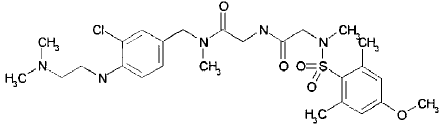 |
| (507) | 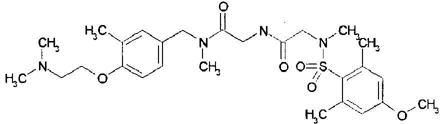 |
| (508) | 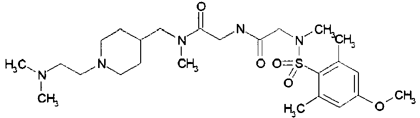 |
| (509) | 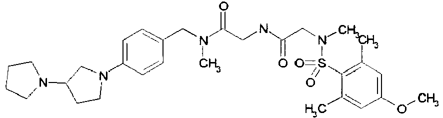 |
| (510) | 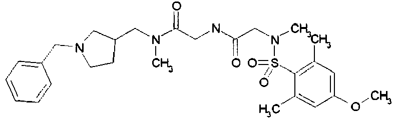 |
| (511) | 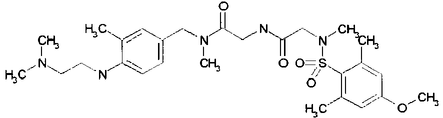 |
| (512) | 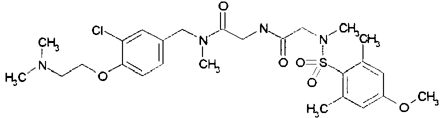 |
| (513) | 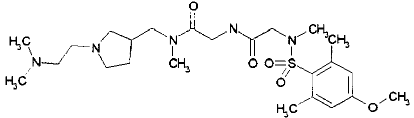 |
| (514) | 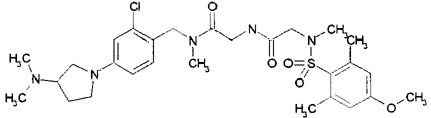 |
| (515) | 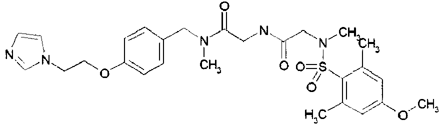 |
| (516) | 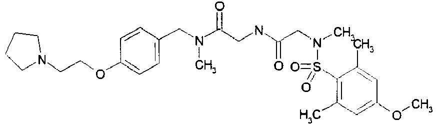 |
| (517) | 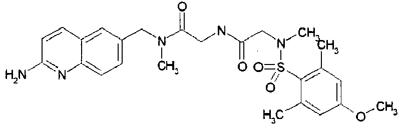 |
| (518) | 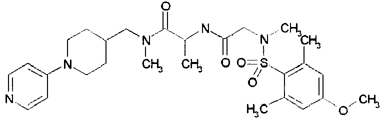 |
| (519) | 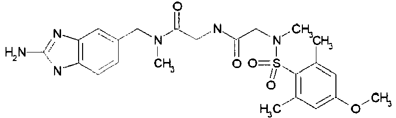 |
| (520) | 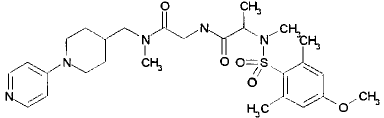 |
| (521) | 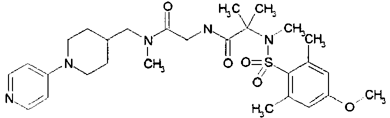 |
| (522) | 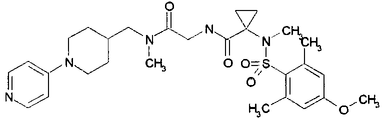 |
| (523) | 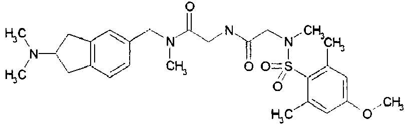 |
| (524) | 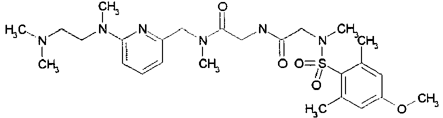 |
| (525) | 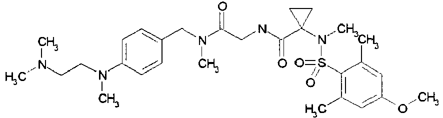 |
| (526) | 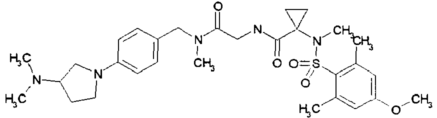 |
| (527) | 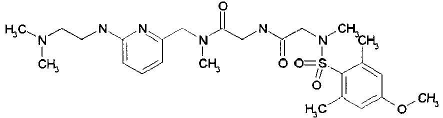 |
| (528) | 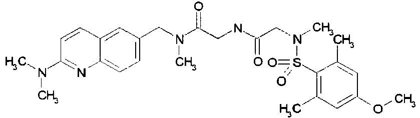 |
| (529) | 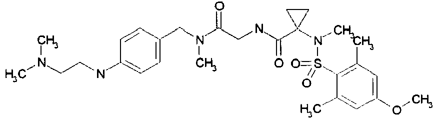 |
| (530) | 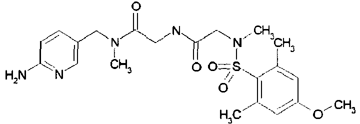 |
| (531) | 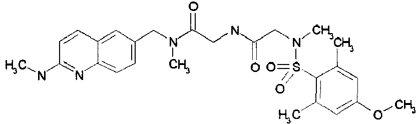 |
| (532) | 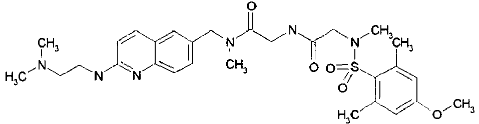 |
| (533) | 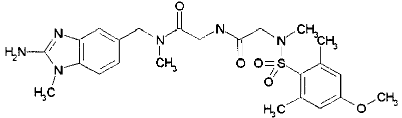 |
| (534) | 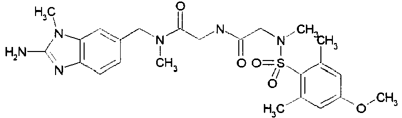 |
| (535) | 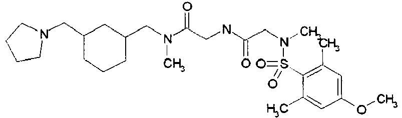 |
| (536) | 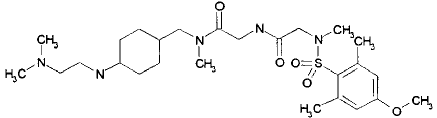 |
| (537) | 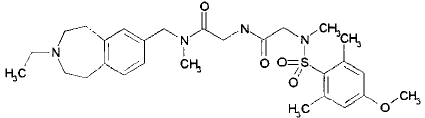 |
| (538) | 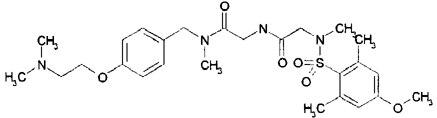 |
| (539) | 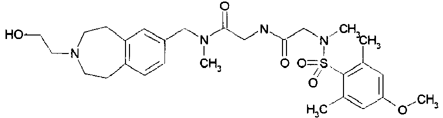 |
| (540) | 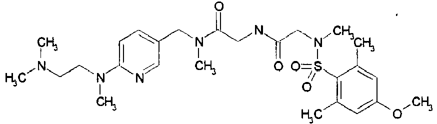 |
| (541) | 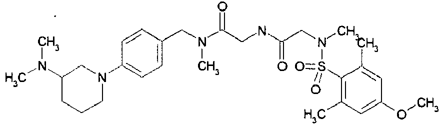 |
| (542) | 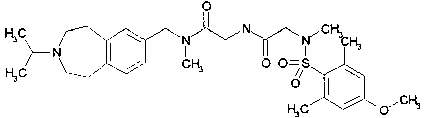 |
| (543) | 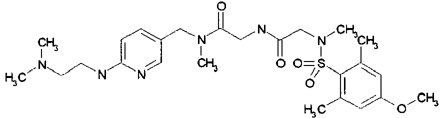 |
| (544) | 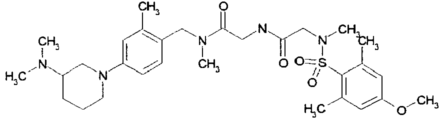 |
| (545) | 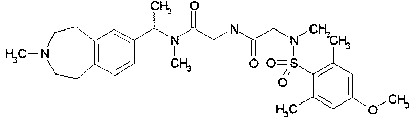 |
| (546) | 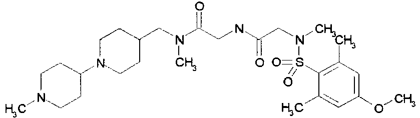 |
| (547) | 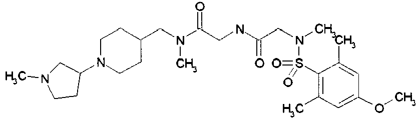 |
| (548) | 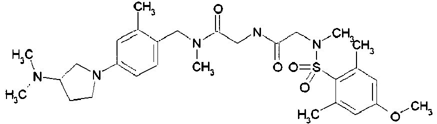 |
| (550) | 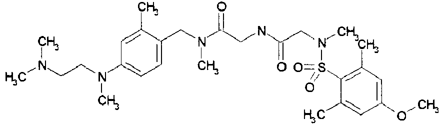 |
| (552) | 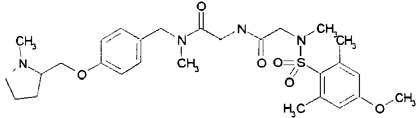 |
| (553) | 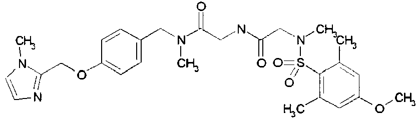 |
| (554) | 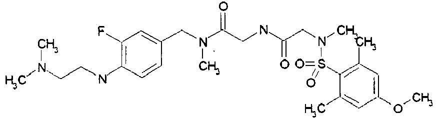 |
| (555) | 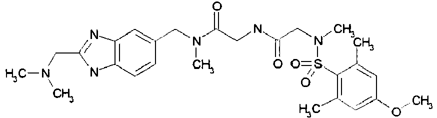 |
| (556) | 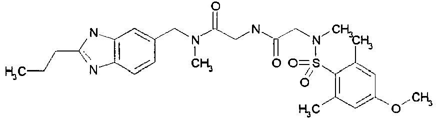 |
| (557) | 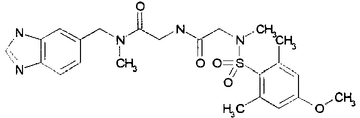 |
| (558) | 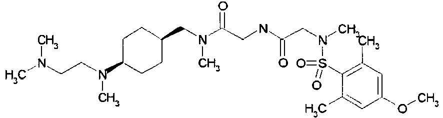 |
| (559) | 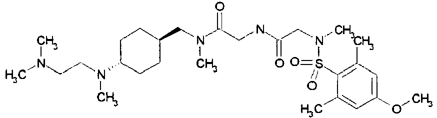 |
| (560) | 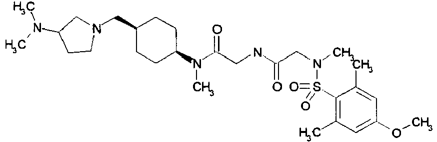 |
| (561) | 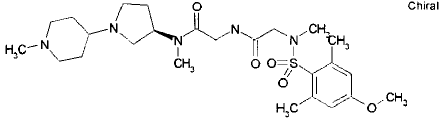 |
| (562) | 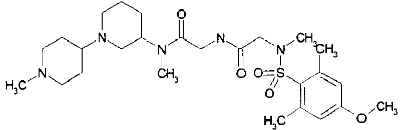 |
| (564) | 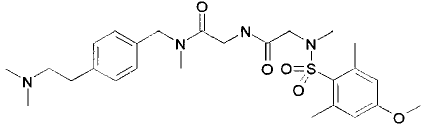 |
| (565) | 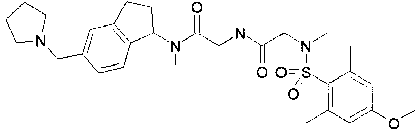 |
| (566) | 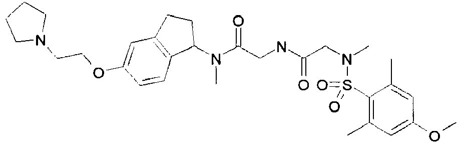 |
| (603) | 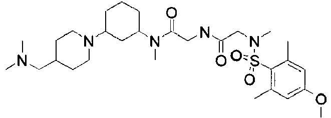 |
| (604) | 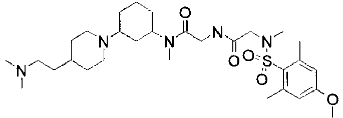 |
| (605) | 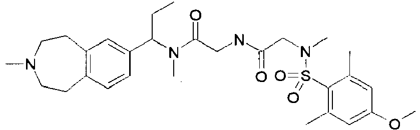 |
| (631) | 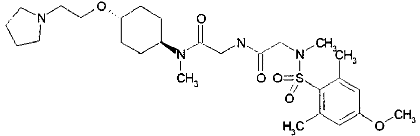 |
| (632) | 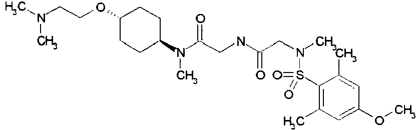 |
| (633) | 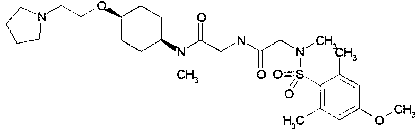 |
| (634) | 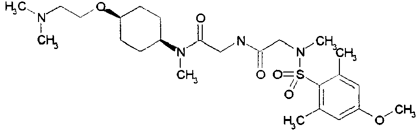 |
deren Enantiomere, deren Diastereomere, deren Gemische und deren Salze, insbesondere deren physiologisch verträgliche Salze mit organischen oder anorganischen Säuren oder Basen.

2. Physiologisch verträgliche Salze der Verbindungen nach Anspruch 1 mit anorganischen oder organischen Säuren oder Basen.

3. Arzneimittel, enthaltend eine Verbindung nach Anspruch 1 oder ein physiologisch verträgliches Salz gemäß Anspruch 2 neben gegebenenfalls einem oder mehreren inerten Trägerstoffen und/oder Verdünnungsmitteln.

4. Verwendung einer Verbindung nach mindestens einem der Ansprüche 1 bis 2 zur Herstellung eines Arzneimittels zur akuten und prophylaktischen Behandlung von akuten Schmerzen, Eingeweideschmerzen, neuropathischen Schmerzen, entzündlichen / Schmerzrezeptor-vermittelten Schmerzen, Tumorschmerzen und Kopfschmerz-Erkrankungen.

5. Verfahren zur Herstellung eines Arzneimittels gemäß Anspruch 3, **dadurch gekennzeichnet, dass** auf nichtchemischem Weg eine Verbindung nach mindestens einem der Ansprüche 1 bis 2 in einen oder mehrere inerte Trägerstoffe und/oder Verdünnungsmittel eingearbeitet wird.

## Claims

1. Compounds of general formula I 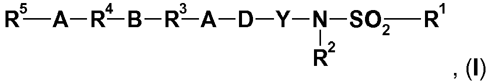 wherein **A, B, D, Y, R¹, R², R³, R⁴** and **R⁵** are defined as in the following compounds:
| **Example** | **Structure** |
|---|---|
| (121) | 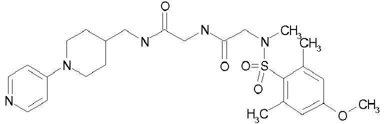 |
| (122) | 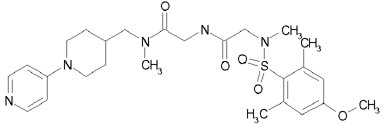 |
| (123) | 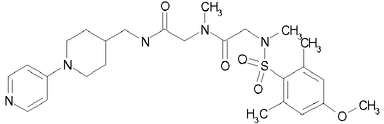 |
| (124) | 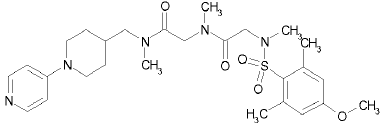 |
| (125) | 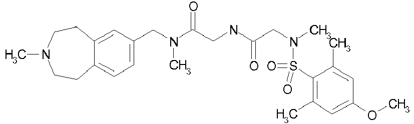 |
| (126) | 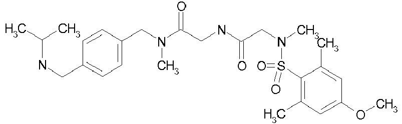 |
| (127) | 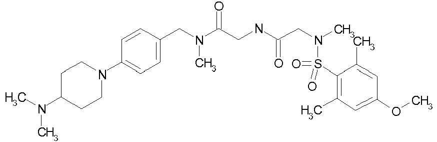 |
| (128) | 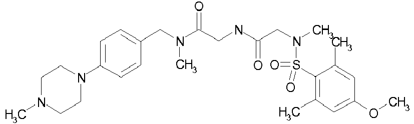 |
| (129) | 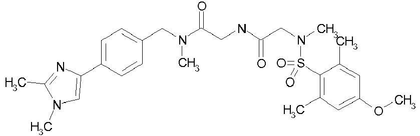 |
| (483) | 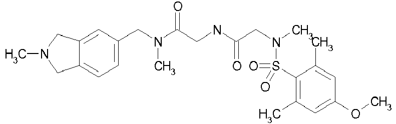 |
| (484) | 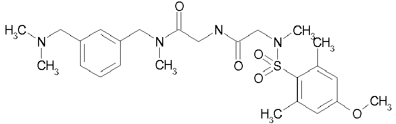 |
| (485) | 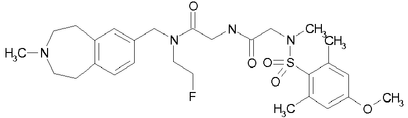 |
| (486) | 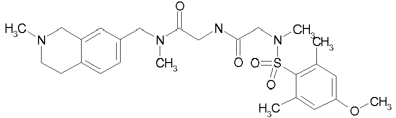 |
| (487) | 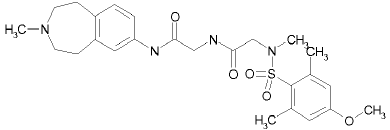 |
| (488) | 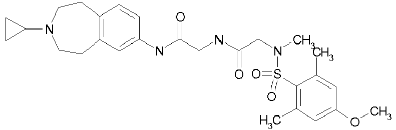 |
| (489) | 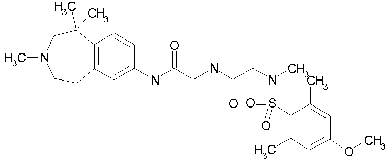 |
| (490) | 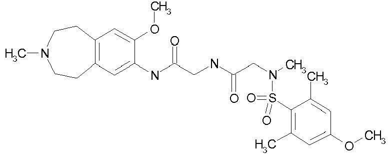 |
| (491) | 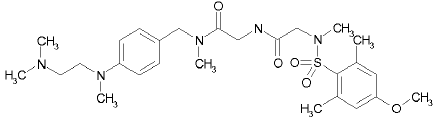 |
| (492) | 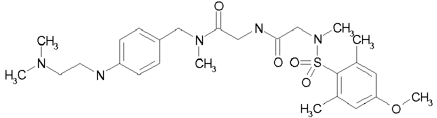 |
| (493) | 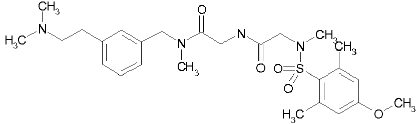 |
| (494) | 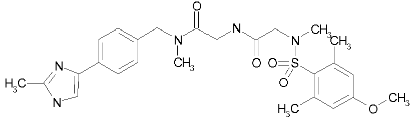 |
| (495) | 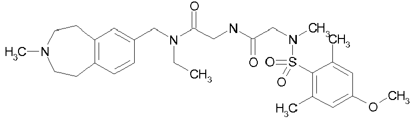 |
| (496) | 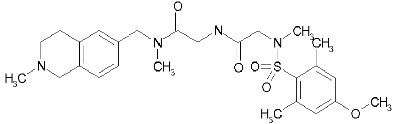 |
| (497) | 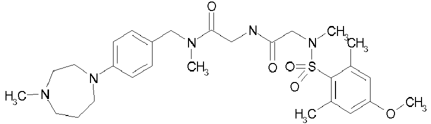 |
| (498) | 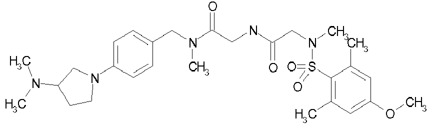 |
| (499) | 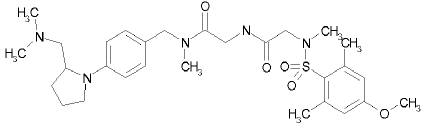 |
| (500) | 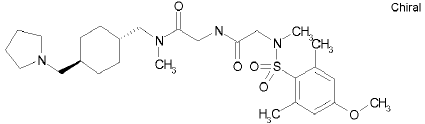 |
| (501) | 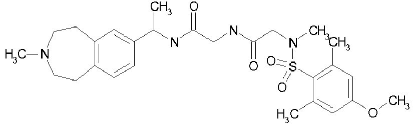 |
| (502) | 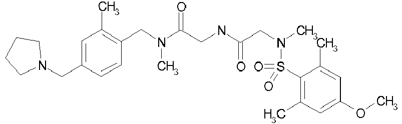 |
| (503) | 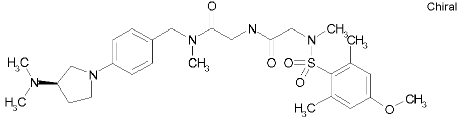 |
| (504) | 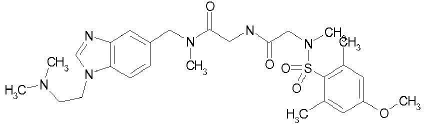 |
| (505) | 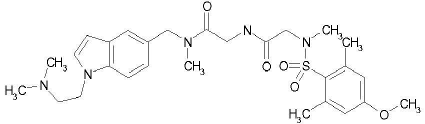 |
| (506) | 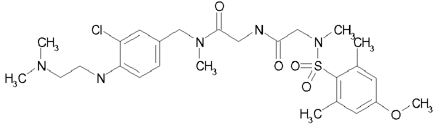 |
| (507) | 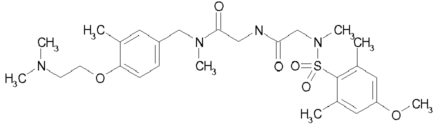 |
| (508) | 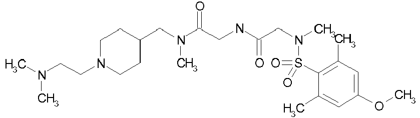 |
| (509) | 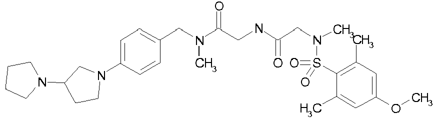 |
| (510) | 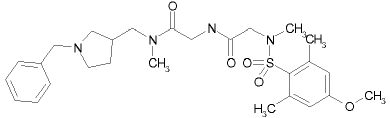 |
| (511) | 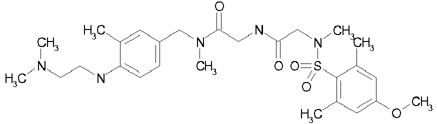 |
| (512) | 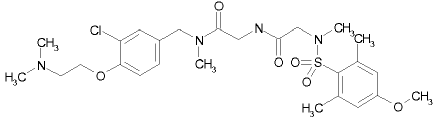 |
| (513) | 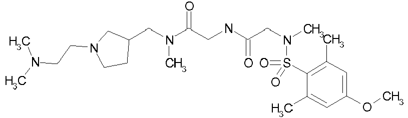 |
| (514) | 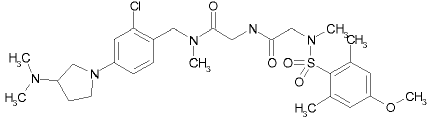 |
| (515) | 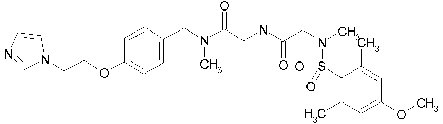 |
| (516) | 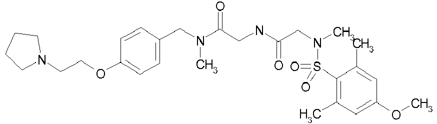 |
| (517) | 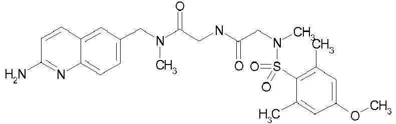 |
| (518) | 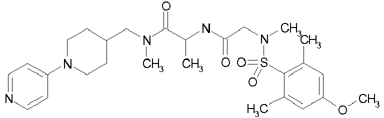 |
| (519) | 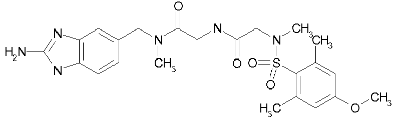 |
| (520) | 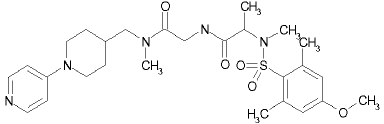 |
| (521) | 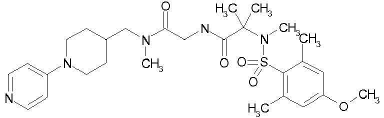 |
| (522) | 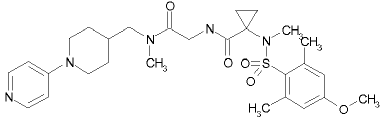 |
| (523) | 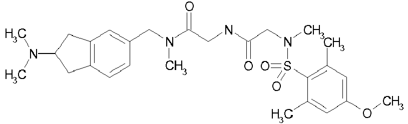 |
| (524) | 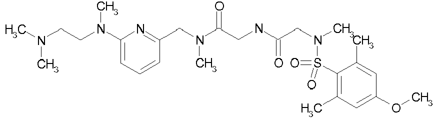 |
| (525) | 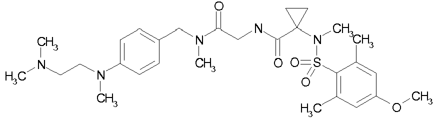 |
| (526) | 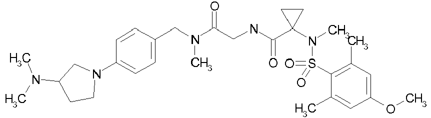 |
| (527) | 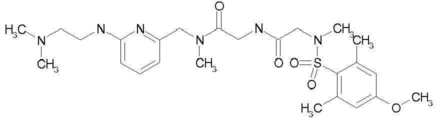 |
| (528) | 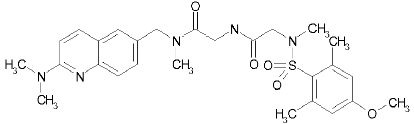 |
| (529) | 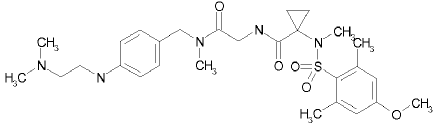 |
| (530) | 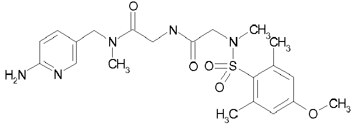 |
| (531) | 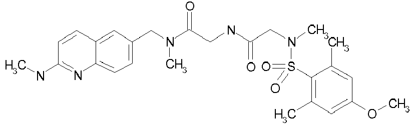 |
| (532) | 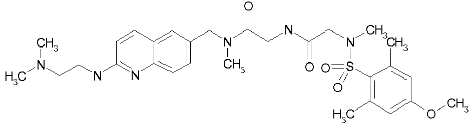 |
| (533) | 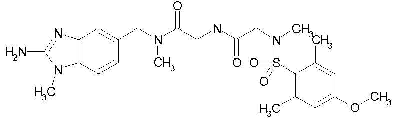 |
| (534) | 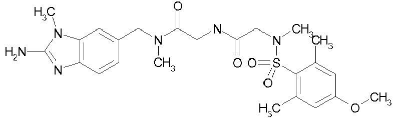 |
| (535) | 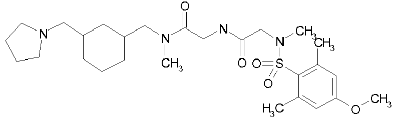 |
| (536) | 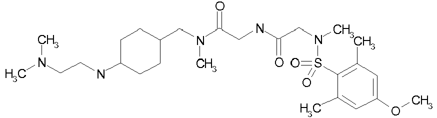 |
| (537) | 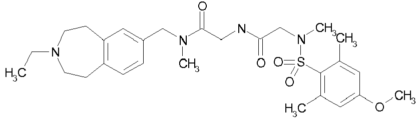 |
| (538) | 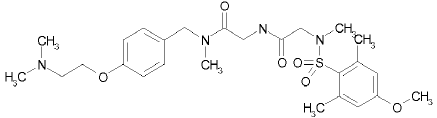 |
| (539) | 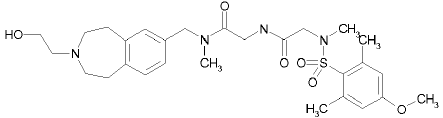 |
| (540) | 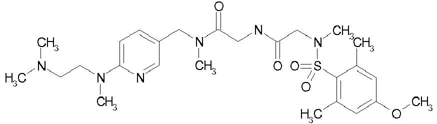 |
| (541) | 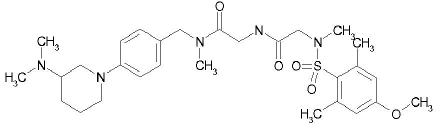 |
| (542) | 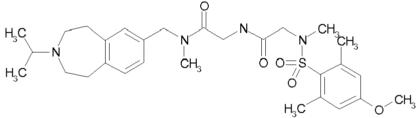 |
| (543) | 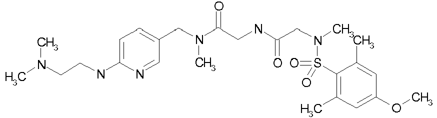 |
| (544) | 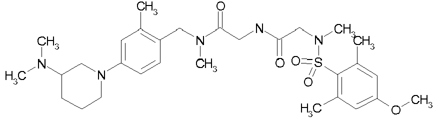 |
| (545) | 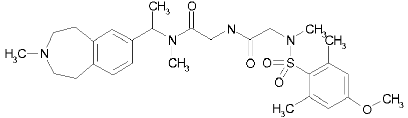 |
| (546) | 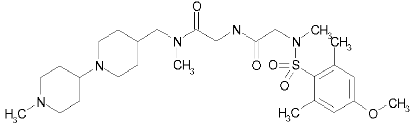 |
| (547) | 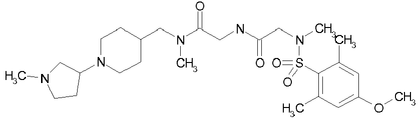 |
| (548) | 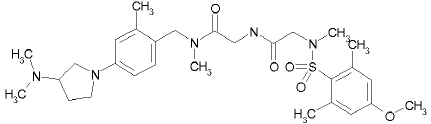 |
| (550) | 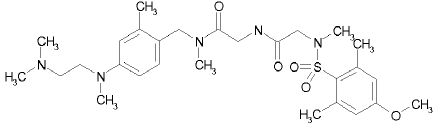 |
| (552) | 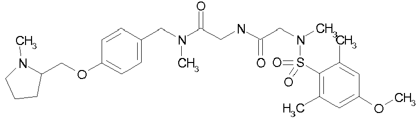 |
| (553) | 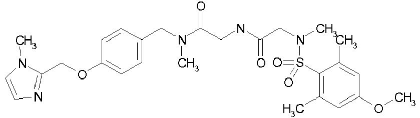 |
| (554) | 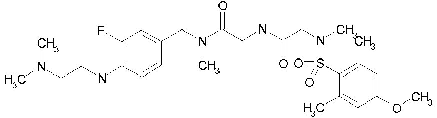 |
| (555) | 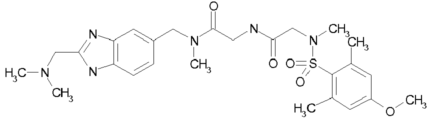 |
| (556) | 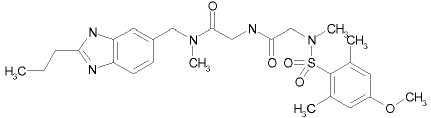 |
| (557) | 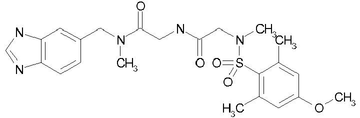 |
| (558) | 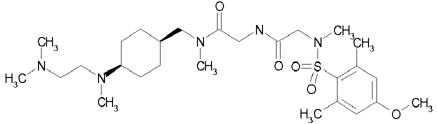 |
| (559) | 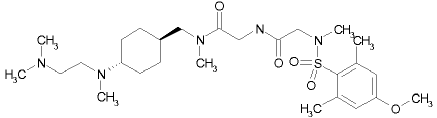 |
| (560) | 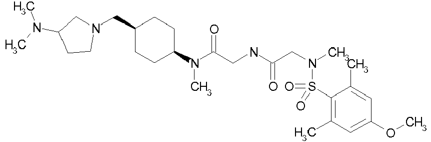 |
| (561) | 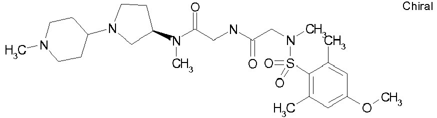 |
| (562) | 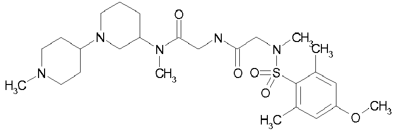 |
| (564) | 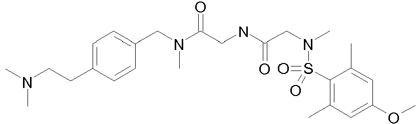 |
| (565) | 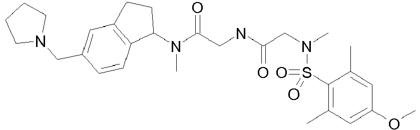 |
| (566) | 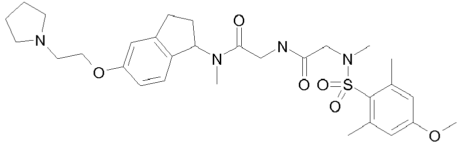 |
| (603) | 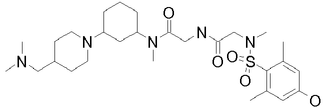 |
| (604) | 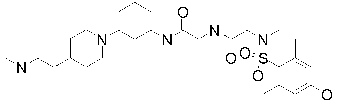 |
| (605) | 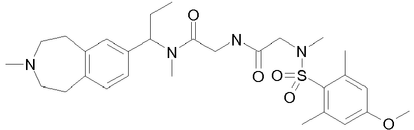 |
| (631) | 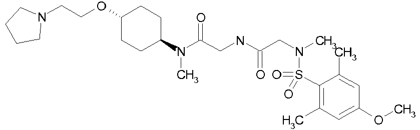 |
| (632) | 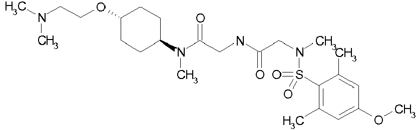 |
| (633) | 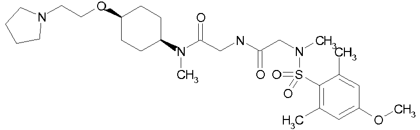 |
| (634) | 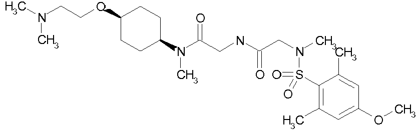 |
the enantiomers, the diastereomers, the mixtures and the salts thereof, particularly the physiologically acceptable salts thereof with organic or inorganic acids or bases.

2. Physiologically acceptable salts of the compounds according to claim 1 with inorganic or organic acids or bases.

3. Medicament, containing a compound according to claim 1 or a physiologically acceptable salt according to claim 2 optionally together with one or more inert carriers and/or diluents.

4. Use of a compound according to at least one of claims 1 to 2 for preparing a medicament for the acute and prophylactic treatment of acute pain, visceral pain, neuropathic pain, inflammatory/pain receptor-mediated pain, tumour pain and headache diseases.

5. Process for preparing a medicament according to claim 3, **characterised in that** a compound according to at least one of claims 1 to 2 is incorporated in one or more inert carriers and/or diluents by a non-chemical method.

## Revendications

1. Composés de formule générale I 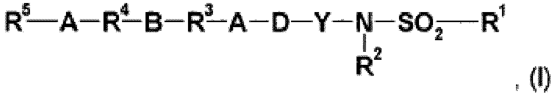 dans laquelle A, B, D, Y, R¹, R², R³, R⁴ et R⁵ sont tels que définis dans les composés suivants :
| Exemple | Structure |
|---|---|
| (121) | 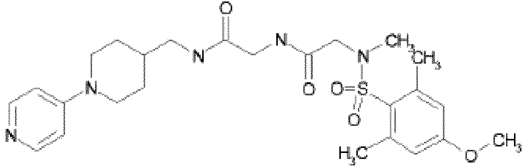 |
| (122) | 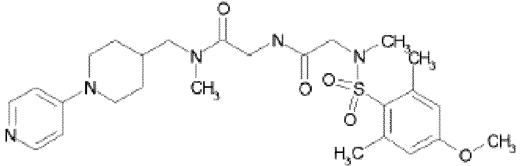 |
| (123) | 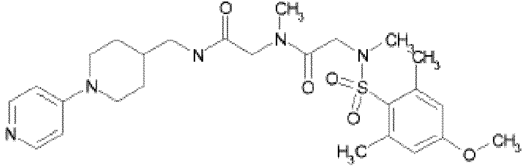 |
| (124) | 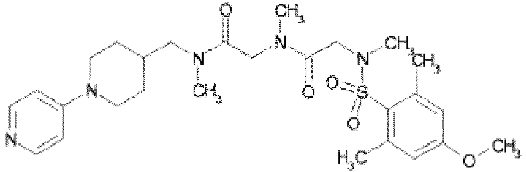 |
| (125) | 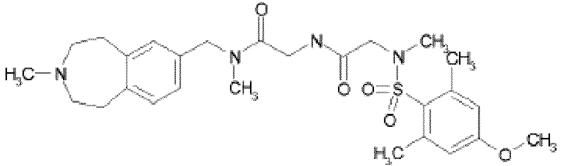 |
| (126) | 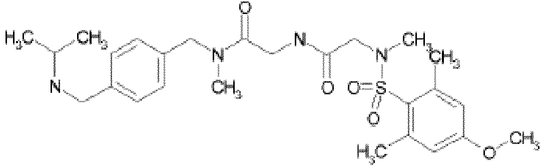 |
| (127) | 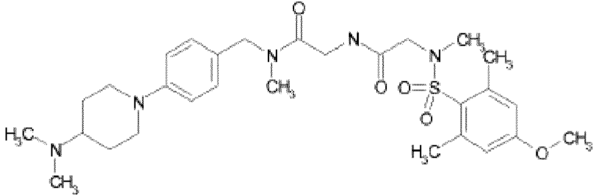 |
| (128) | 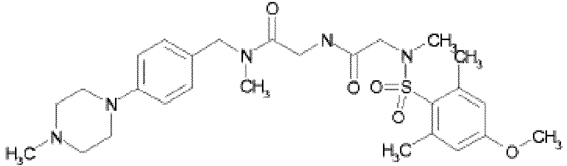 |
| (129) | 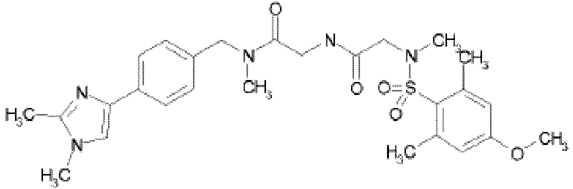 |
| (483) | 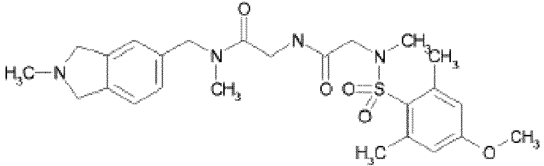 |
| (484) | 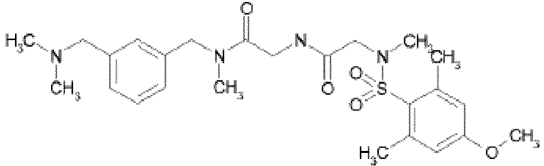 |
| (485) | 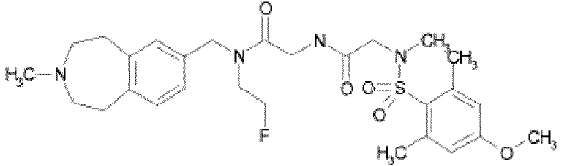 |
| (486) | 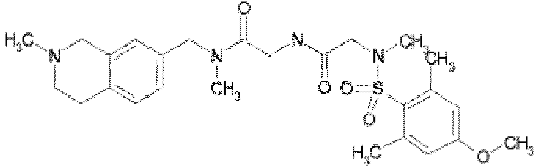 |
| (487) | 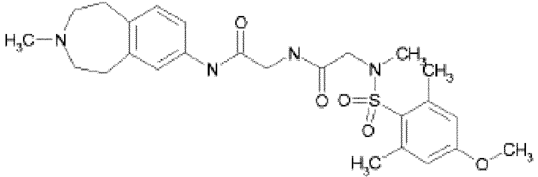 |
| (488) | 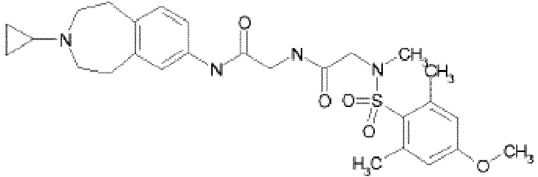 |
| (489) | 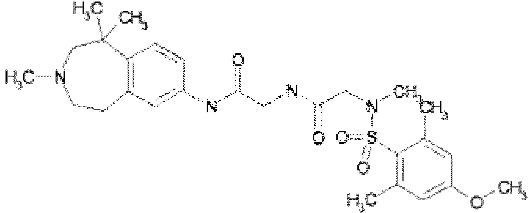 |
| (490) | 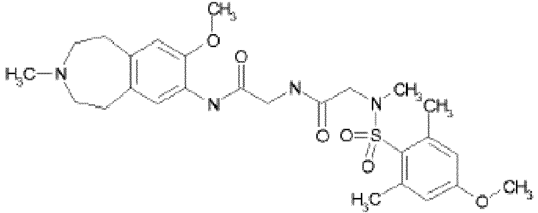 |
| (491) | 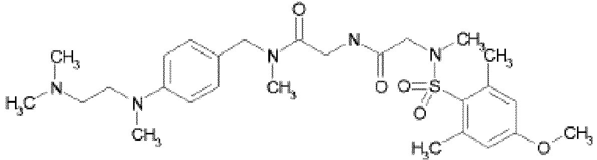 |
| (492) | 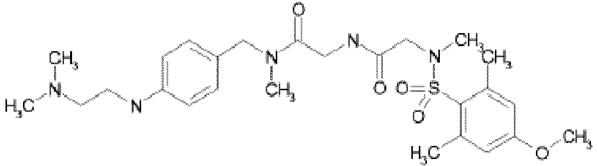 |
| (493) | 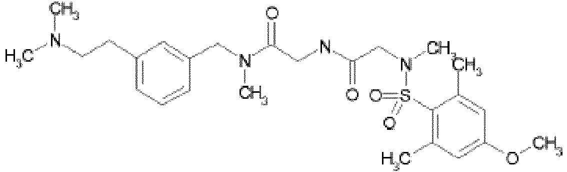 |
| (494) | 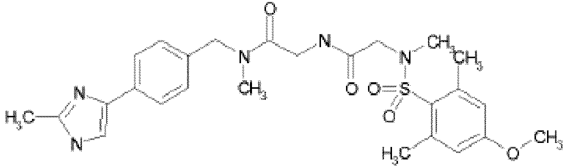 |
| (495) | 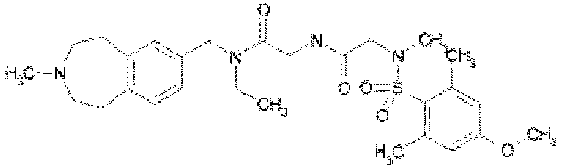 |
| (496) | 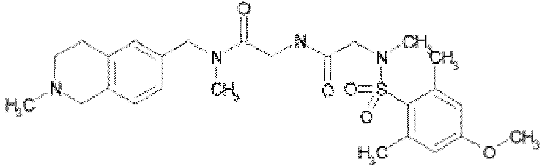 |
| (497) | 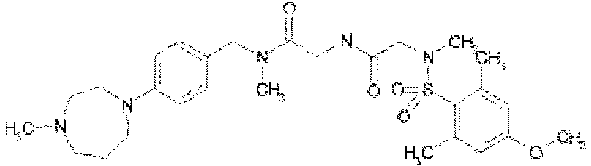 |
| (498) | 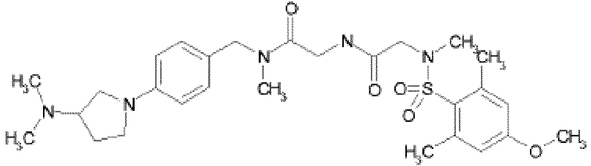 |
| (499) | 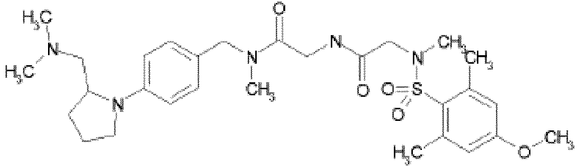 |
| (500) | 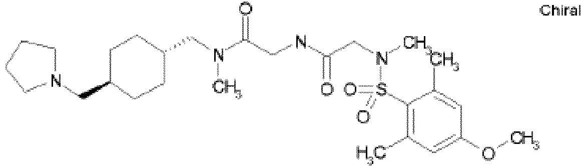 |
| (501) | 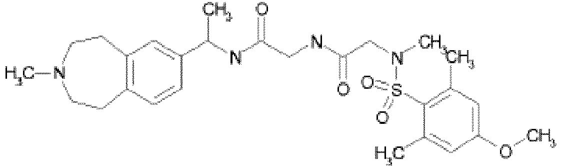 |
| (502) | 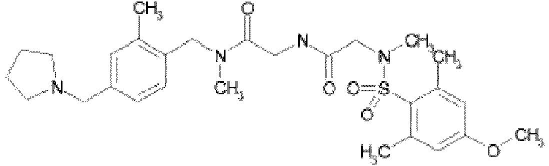 |
| (503) | 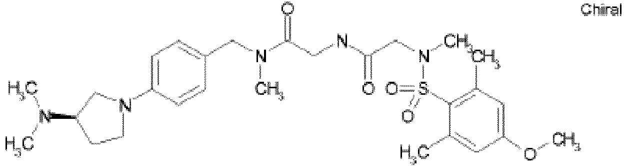 |
| (504) | 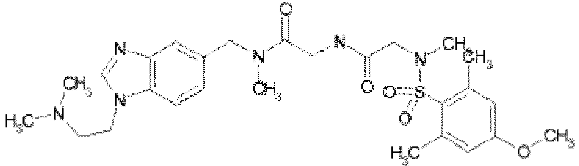 |
| (505) | 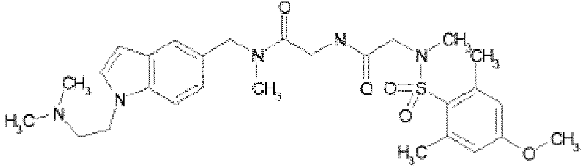 |
| (506) | 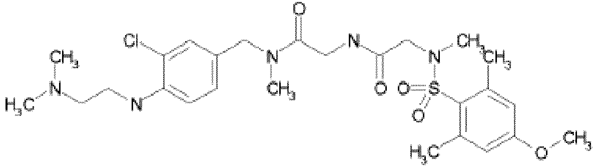 |
| (507) | 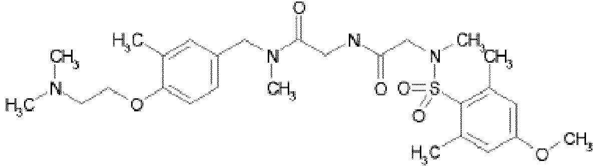 |
| (508) | 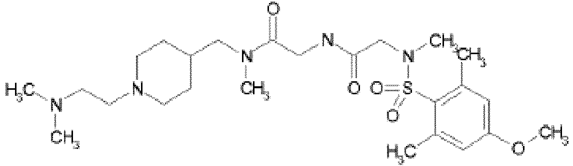 |
| (509) | 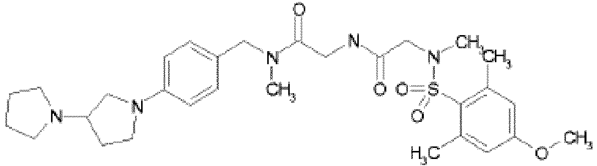 |
| (510) | 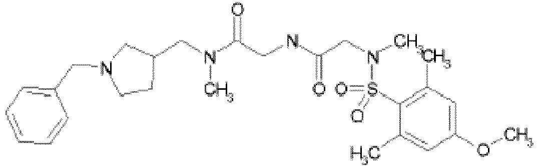 |
| (511) | 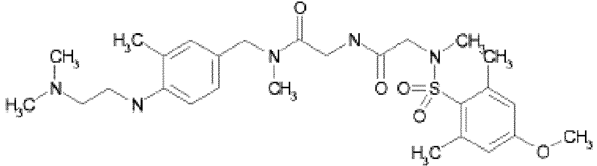 |
| (512) | 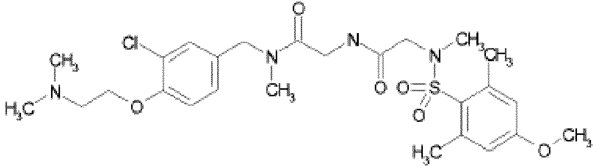 |
| (513) | 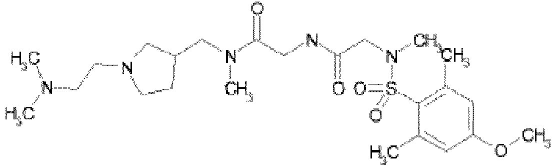 |
| (514) | 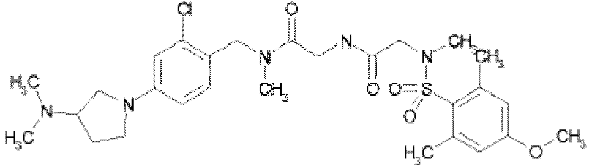 |
| (515) | 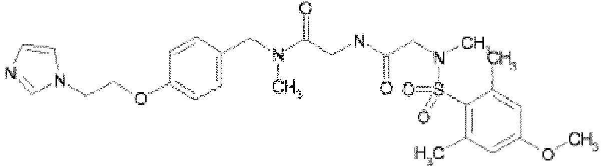 |
| (516) | 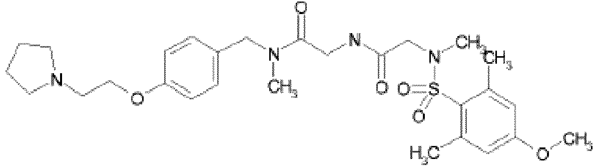 |
| (517) | 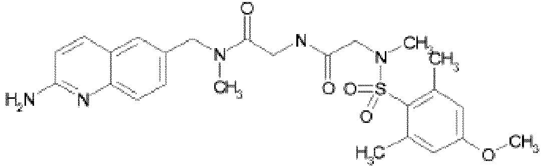 |
| (518) | 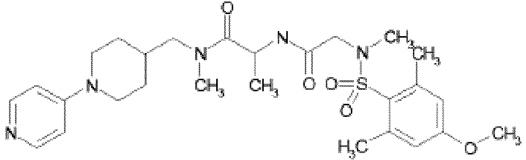 |
| (519) | 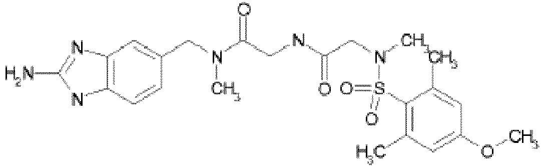 |
| (520) | 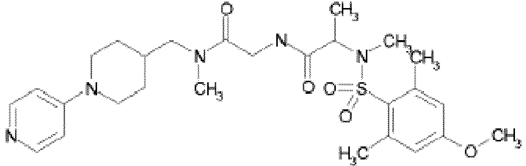 |
| (521) | 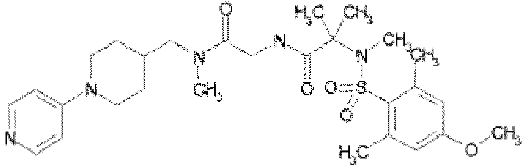 |
| (522) | 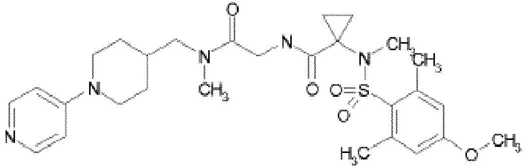 |
| (523) | 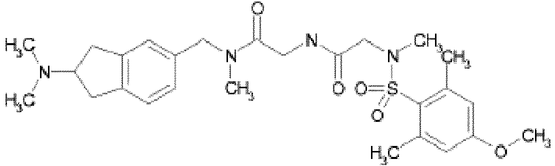 |
| (524) | 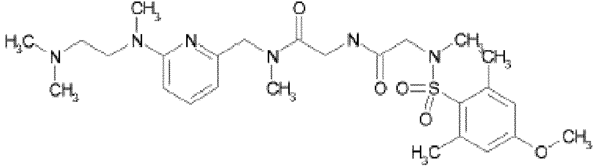 |
| (525) | 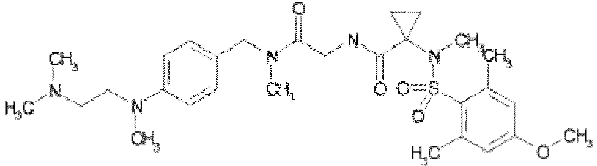 |
| (526) | 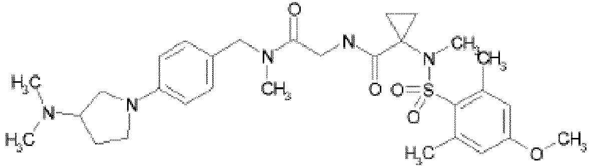 |
| (527) | 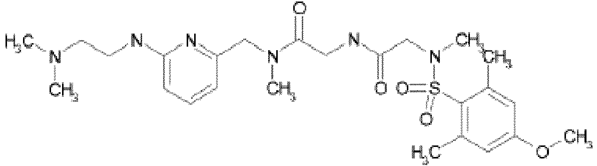 |
| (528) | 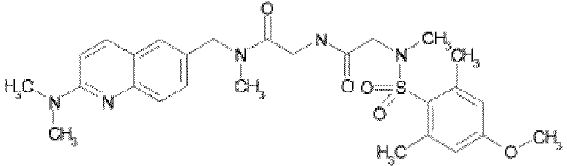 |
| (529) | 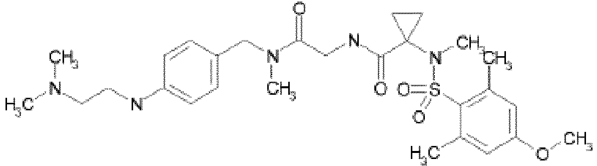 |
| (530) | 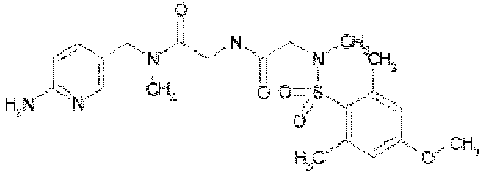 |
| (531) | 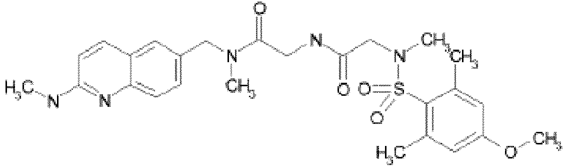 |
| (532) | 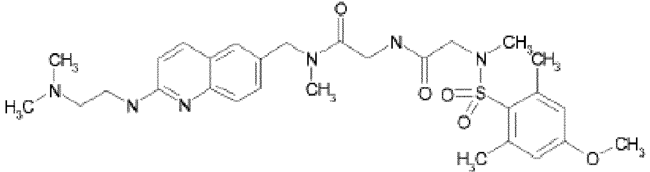 |
| (533) | 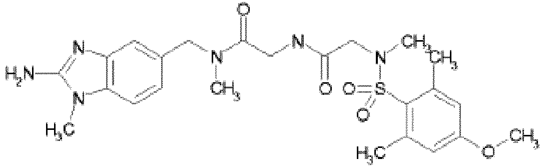 |
| (534) | 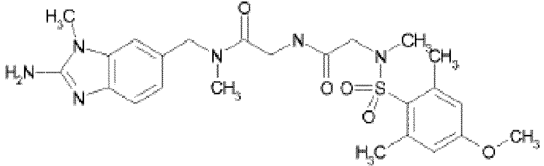 |
| (535) | 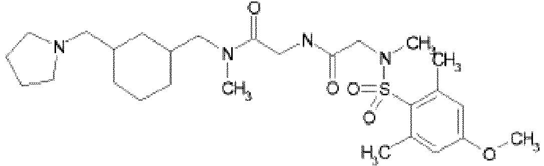 |
| (536) | 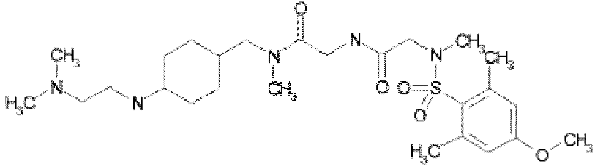 |
| (537) | 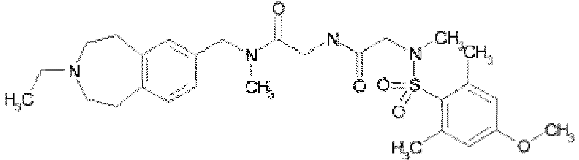 |
| (538) | 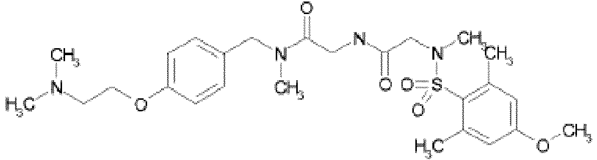 |
| (539) | 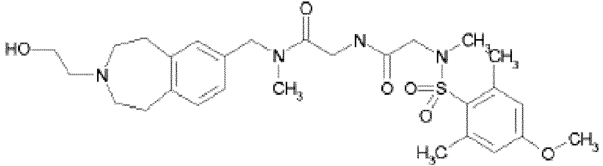 |
| (540) | 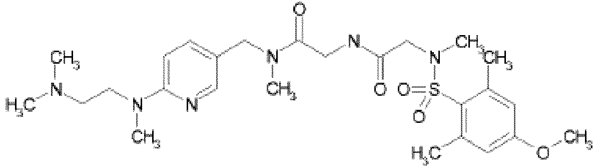 |
| (541) | 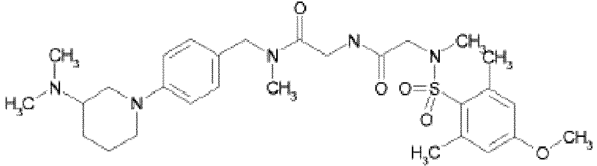 |
| (542) | 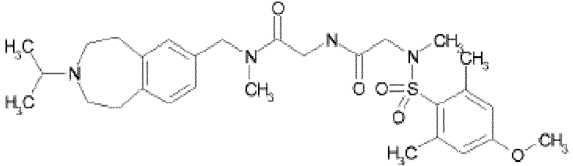 |
| (543) | 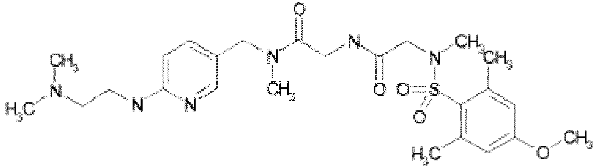 |
| (544) | 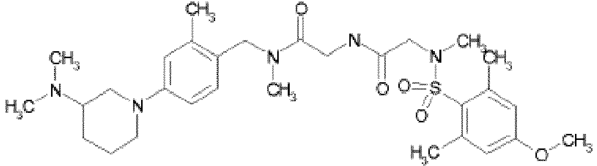 |
| (545) | 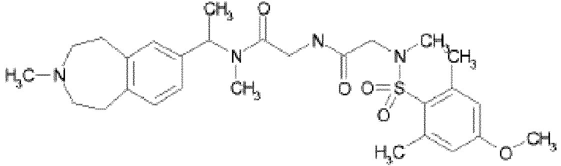 |
| (546) | 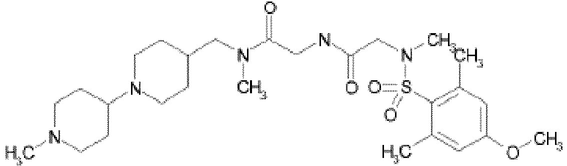 |
| (547) | 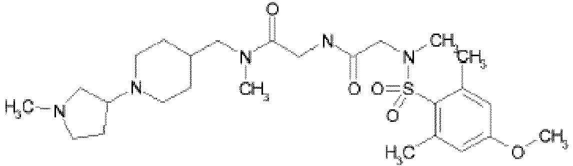 |
| (548) | 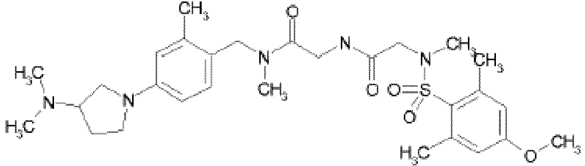 |
| (550) | 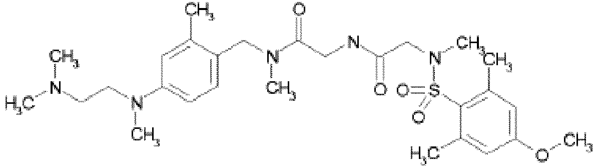 |
| (552) | 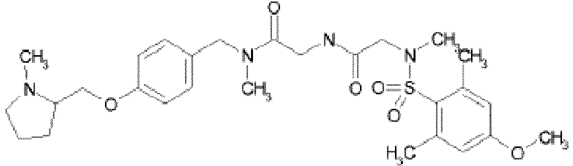 |
| (553) | 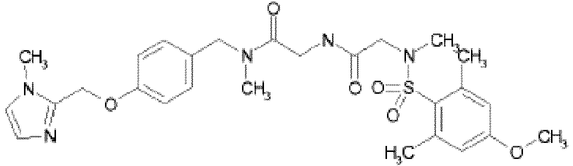 |
| (554) | 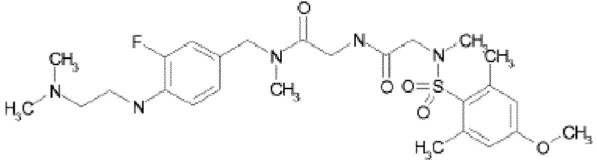 |
| (555) | 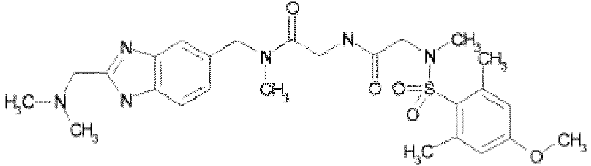 |
| (556) | 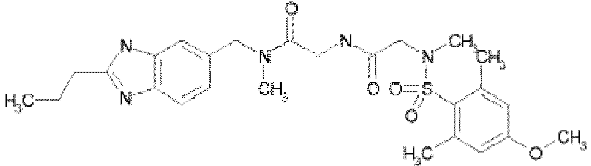 |
| (557) | 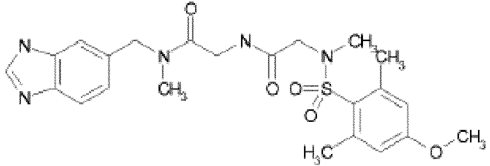 |
| (558) | 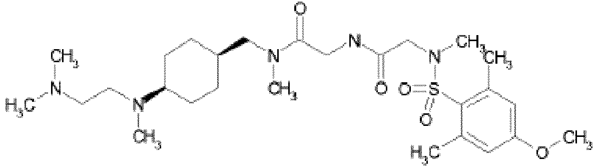 |
| (559) | 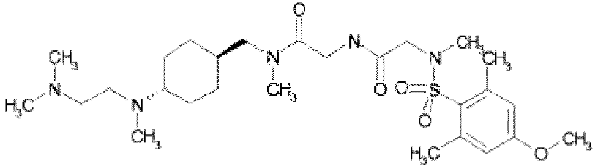 |
| (560) | 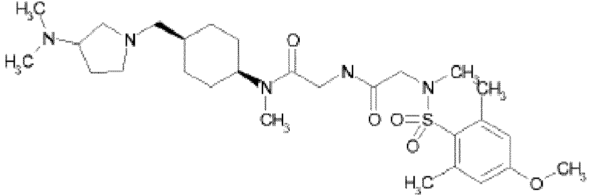 |
| (561) | 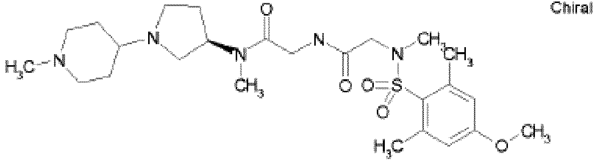 |
| (562) | 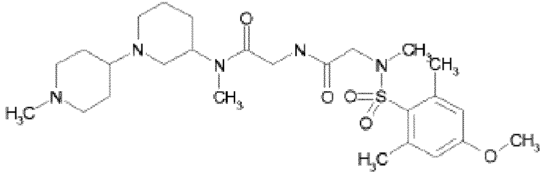 |
| (564) | 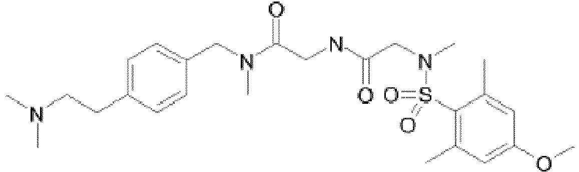 |
| (565) | 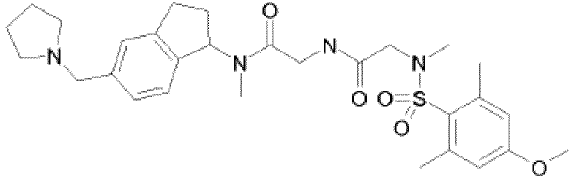 |
| (566) | 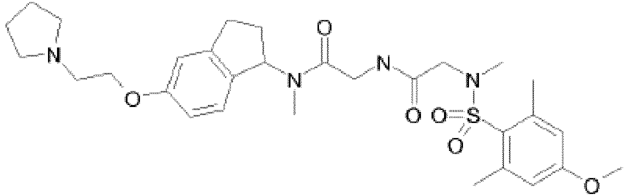 |
| (603) | 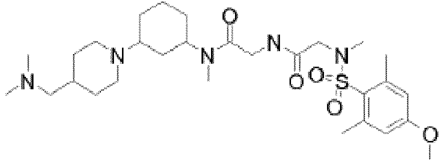 |
| (604) | 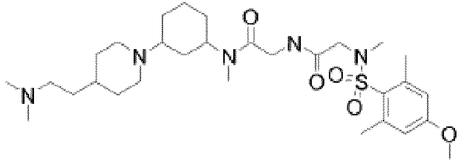 |
| (605) | 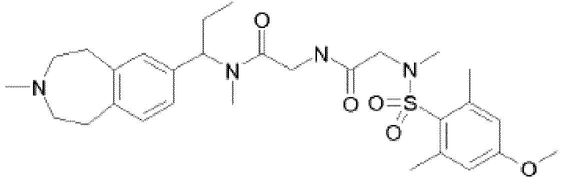 |
| (631) | 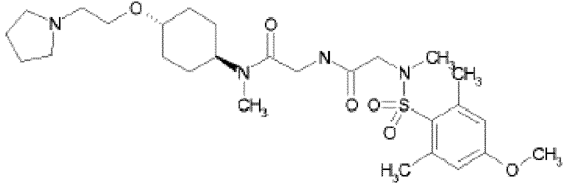 |
| (632) | 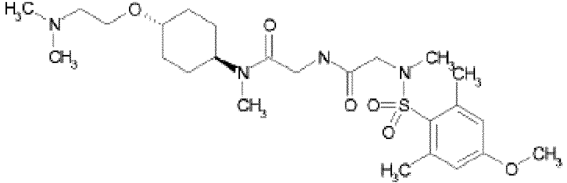 |
| (633) | 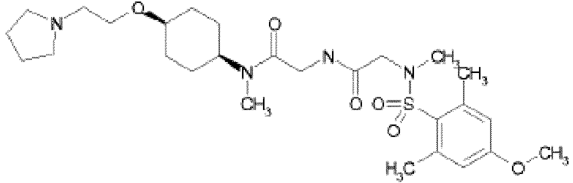 |
| (634) | 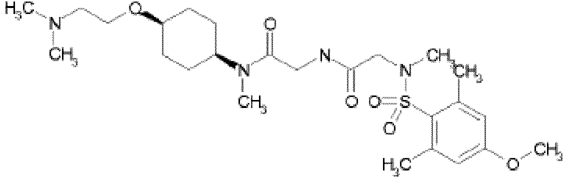 |
leurs énantiomères, leurs diastéréomères, leurs mélanges et leurs sels, en particulier, leurs sels physiologiquement acceptables, avec des acides ou bases organiques ou anorganiques.

2. Sels physiologiquement acceptables des composés selon la revendication 1, avec des acides ou bases anorganiques ou organiques.

3. Médicament, contenant un composé selon la revendication 1 ou un sel physiologiquement acceptable selon la revendication 2, parallèlement à éventuellement, un ou plusieurs véhicules et/ou diluants inertes.

4. Utilisation d'un composé selon au moins l'une des revendications 1 à 2, pour la production d'un médicament pour le traitement aigu ou prophylactique de douleurs aiguës, de douleurs viscérales, de douleurs neuropathiques, de douleurs inflammatoires/ induites par un récepteur de la douleur, de douleurs tumorales et des céphalées.

5. Procédé de production d'un médicament selon la revendication 3, **caractérisé en ce que**, de façon non chimique, un composé selon au moins l'une des revendications 1 à 2 est incorporé dans un ou plusieurs véhicules et/ou diluants inertes.
